(19) Europäisches Patentamt / European Patent Office / Office européen des brevets

(11) **EP 4 610 282 A1**

(12) **EUROPEAN PATENT APPLICATION**
published in accordance with Art. 153(4) EPC

(43) Date of publication:
**03.09.2025 Bulletin 2025/36**

(21) Application number: **23882746.3**

(22) Date of filing: **27.10.2023**

(51) International Patent Classification (IPC):
***C08F 20/06*** (2006.01) ***C07C 43/205*** (2006.01) ***C07C 53/124*** (2006.01) ***C07C 57/075*** (2006.01) ***C07C 57/30*** (2006.01) ***C07C 67/00*** (2006.01) ***C07C 69/54*** (2006.01) ***C07C 255/08*** (2006.01) ***C08F 2/40*** (2006.01) ***C08F 2/44*** (2006.01) ***C08F 220/06*** (2006.01)

(52) Cooperative Patent Classification (CPC):
(C-Sets available)
**C07C 51/50; C08F 2/40; C08F 2/44; C08F 20/06; C08F 220/06** (Cont.)

(86) International application number:
**PCT/JP2023/038845**

(87) International publication number:
**WO 2024/090544 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**28.10.2022 JP 2022172927**
**28.10.2022 JP 2022172928**
**28.10.2022 JP 2022173123**
**28.10.2022 JP 2022173124**
**28.10.2022 JP 2022173222**
**28.10.2022 JP 2022173223**

(71) Applicant: **Mitsubishi Chemical Corporation**
**Tokyo 100-8251 (JP)**

(72) Inventors:
- **SUZUKI, Tatsuya**
**Tokyo 100-8251 (JP)**
- **KURIHARA, Yu**
**Tokyo 100-8251 (JP)**
- **KATOU, Yuuki**
**Tokyo 100-8251 (JP)**
- **KANEMORI, Kouichi**
**Tokyo 100-8251 (JP)**
- **NARUYOSHI, Wataru**
**Tokyo 100-8251 (JP)**

(74) Representative: **Hoffmann Eitle**
**Patent- und Rechtsanwälte PartmbB**
**Arabellastraße 30**
**81925 München (DE)**

# (54) METHACRYLATE-CONTAINING COMPOSITION, METHOD FOR PRODUCING METHACRYLIC ESTER, POLYMERIZABLE COMPOSITION, AND METHOD FOR PRODUCING METHACRYLIC ACID POLYMER

(57) An object of the present invention is to provide a methacrylate-containing composition having high quality stability during storage.

The methacrylate-containing composition according to one example comprises methacrylic acid, a compound (component A1) represented by Formula (11), and a polymerization inhibitor (component B), in which the concentration of the methacrylic acid is 98.00% to 99.99% by mass.

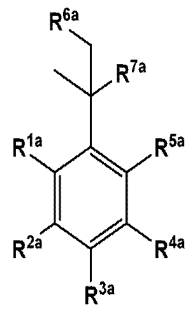

・・・(11)

$R^{1a}$, $R^{2a}$, $R^{3a}$, $R^{4a}$, $R^{5a}$, and $R^{6a}$: a hydrogen atom, an

EP 4 610 282 A1

alkyl group, an alkenyl group, an aryl group, a hydroxy group, an alkoxy group, an amino group, a monovalent group including a carbonyl group, an alkylthio group, or an arylthio group.

Two or more of $R^{1a}$, $R^{2a}$, $R^{3a}$, $R^{4a}$, and $R^{5a}$: groups other than a hydrogen atom.

$R^{7a}$: a hydrogen atom, an alkenyl group, an aryl group, a hydroxy group, an alkoxy group, an amino group, a monovalent group including a carbonyl group, an alkylthio group, or an arylthio group.

(52) Cooperative Patent Classification (CPC): (Cont.)

C-Sets
**C07C 51/50, C07C 57/04**

## Description

### TECHNICAL FIELD

The present invention relates to a methacrylate-containing composition, a method for producing a methacrylic ester, a polymerizable composition, and a method for producing a methacrylic acid polymer.

Priority is claimed on Japanese Patent Application No. 2022-172927, Japanese Patent Application No. 2022-172928, Japanese Patent Application No. 2022-173123, Japanese Patent Application No. 2022-173124, Japanese Patent Application No. 2022-173222, and Japanese Patent Application No. 2022-173223, filed October 28, 2022, the contents of which are incorporated herein by reference.

### BACKGROUND ART

Methacrylic acid (hereinafter, also referred to as "MAA") is known as an extremely useful substance that is used as a raw material for methacrylic esters which are industrially important, and polymers having various uses and types. For example, polymethyl methacrylate which is a homopolymer of methyl methacrylate is used for a signboard, a lighting device, an automobile part, a building-related material, a light guide plate for a flat display, a light diffusion plate, and the like, taking advantage of characteristics such as excellent transparency and weather fastness. In addition, a copolymer of the methacrylic acid and another monomer is used in a coating material, an adhesive, a fiber treatment agent, a resin reforming agent, a rubber reforming agent, a leather treatment agent, a paper processing agent, a lubricant additive, a cement mixing agent, a concrete mixing agent, latex, a photosensitive resin, an ion exchange resin, a water treatment polymer, and the like. Various methods for industrially producing the methacrylic acid have been developed, and the methacrylic acid is produced as a by-product of an acetone cyanohydrin (ACH) method or an intermediate of a C4 direct oxidation method (Non-Patent Document 1). In these production methods, in order to remove unreacted raw materials, by-products, and the like contained in the produced methacrylic acid, purification such as distillation and crystallization is performed to obtain methacrylic acid having a quality suitable for a desired use.

Since the methacrylic acid has a property of easily polymerizing, it has been known that, when producing methacrylic acid or storing produced methacrylic acid, a polymerization inhibitor is added thereto to maintain the quality of methacrylic acid (Non-Patent Document 2). For example, Patent Document 1 discloses that methyl ether of hydroquinone (MEHQ) is particularly preferable among various polymerization inhibitors. Patent Document 2 discloses that phenothiazine and hydroquinone are added to a methacrylate-containing substance as a polymerization inhibitor. Patent Document 3 discloses that methacrylic acid is distilled in the presence of 4-methoxyphenol which is a polymerization inhibitor. Patent Document 4 discloses that a diphenylamine derivative is used as a polymerization inhibitor. Patent Document 5 discloses that a benzentriamine derivative is used as a polymerization inhibitor.

### Citation List

#### Patent Documents

Patent Document 1:Japanese Unexamined Patent Application, First Publication No. 2004-155757
Patent Document 2: Japanese Unexamined Patent Application, First Publication No. 2001-072639
Patent Document 3: Japanese Unexamined Patent Application, First Publication No. 2008-101013
Patent Document 4: Published Japanese Translation No. 2002-533309 of the PCT International Publication
Patent Document 5: Published Japanese Translation No. 2002-513034 of the PCT International Publication

#### Non-Patent Documents

Non-Patent Document 1: T. Kuroda, "Development of Catalyst for Producing Methyl Methacrylate", Catalysts, The Catalysis Society of Japan, 2003, Vol. 45, No. 5, pp. 366 to 371
Non-Patent Document 2: Takayuki Otsu, "On the Function of Polymerization Inhibitor", Organic Synthesis Chemistry, The Chemical Society of Japan, 1975, Vol. 33, No. 8, pp. 634 to 640

### SUMMARY OF INVENTION

Technical Problem

However, even when the polymerization inhibitor is added, the quality of the methacrylic acid may be deteriorated during storage. An object of the present invention is to provide a methacrylate-containing composition having high quality stability during storage.

Solution to Problem

The present inventors have conducted intensive studies to achieve the above object. As a result, it is found that the quality stability during storage is improved and decomposition of the polymerization inhibitor is suppressed by a compound having a specific structure in the methacrylate-containing composition, thereby completing the present invention.
According to one aspect of the present invention, the following methacrylate-containing composition is provided.
A methacrylate-containing composition comprising:

methacrylic acid; and
at least one selected from the group consisting of a component A1 which is a compound represented by Formula (11), a component A21 which is a compound represented by Formula (21), a component A3 which is a compound represented by Formula (31), a component A4 which is a compound represented by Formula (41), a component A5 which is a compound represented by Formula (51), and a component A6 which is a compound represented by Formula (61),
wherein the methacrylate-containing composition may further optionally comprise a component B which is a polymerization inhibitor as necessary, and
the concentration of the methacrylic acid is 98.00% to 99.99% by mass.

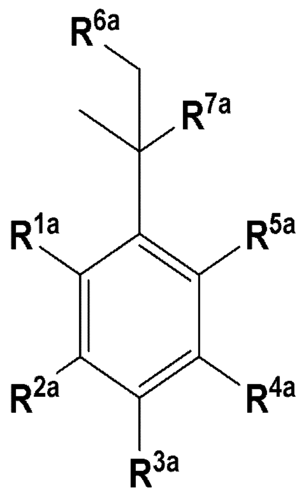

・・・(11)

In Formula (11),

$R^{1a}$, $R^{2a}$, $R^{3a}$, $R^{4a}$, $R^{5a}$, and $R^{6a}$ each independently represent a hydrogen atom, an alkyl group, an alkenyl group, a hydroxy group, an alkoxy group, an amino group, a monovalent group including a carbonyl group, an alkylthio group, or an arylthio group,
two or more of $R^{1a}$, $R^{2a}$, $R^{3a}$, $R^{4a}$, and $R^{5a}$ are groups other than a hydrogen atom, and
$R^{7a}$ represents a hydrogen atom, an alkenyl group, an aryl group, a hydroxy group, an alkoxy group, an amino group, a monovalent group including a carbonyl group, an alkylthio group, or an arylthio group.

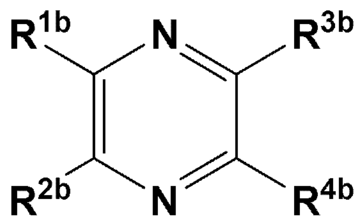

・・・(21)

In Formula (21),
$R^{1b}$, $R^{2b}$, $R^{3b}$, and $R^{4b}$ each independently represent a hydrogen atom, an alkyl group, an alkenyl group, an aryl group, a hydroxy group, an alkoxy group, an amino group, a monovalent group including a carbonyl group, an alkylthio group, or an arylthio group.

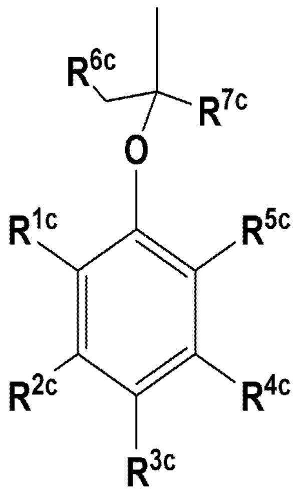

・・・(31)

In Formula (31),
$R^{1c}$, $R^{2c}$, $R^{3c}$, $R^{4c}$, $R^{5c}$, $R^{6c}$, and $R^{7c}$ independently represent a hydrogen atom, an alkyl group, an alkenyl group, an aryl group, a hydroxy group, an alkoxy group, an amino group, a monovalent group including a carbonyl group, an alkylthio group, or an arylthio group.

$R^d$-C≡N ・・・ (41)

In Formula (41),
$R^d$ represents an alkyl group having 1 to 6 carbon atoms, an alkenyl group having 2 to 6 carbon atoms, or an aryl group having 1 to 12 carbon atoms, where these groups may further have a substituent.

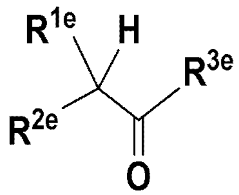

・・・(51)

In Formula (51),

$R^{1e}$ and $R^{2e}$ each independently represent a hydrogen atom, an alkyl group, an alkenyl group, an aryl group, a hydroxy group, an alkoxy group, an alkylthio group, or an arylthio group,
$R^{3e}$ represents a hydrogen atom, an alkyl group, an alkenyl group, an aryl group, a hydroxy group, an alkoxy group, an amino group, an alkylthio group, or an arylthio group, and
$R^{1e}$ and $R^{2e}$, $R^{2e}$ and $R^{3e}$, or $R^{3e}$ and $R^{1e}$ may be linked to each other to form a ring,
provided that the total number of carbon atoms in $R^{1e}$ and $R^{2e}$ is 2 or more.

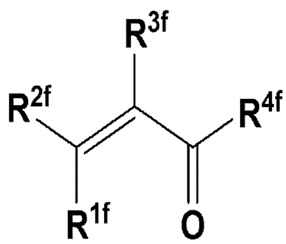

・・・(61)

In Formula (61),

$R^{1f}$, $R^{2f}$, and $R^{3f}$ each independently represent a hydrogen atom, an alkyl group, an alkenyl group, an aryl group, a hydroxy group, an alkoxy group, an amino group, an alkylthio group, or an arylthio group, and
$R^{4f}$ represents an alkyl group, an alkenyl group, an aryl group, a hydroxy group, an alkoxy group, an amino group, a monovalent group including a carbonyl group, an alkylthio group, or an arylthio group,
provided that a case where $R^{1f}$ = H, $R^{2f}$ = H, $R^{3f}$ = $CH_3$, and $R^{4f}$ = OH, that is, a case where the component A6 is methacrylic acid is excluded, and the total number of carbon atoms in $R^{1f}$, $R^{2f}$, and $R^{3f}$ is 1 or more, H represents a hydrogen atom, C represents a carbon atom, and O represents an oxygen atom.

According to another aspect of the present invention, a method for producing a methacrylic ester is provided, the production method comprising esterifying the methacrylic acid in the methacrylate-containing composition.
According to still another aspect of the present invention, a polymerizable composition comprising the methacrylate-containing composition is provided.
According to still another aspect of the present invention, a method for producing a methacrylic acid polymer, comprising polymerizing the polymerizable composition, is provided.

Advantageous Effects of Invention

According to the present invention, it is possible to provide a methacrylate-containing composition having high quality stability, in which decomposition of a polymerization inhibitor during storage is suppressed.

DESCRIPTION OF EMBODIMENTS

Hereinafter, embodiments will be described, but the present invention is not limited to the following.
In the present specification, a numerical value range represented using "to" means a range including the numerical values written before and after "to" as a lower limit value and an upper limit value, and "A to B" means A or more and B or less. The numerical value range described in the present specification can be any numerical value range of any combination of the lower limit value and the upper limit value thereof.

[Methacrylate-containing composition according to first aspect]

A methacrylate-containing composition according to a first aspect comprises methacrylic acid, a component A1 which is a compound represented by Formula (11), and a component B which is a polymerization inhibitor. The concentration of the methacrylic acid is 98.00% to 99.99% by mass.

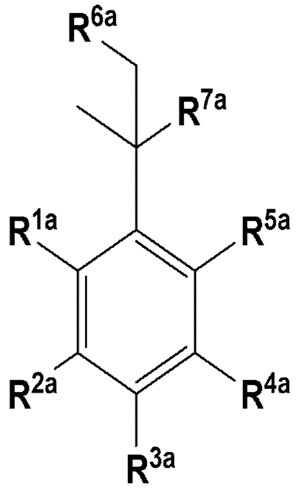

・・・(11)

In Formula (11), $R^{1a}$, $R^{2a}$, $R^{3a}$, $R^{4a}$, $R^{5a}$, and $R^{6a}$ each independently represent a hydrogen atom, an alkyl group, an alkenyl group, a hydroxy group, an alkoxy group, an amino group, a monovalent group including a carbonyl group, an alkylthio group, or an arylthio group, and two or more of $R^{1a}$, $R^{2a}$, $R^{3a}$, $R^{4a}$, and $R^{5a}$ are groups other than a hydrogen atom. $R^{7a}$ represents a hydrogen atom, an alkenyl group, an aryl group, a hydroxy group, an alkoxy group, an amino group, a monovalent group including a carbonyl group, an alkylthio group, or an arylthio group.

In addition, the methacrylate-containing composition may contain other compounds (component C) or water as long as the concentration of the methacrylic acid satisfies 98.00% to 99.99% by mass. Hereinafter, each item will be described in detail.

(Methacrylic acid)

The methacrylate-containing composition according to the first aspect comprises methacrylic acid. The methacrylic acid can be produced, for example, as a by-product of an acetone cyanohydrin (ACH) method or an intermediate of a C4 direct oxidation method. The methacrylic acid contained in the methacrylate-containing composition is preferably produced by a C4 direct oxidation method, and more preferably produced by a C4 direct oxidation method using isobutanol derived from a biomass as a starting raw material.

(Component A1)

The methacrylate-containing composition according to the first aspect comprises the component A1 which is the compound represented by Formula (11). By coexisting the component A1 and the component B described later, it is possible to suppress decomposition of the polymerization inhibitor during storage of the methacrylate-containing composition. The reason for this is presumed to be as follows.

It is known that the methacrylic acid is polymerized by radicals generated during storage, and the polymerization inhibitor (component B) is added to prevent the polymerization. The component B has a function of preventing polymerization by trapping radicals generated during the storage of methacrylic acid, but the polymerization inhibitor is decomposed into another compound when trapping the radicals. Therefore, the concentration of the component B in the methacrylate-containing composition gradually decreases during the storage, and the function of preventing polymerization gradually decreases. Furthermore, unnecessary decomposition products generated by trapping the radicals by the component B gradually increase. Examples of the radicals generated during the storage of methacrylic acid include a hydroxyl radical generated by an oxygen molecule absorbing ultraviolet light derived from sunlight. Since the component A1 is a $\pi$-conjugated compound having a benzene ring, the component A1 absorbs ultraviolet light, and an absorption wavelength and an absorption intensity thereof change depending on the type of the substituent. In the component A1, since an alkyl group having an appropriate bulkiness and an appropriate electron-donating property is bonded to a benzene ring, ultraviolet light in a wide wavelength range can be absorbed. Therefore, when the methacrylate-containing composition comprises the component A1, the ultraviolet light having a wide wavelength range is absorbed, and the generation of the hydroxyl radical is suppressed. Accordingly, since the number of radicals which need to be trapped by the component B is reduced, the decrease in concentration of the component B and the generation of the decomposition product can be suppressed.

The molecular weight of the component A1 is preferably 2,000 or less. When the molecular weight thereof is 2,000 or less, the number of benzene rings per unit mass in the component A1 can be increased, and thus the effect of the present invention can be obtained with a small mass. The molecular weight of the component A1 is more preferably 1,600 or less, still more preferably 1,200 or less, and particularly preferably 800 or less.

$R^{1a}$, $R^{2a}$, $R^{3a}$, $R^{4a}$, $R^{5a}$, and $R^{6a}$ in Formula (11) each independently represent a hydrogen atom, an alkyl group, an alkenyl group, a hydroxy group, an alkoxy group, an amino group, a monovalent group including a carbonyl group, an alkylthio group, or an arylthio group. In addition, $R^{7a}$ in Formula (11) is a hydrogen atom, an alkenyl group, an aryl group, a hydroxy group, an alkoxy group, an amino group, a monovalent group including a carbonyl group, an alkylthio group, or an arylthio group, and two or more of $R^{1a}$, $R^{2a}$, $R^{3a}$, $R^{4a}$, and $R^{5a}$ are groups other than a hydrogen atom. $R^{7a}$ represents a hydrogen atom, an alkenyl group, an aryl group, a hydroxy group, an alkoxy group, an amino group, a monovalent group including a carbonyl group, an alkylthio group, or an arylthio group. $R^{1a}$, $R^{2a}$, $R^{3a}$, $R^{4a}$, $R^{5a}$, $R^{6a}$, and $R^{7a}$ may be the same or different from each other.

In Formula (11), when steric hindrance of $R^{1a}$, $R^{2a}$, $R^{3a}$, $R^{4a}$, $R^{5a}$, $R^{6a}$, and $R^{7a}$ is large, the benzene ring is distorted, and the $\pi$-conjugated system is no longer maintained. However, when $R^{1a}$, $R^{2a}$, $R^{3a}$, $R^{4a}$, $R^{5a}$, $R^{6a}$, and $R^{7a}$ satisfy the above-described conditions, the $\pi$-conjugated system of the component A1 is maintained due to appropriate bulkiness, and thus a property of absorbing ultraviolet light in a wide wavelength range is provided, and the effect of the present invention can be obtained. From the viewpoint of increasing absorbance of the ultraviolet light to suppress the generation of the hydroxyl radical, $R^{1a}$, $R^{2a}$, $R^{3a}$, $R^{4a}$, $R^{5a}$, and $R^{6a}$ are preferably a hydrogen atom, an alkyl group having 1 to 5 carbon atoms, an alkenyl group having 2 to 5 carbon atoms, a hydroxy group, an alkoxycarbonyl group having 1 to 6 carbon atoms, an amino group having 0 to 6 carbon atoms, or a monovalent group including a carbonyl group having 1 to 6 carbon atoms; more preferably a hydrogen atom, an alkyl group having 1 to 5 carbon atoms, a hydroxy group, or an alkoxycarbonyl group having 1 to 6 carbon atoms; still more preferably a hydrogen atom, a methyl group, an ethyl group, a n-propyl group, an isopropyl group, an n-butyl group, an s-butyl group, an i-butyl group, a hydroxy group, or a methoxy group; and particularly preferably a hydrogen atom, a hydroxy group, or a methoxy group. In addition, from the viewpoint of

increasing absorbance of the ultraviolet light to suppress the generation of the hydroxyl radical, $R^{7a}$ is preferably a hydrogen atom, an alkenyl group having 2 to 5 carbon atoms, an aryl group having 6 to 12 carbon atoms, a hydroxy group, an alkoxycarbonyl group having 1 to 6 carbon atoms, an amino group having 0 to 6 carbon atoms, or a monovalent group including a carbonyl group having 1 to 6 carbon atoms; more preferably a hydrogen atom, a carboxy group, or an alkoxycarbonyl group having 2 to 6 carbon atoms; and still more preferably a hydrogen atom, a carboxy group, or a methoxycarbonyl group.

The alkyl group is a chain (linear or branched) alkyl group or a cyclic alkyl group. An alkyl group having 1 to 20 carbon atoms is preferable, an alkyl group having 1 to 10 carbon atoms is more preferable, and an alkyl group having 1 to 5 carbon atoms is still more preferable. Examples of the linear alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, an isopropyl group, or a t-butyl group is preferable. In addition, examples of the cyclic alkyl group include a cyclopentyl group, a cyclohexyl group, and a cyclooctyl group.

The alkenyl group is a chain (linear or branched) alkenyl group or a cyclic alkenyl group. An alkenyl group having 2 to 20 carbon atoms is preferable, an alkenyl group having 2 to 10 carbon atoms is more preferable, and an alkenyl group having 2 to 5 carbon atoms is still more preferable. Examples of the chain alkenyl group include a vinyl group, a 1-propenyl group, an isopropenyl group, a 2-butenyl group, a 1,3-butadienyl group, a 2-pentenyl group, and a 2-hexenyl group. In addition, examples of the cyclic alkenyl group include a cyclopentenyl group and a cyclohexenyl group.

The aryl group is preferably an aryl group having 1 to 20 carbon atoms, and more preferably an aryl group having 1 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The alkoxy group is preferably an alkoxy group having 1 to 20 carbon atoms, more preferably an alkoxy group having 1 to 10 carbon atoms, and still more preferably an alkoxy group having 1 to 6 carbon atoms. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group ($-NH_2$) having no substituent on a nitrogen atom (number of carbon atoms: 0) and an amino group in which a part or all of hydrogen atoms bonded to the nitrogen atom are substituted with carbon atoms. The number of carbon atoms in the amino group in which a part or all of hydrogen atoms bonded to the nitrogen atom are substituted with carbon atoms is preferably 1 to 20, more preferably 1 to 10, and still more preferably 1 to 6. Examples of the amino group include an amino group having no substituent ($-NH_2$), a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group include a formyl group, an acyl group, a carboxy group, an amide group, an alkoxycarbonyl group, a thiocarboxy group, and a thioester group; and a monovalent group including a carbonyl group having 1 to 6 carbon atoms is preferable.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6. Examples of the acyl group include an acetyl group, a propionyl group, a butylcarbonyl group, a vinylcarbonyl group, and a benzoyl group.

The amide group includes an amide group ($-CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group. The total number of carbon atoms derived from the carbonyl group and carbon atoms derived from the alkoxy group is preferably 2 to 20, more preferably 2 to 10, and still more preferably 2 to 6. Examples of the alkoxycarbonyl group include a methoxycarbonyl group, an ethoxycarbonyl group, a butoxycarbonyl group, and a phenoxycarbonyl

group.

The thioester group is a substituent in which a carbonyl group is linked to an alkylthio group or an arylthio group. The total number of carbon atoms derived from the carbonyl group and carbon atoms derived from the alkylthio group or the arylthio group is preferably 2 to 20, more preferably 2 to 10, and still more preferably 2 to 6. Examples of the thioester group include a methylthiocarbonyl group, an ethylthiocarbonyl group, a butylthiocarbonyl group, and a phenylthiocarbonyl group.

In addition, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

The alkylthio group is preferably an alkylthio group having 1 to 20 carbon atoms, more preferably an alkylthio group having 1 to 10 carbon atoms, and still more preferably an alkylthio group having 1 to 5 carbon atoms. Examples of the alkylthio group include a methylthio group, an ethylthio group, a propylthio group, and an isopropylthio group.

The arylthio group is preferably an arylthio group having 1 to 20 carbon atoms, more preferably an arylthio group having 3 to 10 carbon atoms, and still more preferably an arylthio group having 6 to 10 carbon atoms. Examples of the arylthio group include a phenylthio group and a tolylthio group.

Among the compounds satisfying the above-described conditions, from the viewpoint of quality stability of the methacrylate-containing composition during storage, as the component A1, methyl 2-phenylisobutyrate, 2-phenylisobutyric acid, 2-isopropylhydroquinone, 2-isopropyl-4-methoxyphenol, 2-methyl-2-phenylpropionamide, 2-(2-hydroxy-5-methoxyphenyl)-2-methylpropionic acid, 2-(2-methoxy-5-hydroxyphenyl)-2-methylpropionic acid, 2-(2,5-dihydroxyphenyl)-2-methylpropionic acid, methyl 2-(2-hydroxy-5-methoxyphenyl)-2-methylpropionate, methyl 2-(2-methoxy-5-hydroxyphenyl)-2-methylpropionate, or methyl 2-(2,5-dihydroxyphenyl)-2-methylpropionate is preferable; methyl 2-phenylisobutyrate, 2-phenylisobutyric acid, 2-isopropylhydroquinone, 2-isopropyl-4-methoxyphenol, 2-(2-hydroxy-5-methoxyphenyl)-2-methylpropionic acid, or 2-(2-methoxy-5-hydroxyphenyl)-2-methylpropionic acid is more preferable; methyl 2-phenylisobutyrate, 2-phenylisobutyric acid, 2-isopropylhydroquinone, 2-isopropyl-4-methoxyphenol, or 2-(2-hydroxy-5-methoxyphenyl)-2-methylpropionic acid is still more preferable; and methyl 2-phenylisobutyrate, 2-phenylisobutyric acid, 2-isopropylhydroquinone, or 2-isopropyl-4-methoxyphenol is particularly preferable.

The component A1 may be one kind or two or more kinds.

(Component B)

The methacrylate-containing composition according to the first aspect comprises the component B which is a polymerization inhibitor. The polymerization inhibitor means a compound having a function of suppressing the polymerization reaction of the methacrylic acid. Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress a polymerization reaction of the methacrylic acid by a radical polymerization mechanism during the storage of the methacrylic acid. In addition, the component B can trap the above-described hydroxyl radical generated during the storage of the methacrylic acid. That is, when the methacrylate-containing composition comprises the component B in addition to the component A1, the amount of the hydroxyl radical can be reduced by two different mechanisms of suppressing the generation of the hydroxyl radical by the component A1 and removing the generated hydroxyl radical by the component B. Therefore, it is considered that the reduction of the component B and the generation of the decomposition product can be efficiently suppressed.

Examples of the polymerization inhibitor as the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, tris(nonylphenyl)phosphite, 4,4'-isopropylidenediphenol alkylphosphite, tris(2,4-di-tert-butylphenyl)phosphite, tris(biphenyl) phosphite, distearyl pentaerythritol diphosphite, di(2,4-di-tert-butylphenyl)pentaerythritol diphosphite, di(nonylphenyl) pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among the above, from the viewpoint of quality stability of the methacrylate-containing composition during storage, as the component B, at least one polymerization inhibitor selected from the group consisting of a phenol-based compound, an N-oxyl-based compound, an amine-based compound, a phosphorus-containing compound, and a sulfur-containing compound is preferable, and for example, at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is preferable. As the component B, a phenol-based compound is more preferable, and for example, at least one polymerization inhibitor selected from the group consisting of hydroquinone, 4-methoxyphenol, 2,4-dimethyl-6-t-butylphenol, and 2,6-di-t-butyl-4-methylphenol is more preferable. As the component B, for example, at least one polymerization inhibitor selected from the group consisting of hydroquinone, 4-methoxyphenol, and 2,6-di-t-butyl-4-methylphenol is still more preferable. As the component B, for example, at least one polymerization inhibitor selected from the group consisting of hydroquinone and 4-methoxyphenol is particularly preferable.

The component B may be one kind or two or more kinds. When two or more kinds of the components B are contained, the total amount thereof is defined as the contained amount of the component B.

When the methacrylate-containing composition comprises a compound corresponding to both the component A1 and the component B, the compound is regarded as the component B. That is, the methacrylate-containing composition needs to contain another component A1 different from the compound. When the methacrylate-containing composition comprises two or more kinds of compounds corresponding to both the component A1 and the component B, a compound having the highest molar concentration in the methacrylate-containing composition is regarded as the component B, and the other compounds are regarded as the component A1.

(Concentrations of component A1 and component B)

When the concentration of the component A1 is indicated by $M_{A1}$ (μmol/L) and the concentration of the component B is indicated by $M_B$ (μmol/L), from the viewpoint of efficiency of suppressing consumption of the polymerization inhibitor, $M_B/M_{A1}$ is preferably 0.0005 to 100, more preferably 0.001 to 85, still more preferably 0.005 to 70, and particularly preferably 0.005 to 60.

$M_{A1}$ is preferably 1 to 500,000 μmol/L. When $M_{A1}$ is 1 μmol/L or more, the effect of suppressing the consumption of the polymerization inhibitor can be sufficiently obtained. In addition, when the $M_{A1}$ is 500,000 μmol/L or less, the amount of impurities when a methacrylic acid polymer is produced by polymerization of the methacrylate-containing composition according to the first aspect can be reduced, and thus it is possible to prevent physical properties of the polymer from being adversely affected. The lower limit of $M_{A1}$ is more preferably 10 μmol/L or more, still more preferably 30 μmol/L or more, even more preferably 50 μmol/L or more, and particularly preferably 70 μmol/L or more. The upper limit of $M_{A1}$ is more preferably 450,000 μmol/L or less, still more preferably 400,000 μmol/L or less, particularly preferably 350,000 μmol/L or less, and especially preferably 300,000 μmol/L or less.

$M_B$ is preferably 1 to 50,000 μmol/L. When $M_B$ is 1 μmol/L or more, the effect of suppressing the consumption of the polymerization inhibitor can be sufficiently obtained. In addition, when the $M_B$ is 50,000 μmol/L or less, the amount of impurities when a methacrylic acid polymer is produced by polymerization of the methacrylate-containing composition according to the first aspect can be reduced, and thus it is possible to prevent physical properties of the polymer from being adversely affected. The lower limit of $M_B$ is more preferably 10 μmol/L or more, still more preferably 100 μmol/L or more, particularly preferably 1,000 μmol/L or more, and especially preferably 2,000 μmol/L or more. The upper limit of $M_B$ is more preferably 40,000 μmol/L or less, still more preferably 30,000 μmol/L or less, and particularly preferably 25,000 μmol/L or less.

(Concentration of methacrylic acid)

The concentration of the methacrylic acid in the methacrylate-containing composition according to the first aspect is 98.00% to 99.99% by mass. When the concentration of the methacrylic acid is 98.00% by mass or more, the amount of impurities when a methacrylic acid polymer is produced by polymerization of the methacrylate-containing composition according to the first aspect can be reduced, and thus it is possible to prevent physical properties of the polymer from being adversely affected. In addition, when the concentration of the methacrylic acid is 99.99% by mass or less, a purification cost can be reduced. The lower limit of the concentration of the methacrylic acid is more preferably 98.50% by mass or more, still more preferably 99.00% by mass or more, particularly preferably 99.50% by mass or more, and most preferably 99.80% by mass or more.

(Component C)

The methacrylate-containing composition according to the first aspect may further comprise other compounds as the component C as long as the concentration of the methacrylic acid satisfies 98.00% to 99.99% by mass. Examples of the component C include impurities generated in the process of producing the methacrylic acid. For example, the methacrylic acid may contain diacetyl as an impurity, but from the viewpoint of reducing coloration of the methacrylate-containing

composition, the concentration of the diacetyl is preferably 55 μmol/L or less, more preferably 20 μmol/L or less, still more preferably 10 μmol/L or less, and particularly preferably 1 μmol/L or less.

(Analysis of methacrylate-containing composition)

The fact that the methacrylate-containing composition comprises the component A1, the component B, the component C, and the water can be confirmed by, for example, GC-MS measurement. In a GC-MS chart of the methacrylate-containing composition, when a peak is present at the same retention time as a sample of the component A1 and an m/z value detected in a mass spectrum of the peak matches exact mass of the component A1, it can be determined that the methacrylate-containing composition comprises the component A1. When the sample of the component A1 cannot be obtained, when the pattern of the mass spectrum of the peak appearing in the GC-MS chart of the methacrylate-containing composition and the pattern of a mass spectrum of the component A1 in mass spectrum database and match each other, it can be determined that the peak is the peak of the component A1. That is, it can be determined that the methacrylate-containing composition comprises the component A1. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

In addition, the concentration of the methacrylic acid can be calculated, for example, by performing GC-FID measurement of the methacrylate-containing composition, quantifying by an area percentage method, and correcting the quantified moisture concentration using a Karl Fischer moisture meter. The concentration of the component A1 can be quantified, for example, by performing GC measurement of the methacrylate-containing composition and using an internal standard method. When a sample of the component A1 cannot be obtained and the component A1 cannot be quantified by the internal standard method, the concentration of the component A1 can be calculated using the following expression by performing GC-FID measurement on any organic compound having a known concentration under the same conditions as those of the methacrylate-containing composition.

$$\text{Concentration of component A1 } (\mu\text{mol/L}) = \frac{N}{N_{A1}} \times \frac{S_{A1}}{S} \times M$$

Here, N is the number of carbon atoms in one molecule of the organic compound having a known concentration, $N_{A1}$ is the number of carbon atoms in one molecule of the component A1, $S_{A1}$ is a peak area of the component A1, S is a peak area of the organic compound having a known concentration, and M is the concentration (μmol/L) of the organic compound having a known concentration.

When the volatility is low and the quantification cannot be performed by the GC measurement, the quantification can be performed using a chromatography method such as LC.

Concentrations of the component B and the component C can also be calculated by the same method as that for the component A1 described above.

In addition, the fact that the methacrylate-containing composition comprises water, and the concentration thereof can be confirmed by Karl Fischer method.

[Method for producing methacrylate-containing composition]

Examples of a method for producing the methacrylate-containing composition according to the first aspect include a method of adding the component A1 and the component B to methacrylic acid. As the methacrylic acid, a commercially available product may be used, or methacrylic acid produced by a known method such as an acetone cyanohydrin (ACH) method and a C4 direct oxidation method may be used. As the component A1 and the component B, a commercially available product may be used, or a component synthesized by a known method may be used. When a commercially available product containing the component B as methacrylic acid is used, the component B contained in the commercially available product may be used as the component B of the present invention, or another component B may be additionally added. When methacrylic acid produced by a known method such as an acetone cyanohydrin (ACH) method and a C4 direct oxidation method is used, the methacrylate-containing composition may be produced by adding the component A1 or the component B in the middle of the process of a raw material step or a production step. In addition, when the component A1 or the component B is generated as a by-product in the methacrylic acid production process, the methacrylate-containing composition may be produced by leaving a part of the generated component A1 or component B.

[Evaluation method for storage stability and thermal stability]

The methacrylate-containing composition according to the first aspect has high quality stability during storage. Examples of an evaluation method for the quality stability of the methacrylate-containing composition during storage include a method of actually storing the methacrylate-containing composition for a long period of time and confirming the amount of the polymerization inhibitor reduced. In addition, from the viewpoint of ease of work, a method of heating the methacrylate-containing composition for a short period of time and confirming the amount of the polymerization inhibitor reduced may be used. When heating for a short period of time, the heating temperature is preferably 50°C to 100°C and a heating time is preferably 1 to 24 hours. In the present invention, the quality stability of the methacrylate-containing composition during storage is evaluated by the amount of decrease in polymerization inhibitor when the methacrylate-containing composition is stored at 25°C for 21 days.

[Method for producing methacrylic ester according to first aspect]

A method for producing a methacrylic ester according to the first aspect includes a step of esterifying the methacrylic acid in the methacrylate-containing composition according to the first aspect.

The alcohol to be reacted with the methacrylate-containing composition is not particularly limited, and examples thereof include methanol, ethanol, n-propanol, isopropanol, n-butanol, and isobutanol. Examples of the methacrylic ester to be obtained include methyl methacrylate, ethyl methacrylate, propyl methacrylate, isopropyl methacrylate, butyl methacrylate, and isobutyl methacrylate. The esterification reaction can be carried out in the presence of an acidic catalyst such as a sulfonic acid-type cation exchange resin. The temperature during the esterification reaction is preferably 50°C to 200°C.

The pressure during the esterification reaction, the location of the catalyst in the reaction vessel, the proportion of the catalyst in the reaction vessel, and the like are not particularly limited, and a commonly used form can be adopted.

[Method for producing methacrylic acid polymer according to first aspect]

A method for producing a methacrylic acid polymer according to the first aspect includes a step of polymerizing a polymerizable composition comprising the methacrylate-containing composition according to the first aspect.

The polymerizable composition may further comprise a monomer copolymerizable with the methacrylic acid and other additives, as necessary.

Examples of the monomer copolymerizable with the methacrylic acid include the following:

methacrylic esters such as methyl methacrylate, ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;
acrylic esters such as methyl acrylate, ethyl acrylate, n-butyl acrylate, iso-butyl acrylate, tert-butyl acrylate, and 2-ethylhexyl acrylate;
unsaturated carboxylic acids such as acrylic acid, maleic acid, and itaconic acid;
unsaturated carboxylic anhydrides such as maleic anhydride and itaconic anhydride;
maleimides such as N-phenylmaleimide and N-cyclohexylmaleimide;
hydroxy group-containing vinyl monomers such as 2-hydroxyethyl acrylate, 2-hydroxyethyl methacrylate, and 2-hydroxypropyl methacrylate;
vinyl esters such as vinyl acetate and vinyl benzoate;
vinyl chloride, vinylidene chloride, and derivatives thereof;
nitrogen-containing vinyl monomers such as methacrylamide and acrylonitrile;
epoxy group-containing monomers such as glycidyl acrylate and glycidyl methacrylate;
aromatic vinyl monomers such as styrene and $\alpha$-methylstyrene;
alkanediol di(meth)acrylates such as ethylene glycol di(meth)acrylate, 1,2-propylene glycol di(meth)acrylate, 1,3-butylene glycol di(meth)acrylate, and 1,6-hexanediol di(meth)acrylate;
polyoxyalkylene glycol di(meth)acrylates such as diethylene glycol di(meth)acrylate, dipropylene glycol di(meth) acrylate, triethylene glycol (meth)acrylate, tetraethylene glycol di(meth)acrylate, polyethylene glycol di(meth)acrylate, and neopentyl glycol di(meth)acrylate;
vinyl monomers having two or more ethylenically unsaturated bonds in a molecule, such as divinylbenzene;
unsaturated polyester prepolymer obtained from at least one polyvalent carboxylic acid including an ethylenically unsaturated polycarboxylic acid and at least one diol; and
vinyl ester prepolymer obtained by acrylic-modifying a terminal of epoxy group.

Among the above, the monomer copolymerizable with the methacrylic acid is preferably at least one selected from the group consisting of methacrylic ester and acrylic ester. The monomer copolymerizable with the methacrylic acid is more preferably methacrylic ester and particularly preferably methyl methacrylate.

The monomer copolymerizable with the methacrylic acid may be one kind or two or more kinds. In addition, when the component A1 is a monomer copolymerizable with the methacrylic acid, the component A1 may be used as the monomer copolymerizable with the methacrylic acid, or another monomer copolymerizable with the methacrylic acid may be used separately from the component A1.

In the polymerizable composition, a contained amount of the monomer copolymerizable with the methacrylic acid is preferably 50.00 to 99.99 parts by mass with respect to 100 parts by mass of the polymerizable composition. The lower limit of the contained amount of the monomer copolymerizable with the methacrylic acid is more preferably 60 parts by mass or more, still more preferably 70 parts by mass or more, particularly preferably 80 parts by mass or more, and most preferably 90 parts by mass or more with respect to 100 parts by mass of the polymerizable composition. The upper limit of the contained amount of the monomer copolymerizable with the methacrylic acid is more preferably 99.9 parts by mass or less and still more preferably 99 parts by mass or less with respect to 100 parts by mass of the polymerizable composition.

Examples of the other additives include a polymerization initiator, a chain transfer agent, a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent. The other additives may be one kind or two or more kinds.

As the other additives, a polymerization initiator is preferable. Examples of the polymerization initiator include the following:

azo compounds such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate;
organic peroxides such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxyisononanoate, di-t-butyl peroxide, di-t-hexyl peroxide, lauroyl peroxide, and dilauroyl peroxide;
persulfate compounds such as potassium persulfate; and
a redox polymerization initiator.

Among the above, from the viewpoint of storage stability and reactivity with the monomer copolymerizable with the methacrylic acid, the polymerization initiator is preferably at least one selected from the group consisting of an azo compound and an organic peroxide.

The amount of the polymerization initiator used is preferably 0.0001 to 1 part by mass with respect to 100 parts by mass of the total amount of the methacrylic acid and the monomer copolymerizable with the methacrylic acid.

Examples of a polymerization method for the polymerizable composition include a bulk polymerization method, a solution polymerization method, an emulsion polymerization method, and a suspension polymerization method.

The polymerization temperature is preferably 125°C to 210°C. In this manner, an appropriate polymerization rate can be obtained. The lower limit of the polymerization temperature is more preferably 130°C or higher, and the upper limit thereof is more preferably 180°C or lower. The polymerization time is not particularly limited, and can be, for example, 0.5 to 24 hours.

[Methacrylate-containing composition according to second aspect]

As a first embodiment of a second aspect, the methacrylate-containing composition comprises methacrylic acid, a component A21 which is a compound represented by Formula (21), and a polymerization inhibitor (component B). The concentration of the methacrylic acid is 98.00% to 99.99% by mass.

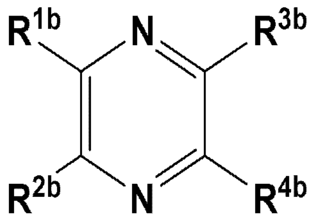

· · · (2 1)

In Formula (21), $R^{1b}$, $R^{2b}$, $R^{3b}$, and $R^{4b}$ each independently represent a hydrogen atom, an alkyl group, an alkenyl group, an aryl group, a hydroxy group, an alkoxy group, an amino group, a monovalent group including a carbonyl group, an alkylthio group, or an arylthio group. In addition, $R^{1b}$ and $R^{2b}$, or $R^{3b}$ and $R^{4b}$ may be linked to each other to form a ring.

In addition, the methacrylate-containing composition may further comprise other compounds (component C) or water as long as the concentration of the methacrylic acid satisfies 98.00% to 99.99% by mass. Hereinafter, each item will be described in detail.

(Methacrylic acid)

The methacrylate-containing composition according to the second aspect comprises methacrylic acid. The methacrylic acid can be produced, for example, as a by-product of an acetone cyanohydrin (ACH) method or an intermediate of a C4 direct oxidation method. The methacrylic acid contained in the methacrylate-containing composition is preferably produced by a C4 direct oxidation method, and more preferably produced by a C4 direct oxidation method using isobutanol derived from a biomass as a starting raw material.

(Component A21)

The methacrylate-containing composition according to the first embodiment of the second aspect comprises the component A21 which is the compound represented by Formula (21). By coexisting the component A21 and the component B described later, it is possible to suppress decomposition of the polymerization inhibitor during storage of the methacrylate-containing composition. The reason for this is presumed to be as follows.

It is known that the methacrylic acid is polymerized by radicals generated during storage, and the polymerization inhibitor (component B) is added to prevent the polymerization. The component B has a function of preventing polymerization by trapping radicals generated during the storage of methacrylic acid, but the polymerization inhibitor is decomposed into another compound when trapping the radicals. Therefore, the concentration of the component B in the methacrylate-containing composition gradually decreases during the storage, and the function of preventing polymerization gradually decreases. Furthermore, unnecessary decomposition products generated by trapping the radicals by the component B gradually increase. Examples of the radicals generated during the storage of methacrylic acid include a hydroxyl radical generated by an oxygen molecule absorbing ultraviolet light derived from sunlight. Since the component A21 has an aromatic ring, the component A21 absorbs the ultraviolet light, and an absorption wavelength thereof changes depending on the type of the substituent. The component A21 having the structure represented by Formula (21) can absorb ultraviolet light in a wide wavelength range. Therefore, when the methacrylate-containing composition comprises the component A21, ultraviolet light in a wide wavelength is absorbed, and the generation of the hydroxyl radical due to the absorption of the ultraviolet light by oxygen molecules is suppressed. Accordingly, since the number of radicals which need to be trapped by the component B is reduced, the decrease in concentration of the component B and the generation of the decomposition product can be suppressed.

The molecular weight of the component A21 is preferably 1,000 or less. In this manner, the number of pyrazine rings per unit mass of the component A21 is increased, and thus the effect of the present invention can be obtained with a small amount of the component A21. The molecular weight of the component A21 is more preferably 800 or less, still more preferably 600 or less, and particularly preferably 400 or less.

**In** Formula (21), $R^{1b}$, $R^{2b}$, $R^{3b}$, and $R^{4b}$ each independently represent a hydrogen atom, an alkyl group, an alkenyl group, an aryl group, a hydroxy group, an alkoxy group, an amino group, a monovalent group including a carbonyl group, an alkylthio group, or an arylthio group. $R^{1b}$, $R^{2b}$, $R^{3b}$, and $R^{4b}$ may be the same or different from each other.

From the viewpoint of increasing absorbance of the ultraviolet light to suppress the generation of the hydroxyl radical, $R^{1b}$, $R^{2b}$, $R^{3b}$, and $R^{4b}$ are preferably a hydrogen atom, an alkyl group having 1 to 5 carbon atoms, an alkenyl group having 2 to 5 carbon atoms, an aryl group having 6 to 12 carbon atoms, an alkoxy group having 1 to 6 carbon atoms, an amino group having 0 to 6 carbon atoms, a monovalent group including a carbonyl group having 1 to 6 carbon atoms, an alkylthio group having 1 to 5 carbon atoms, or an arylthio group having 6 to 10 carbon atoms; more preferably a hydrogen atom, an alkyl group having 1 to 5 carbon atoms, an alkoxy group having 1 to 6 carbon atoms, a monovalent group including a carbonyl group having 1 to 6 carbon atoms, or an alkylthio group having 6 to 10 carbon atoms; still more preferably a

hydrogen atom, a methyl group, an ethyl group, an n-propyl group, an isopropyl group, a methylthio group, a methoxy group, a carboxy group, a carbomethoxy group, or a hydroxymethyl group; and particularly preferably a hydrogen atom, a methyl group, an isopropyl group, a methylthio group, a methoxy group, a carboxy group, a carbomethoxy group, or a hydroxymethyl group.

The alkyl group is a chain (linear or branched) alkyl group or a cyclic alkyl group. An alkyl group having 1 to 20 carbon atoms is preferable, an alkyl group having 1 to 10 carbon atoms is more preferable, and an alkyl group having 1 to 5 carbon atoms is still more preferable. Examples of the linear alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, or an isopropyl group is preferable. In addition, examples of the cyclic alkyl group include a cyclopentyl group, a cyclohexyl group, and a cyclooctyl group.

The alkenyl group is a chain (linear or branched) alkenyl group or a cyclic alkenyl group. An alkenyl group having 2 to 20 carbon atoms is preferable, an alkenyl group having 2 to 10 carbon atoms is more preferable, and an alkenyl group having 2 to 5 carbon atoms is still more preferable. Examples of the chain alkenyl group include a vinyl group, a 1-propenyl group, an isopropenyl group, a 2-butenyl group, a 1,3-butadienyl group, a 2-pentenyl group, and a 2-hexenyl group. In addition, examples of the cyclic alkenyl group include a cyclopentenyl group and a cyclohexenyl group.

The aryl group is preferably an aryl group having 1 to 20 carbon atoms, and more preferably an aryl group having 1 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The alkoxy group is preferably an alkoxy group having 1 to 20 carbon atoms, more preferably an alkoxy group having 1 to 10 carbon atoms, still more preferably an alkoxy group having 1 to 6 carbon atoms, and particularly preferably an alkoxy group having 1 to 5 carbon atoms. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group ($-NH_2$) having no substituent on a nitrogen atom (number of carbon atoms: 0) and an amino group in which a part or all of hydrogen atoms bonded to the nitrogen atom are substituted with carbon atoms. The number of carbon atoms in the amino group in which a part or all of hydrogen atoms bonded to the nitrogen atom are substituted with carbon atoms is preferably 1 to 20, more preferably 1 to 10, and still more preferably 1 to 6. Examples of the amino group include an amino group having no substituent ($-NH_2$), a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group include a formyl group, an acyl group, a carboxy group, an amide group, an alkoxycarbonyl group, a thiocarboxy group, and a thioester group; and a monovalent group including a carbonyl group having 1 to 6 carbon atoms is preferable.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6. Examples of the acyl group include an acetyl group, a propionyl group, a butylcarbonyl group, a vinylcarbonyl group, and a benzoyl group.

The amide group includes an amide group ($-CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and the number of carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group. The total number of carbon atoms derived from the carbonyl group and carbon atoms derived from the alkoxy group is preferably 2 to 20, more preferably 2 to 10, and still more preferably 2 to 6. Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group.

The total number of carbon atoms derived from the carbonyl group and carbon atoms derived from the alkylthio group or the arylthio group is preferably 2 to 20, more preferably 2 to 10, and still more preferably 2 to 6. Examples of the thioester group include a methylthiocarbonyl group, an ethylthiocarbonyl group, a butylthiocarbonyl group, and a phenylthiocarbonyl group.

In addition, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

The alkylthio group is preferably an alkylthio group having 1 to 20 carbon atoms, more preferably an alkylthio group having 1 to 10 carbon atoms, and still more preferably an alkylthio group having 1 to 5 carbon atoms. Examples of the alkylthio group include a methylthio group, an ethylthio group, a propylthio group, and an isopropylthio group.

The arylthio group is preferably an arylthio group having 1 to 20 carbon atoms, more preferably an arylthio group having 3 to 10 carbon atoms, and still more preferably an arylthio group having 6 to 10 carbon atoms. Examples of the arylthio group include a phenylthio group and a tolylthio group.

Among the compounds satisfying the above-described conditions, from the viewpoint of quality stability of the methacrylate-containing composition during storage, as the component A21, 2,3,5,6-tetramethylpyrazine, pyrazine, 2,3,5-trimethylpyrazine, 2-methoxypyrazine, 2-isopropyl-3-methoxypyrazine, 2,5-dimethylpyrazine, 2-aminopyrazine, methyl 2-pyrazinecarboxylate, 2-(methylthio)pyrazine, 2-pyrazinemethanol, quinoxaline, 2-vinylpyrazine, 2,5-diisopropylpyrazine, 2-ethyl-3,5-dimethylpyrazine, 2,5-dimethyl-3-isobutylpyrazine, 2-isopropyl-3-methoxy-5-isobutylpyrazine, 2,5-dimethyl-3-(methylthio)pyrazine, pyrazine methylamine, 2-phenylpyrazine, 5,6,7,8-tetrahydroquinoxaline, phenazine, or 1,2,3,4,6,7,8,9-octahydrophenazine is preferable; 2,3,5,6-tetramethylpyrazine, pyrazine, 2,3,5-trimethylpyrazine, 2-methoxypyrazine, 2-isopropyl-3-methoxypyrazine, 2,5-dimethylpyrazine, 2-aminopyrazine, methyl 2-pyrazinecarboxylate, 2-(methylthio)pyrazine, 2-pyrazinemethanol, quinoxaline, or 2-vinylpyrazine is more preferable; and 2,3,5,6-tetramethylpyrazine, pyrazine, 2,3,5-trimethylpyrazine, 2-methoxypyrazine, 2-isopropyl-3-methoxypyrazine, 2,5-dimethylpyrazine, methyl 2-pyrazinecarboxylate, 2-(methylthio)pyrazine, or 2-pyrazinemethanol is still more preferable.

The component A21 may be one kind or two or more kinds.

(Component B)

The methacrylate-containing composition according to the first embodiment of the second aspect comprises the component B. The component B is a compound acting as a polymerization inhibitor. In the present specification, the polymerization inhibitor means a compound having a function of suppressing the polymerization reaction of the methacrylic acid. Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress a polymerization reaction of the methacrylic acid by a radical polymerization mechanism during the storage of the methacrylic acid. In addition, the component B can trap the above-described hydroxyl radical generated during the storage of the methacrylic acid. That is, when the methacrylate-containing composition comprises the component B in addition to the component A21, the amount of the hydroxyl radical can be reduced by two different mechanisms of suppressing the generation of the hydroxyl radical by the component A21 and removing the generated hydroxyl radical by the component B. Therefore, it is considered that the reduction of the component B can be efficiently suppressed.

Examples of the polymerization inhibitor as the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, tris(nonylphenyl)phosphite, 4,4'-isopropylidenediphenol alkylphosphite, tris(2,4-di-tert-butylphenyl)phosphite, tris(biphenyl) phosphite, distearyl pentaerythritol diphosphite, di(2,4-di-tert-butylphenyl)pentaerythritol diphosphite, di(nonylphenyl) pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among the above, from the viewpoint of quality stability of the methacrylate-containing composition during storage, as the component B, at least one polymerization inhibitor selected from the group consisting of a phenol-based compound, an N-oxyl-based compound, an amine-based compound, a phosphorus-containing compound, and a sulfur-containing compound is preferable, and for example, at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is preferable. As the component B, a phenol-based compound is more preferable, and for example, at least one polymerization inhibitor selected from the group consisting of hydroquinone, 4-methoxyphenol, 2,4-dimethyl-6-t-butylphenol, and 2,6-di-t-butyl-4-methylphenol is more preferable. As the component B, a phenol-based compound is more preferable, and for example, at least one polymerization inhibitor selected from the group consisting of hydroquinone and 4-methoxyphenol is still more preferable.

The component B may be one kind or two or more kinds.

When the methacrylate-containing composition comprises a compound corresponding to both the component A21 and the component B, the compound is regarded as the component B. That is, the methacrylate-containing composition needs to contain another component A21 different from the compound. When the methacrylate-containing composition comprises two or more kinds of compounds corresponding to both the component A21 and the component B, a compound having the highest molar concentration in the methacrylate-containing composition is regarded as the component B, and the other compounds are regarded as the component A21.

(Concentrations of component A21 and component B)

When the concentration of the component A21 is indicated by $M_{A21}$ (μmol/L) and the concentration of the component B is indicated by $M_B$ (μmol/L), from the viewpoint of suppressing the generation of pyruvic acid, $M_B/M_{A21}$ is preferably 0.005 to 100, more preferably 0.01 to 80, still more preferably 0.05 to 60, and particularly preferably 0.1 to 40.

$M_{A21}$ is preferably 1 to 50,000 μmol/L. When $M_{A21}$ is 1 μmol/L or more, the effect of suppressing the decomposition of the polymerization inhibitor can be sufficiently obtained. In addition, when the $M_{A21}$ is 50,000 μmol/L or less, the amount of impurities when a methacrylic acid polymer is produced by polymerization of the methacrylate-containing composition according to the second aspect can be reduced, and thus it is possible to prevent physical properties of the polymer from being adversely affected. The lower limit of $M_{A21}$ is more preferably 10 μmol/L or more, still more preferably 30 μmol/L or more, particularly preferably 50 μmol/L or more, and especially preferably 60 μmol/L or more. The upper limit of $M_{A21}$ is more preferably 30,000 μmol/L or less, still more preferably 10,000 μmol/L or less, particularly preferably 5,000 μmol/L or less, especially preferably 2,500 μmol/L or less, and most preferably 500 μmol/L or less.

$M_B$ is preferably 1 to 50,000 μmol/L. When $M_B$ is 1 μmol/L or more, the effect of suppressing the decomposition of the polymerization inhibitor can be sufficiently obtained. In addition, when the $M_B$ is 50,000 μmol/L or less, the amount of impurities when a methacrylic acid polymer is produced by polymerization of the methacrylate-containing composition according to the second aspect can be reduced, and thus it is possible to prevent physical properties of the polymer from being adversely affected. The lower limit of $M_B$ is more preferably 10 μmol/L or more, still more preferably 100 μmol/L or more, particularly preferably 1,000 μmol/L or more, especially preferably 1,500 μmol/L or more, and most preferably 2,000 μmol/L or more. The upper limit of $M_B$ is more preferably 10,000 μmol/L or less, still more preferably 5,000 μmol/L or less, particularly preferably 3,000 μmol/L or less, and especially preferably 2,500 μmol/L or less.

(Concentration of methacrylic acid)

In the first embodiment, the concentration of the methacrylic acid in the methacrylate-containing composition according to the second aspect is 98.00% to 99.99% by mass. When the concentration of the methacrylic acid is 98.00% by mass or more, the amount of impurities when a methacrylic acid polymer is produced by polymerization of the methacrylate-containing composition according to the second aspect can be reduced, and thus it is possible to prevent physical properties of the polymer from being adversely affected. In addition, when the concentration of the methacrylic acid is 99.99% by mass or less, a purification cost can be reduced. The lower limit of the concentration of the methacrylic acid is more preferably 98.50% by mass or more, still more preferably 99.00% by mass or more, particularly preferably 99.50% by mass or more, and most preferably 99.80% by mass or more.

(Component C)

The methacrylate-containing composition according to the first embodiment of the second aspect may further comprise other compounds as the component C as long as the concentration of the methacrylic acid satisfies 98.00% to 99.99% by mass. Examples of the component C include impurities generated in the process of producing the methacrylic acid. For example, the methacrylic acid may contain diacetyl as an impurity, but from the viewpoint of reducing coloration of the methacrylate-containing composition, the concentration of the diacetyl is preferably 55 μmol/L or less, more preferably 20 μmol/L or less, still more preferably 10 μmol/L or less, and particularly preferably 1 μmol/L or less.

(Analysis of methacrylate-containing composition)

The fact that the methacrylate-containing composition comprises the component A21, the component B, the component C, and the water can be confirmed by, for example, GC-MS measurement. In a GC-MS chart of the methacrylate-containing composition, when a peak is present at the same retention time as a sample of the component A21 and an m/z value detected in a mass spectrum of the peak matches exact mass of the component A21, it can be determined that the methacrylate-containing composition comprises the component A21. When the sample of the component A21 cannot be obtained, when a pattern of the mass spectrum of the peak appearing in the GC-MS chart of the methacrylate-containing composition and a pattern of a mass spectrum of the component A21 in mass spectrum database and match each other, it can be determined that the peak is the peak of the component A21. That is, it can be determined that the methacrylate-containing composition comprises the component A21. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

In addition, the concentration of the methacrylic acid can be calculated, for example, by performing GC-FID measurement of the methacrylate-containing composition, quantifying by an area percentage method, and correcting the quantified moisture concentration using a Karl Fischer moisture meter. The concentration of the component A21 can be quantified, for example, by performing GC measurement of the methacrylate-containing composition and using an internal standard method. When a sample of the component A21 cannot be obtained and the component A21 cannot be quantified by the internal standard method, the concentration of the component A21 can be calculated using the following expression by performing GC-FID measurement on any organic compound having a known concentration under the same conditions as those of the methacrylate-containing composition.

$$\text{Concentration of component A21 } (\mu\text{mol/L}) = \frac{N}{N_{A21}} \times \frac{S_{A21}}{S} \times M$$

Here, N is the number of carbon atoms in one molecule of the organic compound having a known concentration, $N_{A21}$ is the number of carbon atoms in one molecule of the component A21, $S_{A21}$ is a peak area of the component A21, S is a peak area of the organic compound having a known concentration, and M is the concentration (μmol/L) of the organic compound having a known concentration.

When the volatility is low and the quantification cannot be performed by the GC measurement, the quantification can be performed using a chromatography method such as LC.

Concentrations of the component B and the component C can also be calculated by the same method as that for the component A21 described above.

In addition, the fact that the methacrylate-containing composition comprises water, and the concentration thereof can be confirmed by Karl Fischer method.

(Component B)

In a second embodiment of the second aspect, it is preferable that the methacrylate-containing composition comprises a component B. A preferred aspect of the component B is the same as that in the first embodiment. In addition, the component B may be one kind or two or more kinds.

(Concentration of component B)

A preferred aspect of $M_B$ is the same as that in the first embodiment.

(Concentration of methacrylic acid)

A preferred concentration of the methacrylic acid is the same as that in the first embodiment.

(Component C)

Details and preferred aspects of the component C are the same as those in the first embodiment.

(Analysis of methacrylate-containing composition)

A method of confirming that the methacrylate-containing composition comprises the component B, the component C, and water, and a method of measuring the concentrations of the methacrylic acid, the component B, the component C, and water are the same as those in the first embodiment.

[Method for producing methacrylate-containing composition]

Examples of a method for producing the methacrylate-containing composition according to the second aspect include a method of adding the component A21 and, as necessary, the component B to methacrylic acid. As the methacrylic acid, a commercially available product may be used, or methacrylic acid produced by a known method such as an acetone cyanohydrin (ACH) method and a C4 direct oxidation method may be used. As the component A21 and the component B, a commercially available product may be used, or a component synthesized by a known method may be used. When methacrylic acid produced by a known method such as an acetone cyanohydrin (ACH) method and a C4 direct oxidation method is used, the methacrylate-containing composition may be produced by adding the component A21 or the component B in the middle of the process of a raw material step or a production step. In addition, when the component A21 or the component B is generated as a by-product in the methacrylic acid production process, the methacrylate-containing composition may be produced by leaving a part of the generated component A21 or component B.

[Evaluation method for storage stability and thermal stability]

The methacrylate-containing composition according to the second aspect has high quality stability during storage. Examples of an evaluation method for the quality stability of the methacrylate-containing composition during storage include a method of actually storing the methacrylate-containing composition for a long period of time and confirming the amount of the polymerization inhibitor reduced. In addition, from the viewpoint of ease of work, a method of heating the methacrylate-containing composition for a short period of time and confirming the amount of the polymerization inhibitor reduced may be used. When heating for a short period of time, the heating temperature is preferably 50°C to 100°C and a heating time is preferably 1 to 24 hours. In the present invention, the quality stability of the methacrylate-containing composition during storage is evaluated by the amount of decrease in polymerization inhibitor when the methacrylate-containing composition is stored at 25°C for 21 days.

[Method for producing methacrylic ester]

A method for producing a methacrylic ester according to the second aspect includes a step of esterifying the methacrylate-containing composition according to the second aspect.

The alcohol to be reated with the methacrylate-containing composition is not particularly limited, and examples thereof include methanol, ethanol, n-propanol, isopropanol, n-butanol, and isobutanol. Examples of the methacrylic ester to be obtained include methyl methacrylate, ethyl methacrylate, propyl methacrylate, isopropyl methacrylate, butyl methacrylate, and isobutyl methacrylate. The esterification reaction can be carried out in the presence of an acidic catalyst such as a sulfonic acid-type cation exchange resin. The temperature during the esterification reaction is preferably 50°C to 200°C.

The pressure during the esterification reaction, the location of the catalyst in the reaction vessel, the proportion of the catalyst in the reaction vessel, and the like are not particularly limited, and a commonly used form can be adopted.

[Method for producing methacrylic acid polymer according to second aspect]

A method for producing a methacrylic acid polymer according to the second aspect includes a step of polymerizing a polymerizable composition comprising the methacrylate-containing composition according to the second aspect.

The polymerizable composition may further comprise a monomer copolymerizable with the methacrylic acid and other additive substances, as necessary.

Examples of the monomer copolymerizable with the methacrylic acid include the following:

methacrylic esters such as methyl methacrylate, ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;

acrylic esters such as methyl acrylate, ethyl acrylate, n-butyl acrylate, iso-butyl acrylate, tert-butyl acrylate, and 2-ethylhexyl acrylate;

unsaturated carboxylic acids such as acrylic acid, maleic acid, and itaconic acid;
unsaturated carboxylic anhydrides such as maleic anhydride and itaconic anhydride;
maleimides such as N-phenylmaleimide and N-cyclohexylmaleimide;
hydroxy group-containing vinyl monomers such as 2-hydroxyethyl acrylate, 2-hydroxyethyl methacrylate, and 2-hydroxypropyl methacrylate;
vinyl esters such as vinyl acetate and vinyl benzoate;
vinyl chloride, vinylidene chloride, and derivatives thereof;
nitrogen-containing vinyl monomers such as methacrylamide and acrylonitrile;
epoxy group-containing monomers such as glycidyl acrylate and glycidyl methacrylate;
aromatic vinyl monomers such as styrene and $\alpha$-methylstyrene;
alkanediol di(meth)acrylates such as ethylene glycol di(meth)acrylate, 1,2-propylene glycol di(meth)acrylate, 1,3-butylene glycol di(meth)acrylate, and 1,6-hexanediol di(meth)acrylate;
polyoxyalkylene glycol di(meth)acrylates such as diethylene glycol di(meth)acrylate, dipropylene glycol di(meth) acrylate, triethylene glycol (meth)acrylate, tetraethylene glycol di(meth)acrylate, polyethylene glycol di(meth)acrylate, and neopentyl glycol di(meth)acrylate;
vinyl monomers having two or more ethylenically unsaturated bonds in a molecule, such as divinylbenzene;
unsaturated polyester prepolymer obtained from at least one polyvalent carboxylic acid including an ethylenically unsaturated polycarboxylic acid and at least one diol; and
vinyl ester prepolymer obtained by acrylic-modifying a terminal of epoxy group.

Among the above, the monomer copolymerizable with the methacrylic acid is preferably at least one selected from the group consisting of methacrylic ester and acrylic ester. The monomer copolymerizable with the methacrylic acid is more preferably methacrylic ester and particularly preferably methyl methacrylate.

The monomer copolymerizable with the methacrylic acid may be one kind or two or more kinds. In addition, when the component A21 is a monomer copolymerizable with the methacrylic acid, the component A21 may be used as the monomer copolymerizable with the methacrylic acid, or another monomer copolymerizable with the methacrylic acid may be used separately from the component A21.

In the polymerizable composition, a contained amount of the monomer copolymerizable with the methacrylic acid is preferably 50.00 to 99.99 parts by mass with respect to 100 parts by mass of the polymerizable composition. The lower limit of the contained amount of the monomer copolymerizable with the methacrylic acid is more preferably 60 parts by mass or more, still more preferably 70 parts by mass or more, particularly preferably 80 parts by mass or more, and most preferably 90 parts by mass or more with respect to 100 parts by mass of the polymerizable composition. The upper limit of the contained amount of the monomer copolymerizable with the methacrylic acid is more preferably 99.9 parts by mass or less and still more preferably 99 parts by mass or less with respect to 100 parts by mass of the polymerizable composition.

As the other additives, a polymerization initiator is preferable. In addition, the polymerizable composition may further comprise, as necessary, a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, a fluorescent agent, or the like. The other additives may be one kind or two or more kinds.

Examples of the polymerization initiator include the following:

azo compounds such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate;
organic peroxides such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxyisononanoate, di-t-butyl peroxide, di-t-hexyl peroxide, lauroyl peroxide, and dilauroyl peroxide;
persulfate compounds such as potassium persulfate; and
a redox polymerization initiator.

Among the above, from the viewpoint of storage stability and reactivity with the monomer copolymerizable with the methacrylic acid, the polymerization initiator is preferably at least one selected from the group consisting of an azo compound and an organic peroxide.

The amount of the polymerization initiator used is preferably 0.0001 to 1 part by mass with respect to 100 parts by mass of the total amount of the methacrylic acid and the monomer copolymerizable with the methacrylic acid.

Examples of a polymerization method for the polymerizable composition include a bulk polymerization method, a solution polymerization method, an emulsion polymerization method, and a suspension polymerization method.

The polymerization temperature is preferably 125°C to 210°C. In this manner, an appropriate polymerization rate can be obtained. The lower limit of the polymerization temperature is more preferably 130°C or higher, and the upper limit thereof is more preferably 180°C or lower. The polymerization time is not particularly limited, and can be, for example, 0.5 to 24 hours.

[Methacrylate-containing composition according to third aspect]

A methacrylate-containing composition according to a third aspect comprises methacrylic acid and a component A3 which is a compound represented by Formula (31). The concentration of the methacrylic acid is 98.00% to 99.99% by mass.

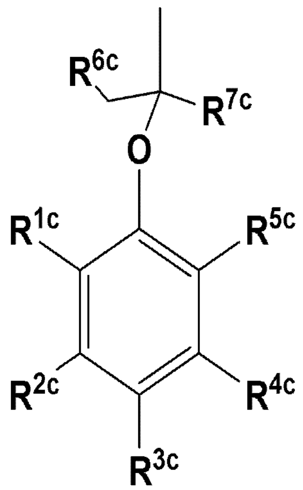

・・・(31)

In Formula (31), $R^{1c}$, $R^{2c}$, $R^{3c}$, $R^{4c}$, $R^{5c}$, $R^{6c}$, and $R^{7c}$ independently represent a hydrogen atom, an alkyl group, an alkenyl group, an aryl group, a hydroxy group, an alkoxy group, an amino group, a monovalent group including a carbonyl group, an alkylthio group, or an arylthio group.

In addition, the methacrylate-containing composition may further comprise a polymerization inhibitor (component B, other compounds (component C), or water as long as the concentration of the methacrylic acid satisfies 98.00% to 99.99% by mass. Hereinafter, each item will be described in detail.

(Methacrylic acid)

The methacrylate-containing composition according to the third aspect comprises methacrylic acid. The methacrylic acid can be produced, for example, as a by-product of an acetone cyanohydrin (ACH) method or an intermediate of a C4 direct oxidation method. The methacrylic acid contained in the methacrylate-containing composition is preferably produced by a C4 direct oxidation method, and more preferably produced by a C4 direct oxidation method using isobutanol derived from a biomass as a starting raw material.

(Component A3)

The methacrylate-containing composition according to the third aspect comprises the compound (component A3) represented by Formula (31). Since the methacrylate-containing composition comprises the component A3, it is possible to suppress decomposition of the polymerization inhibitor during storage of the methacrylate-containing composition. The reason for this is presumed to be as follows.

It is known that the methacrylic acid is polymerized by radicals generated during storage, and the polymerization inhibitor (component B) is added to prevent the polymerization. The component B has a function of preventing polymerization by trapping radicals generated during the storage of methacrylic acid, but the polymerization inhibitor is decomposed into another compound when trapping the radicals. Therefore, the concentration of the component B in the methacrylate-containing composition gradually decreases during the storage, and the function of preventing polymerization gradually decreases. Furthermore, unnecessary decomposition products generated by trapping the radicals by the component B gradually increase. Examples of the radicals generated during the storage of methacrylic acid include a hydroxyl radical generated by an oxygen molecule absorbing ultraviolet light derived from sunlight. Since the component A3 is a $\pi$-conjugated compound having a benzene ring, the component A3 absorbs ultraviolet light, and an absorption wavelength thereof changes depending on the type of the substituent. The component A3 can absorb ultraviolet light in a wide wavelength range. Therefore, when the methacrylate-containing composition comprises the component A3, the ultraviolet light having a wide wavelength range is absorbed, and the generation of the hydroxyl radical is suppressed. Accordingly, since the number of radicals which need to be trapped by the component B is reduced, the decrease in concentration of the component B and the generation of the decomposition product can be suppressed.

The molecular weight of the component A3 is preferably 2,000 or less. When the molecular weight thereof is 2,000 or less, the number of benzene rings per unit mass in the component A3 can be increased, and thus the effect of the present invention can be obtained with a small mass. The molecular weight of the component A3 is more preferably 1,600 or less, still more preferably 1,200 or less, and particularly preferably 800 or less.

$R^{1c}$, $R^{2c}$, $R^{3c}$, $R^{4c}$, $R^{5c}$, $R^{6c}$, and $R^{7c}$ in Formula (31) independently represent a hydrogen atom, an alkyl group, an alkenyl group, an aryl group, a hydroxy group, an alkoxy group, an amino group, a monovalent group including a carbonyl group, an alkylthio group, or an arylthio group. $R^{1c}$, $R^{2c}$, $R^{3c}$, $R^{4c}$, $R^{5c}$, $R^{6c}$, and $R^{7c}$ may be the same or different from each other.

When $R^{1c}$, $R^{2c}$, $R^{3c}$, $R^{4c}$, $R^{5c}$, $R^{6c}$, and $R^{7c}$ satisfy the above-described conditions, the $\pi$-conjugated system of the component A3 is maintained, and the ultraviolet light in a wide wavelength range can be absorbed, so that the generation of the hydroxyl radical is suppressed and the effect of the present invention can be obtained. From the viewpoint of increasing absorbance of the ultraviolet light to suppress the generation of the hydroxyl radical, $R^{1c}$, $R^{2c}$, $R^{3c}$, $R^{4c}$, $R^{5c}$, $R^{6c}$, and $R^{7c}$ are preferably a hydrogen atom, an alkyl group having 1 to 5 carbon atoms, an alkenyl group having 2 to 5 carbon atoms, an aryl group having 6 to 12 carbon atoms, a hydroxy group, an alkoxy group having 1 to 6 carbon atoms, an amino group having 0 to 6 carbon atoms, or a monovalent group including a carbonyl group having 1 to 6 carbon atoms; more preferably a hydrogen atom, an alkyl group having 1 to 5 carbon atoms, a hydroxy group, an alkoxy group having 1 to 5 carbon atoms, a carboxy group, or an alkoxycarbonyl group having 2 to 6 carbon atoms; still more preferably a hydrogen atom, an alkyl group having 1 to 5 carbon atoms, or a hydroxy group; particularly preferably a hydrogen atom, a methyl group, an ethyl group, an n-propyl group, an isopropyl group, an n-butyl group, an s-butyl group, an i-butyl group, a t-butyl group, or a hydroxy group; especially preferably a hydrogen atom, a methyl group, an ethyl group, an n-propyl group, an isopropyl group, or a hydroxy group; and most preferably a hydrogen atom, a methyl group, or a hydroxy group.

The alkyl group is a chain (linear or branched) alkyl group or a cyclic alkyl group. An alkyl group having 1 to 20 carbon atoms is preferable, an alkyl group having 1 to 10 carbon atoms is more preferable, and an alkyl group having 1 to 5 carbon atoms is still more preferable. Examples of the linear alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, or an isopropyl group is preferable. In addition, examples of the cyclic alkyl group include a cyclopentyl group, a cyclohexyl group, and a cyclooctyl group.

The alkenyl group is a chain (linear or branched) alkenyl group or a cyclic alkenyl group. An alkenyl group having 2 to 20 carbon atoms is preferable, an alkenyl group having 2 to 10 carbon atoms is more preferable, and an alkenyl group having 2 to 5 carbon atoms is still more preferable. Examples of the chain alkenyl group include a vinyl group, a 1-propenyl group, an isopropenyl group, a 2-butenyl group, a 1,3-butadienyl group, a 2-pentenyl group, and a 2-hexenyl group. In addition, examples of the cyclic alkenyl group include a cyclopentenyl group and a cyclohexenyl group.

The aryl group is preferably an aryl group having 1 to 20 carbon atoms, and more preferably an aryl group having 1 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The alkoxy group is preferably an alkoxy group having 1 to 20 carbon atoms, more preferably an alkoxy group having 1 to 10 carbon atoms, and still more preferably an alkoxy group having 1 to 6 carbon atoms. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group ($-NH_2$) having no substituent on a nitrogen atom (number of carbon atoms: 0) and an amino group in which a part or all of hydrogen atoms bonded to the nitrogen atom are substituted with

carbon atoms. The number of carbon atoms in the amino group in which a part or all of hydrogen atoms bonded to the nitrogen atom are substituted with carbon atoms is preferably 1 to 20, more preferably 1 to 10, and still more preferably 1 to 6. Examples of the amino group include an amino group having no substituent (-$NH_2$), a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group include a formyl group, an acyl group, a carboxy group, an amide group, an alkoxycarbonyl group, a thiocarboxy group, and a thioester group; and a monovalent group including a carbonyl group having 1 to 6 carbon atoms is preferable.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6. Examples of the acyl group include an acetyl group, a propionyl group, a butylcarbonyl group, a vinylcarbonyl group, and a benzoyl group.

The amide group includes an amide group (-$CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group. The total number of carbon atoms derived from the carbonyl group and carbon atoms derived from the alkoxy group is preferably 2 to 20, more preferably 2 to 10, and still more preferably 2 to 6. Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group. The total number of carbon atoms derived from the carbonyl group and carbon atoms derived from the alkylthio group or the arylthio group is preferably 2 to 20, more preferably 2 to 10, and still more preferably 2 to 6. Examples of the thioester group include a methylthiocarbonyl group, an ethylthiocarbonyl group, a butylthiocarbonyl group, and a phenylthiocarbonyl group.

**In** addition, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

The alkylthio group is preferably an alkylthio group having 1 to 20 carbon atoms, more preferably an alkylthio group having 1 to 10 carbon atoms, and still more preferably an alkylthio group having 1 to 5 carbon atoms. Examples of the alkylthio group include a methylthio group, an ethylthio group, a propylthio group, and an isopropylthio group.

The arylthio group is preferably an arylthio group having 1 to 20 carbon atoms, more preferably an arylthio group having 3 to 10 carbon atoms, and still more preferably an arylthio group having 6 to 10 carbon atoms. Examples of the arylthio group include a phenylthio group and a tolylthio group.

Among the compounds satisfying the above-described conditions, from the viewpoint of quality stability of the methacrylate-containing composition during storage, as the component A3, t-butylphenyl ether, isopropylphenyl ether, 1-isopropoxy-3-methylbenzene, 4-isopropoxyphenol, 1-t-butoxy-4-methylbenzene, 2-(2,4-dimethyl-6-t-butylphenoxy)-2-methylpropionic acid, 2-(2,6-di-t-butyl-4-methylphenoxy)-2-methylpropionic acid, 2-(4-methoxyphenoxy)-2-methylpropionic acid, 2-(4-hydroxyphenoxy)-2-methylpropionic acid, 2-(2,4-dimethyl-6-t-butylphenoxy)-2-methylpropionic acid methyl, 2-(2,6-di-t-butyl-4-methylphenoxy)-2-methylpropionic acid methyl, 2-(4-methoxyphenoxy)-2-methylpropionic acid methyl, or 2-(4-hydroxyphenoxy)-2-methylpropionic acid methyl is preferable; t-butylphenyl ether, isopropylphenyl ether, 1-isopropoxy-3-methylbenzene, 4-isopropoxyphenol, or 2-(4-methoxyphenoxy)-2-methylpropionic acid is more preferable; and t-butylphenyl ether or 2-(4-methoxyphenoxy)-2-methylpropionic acid is still more preferable.

The component A3 may be one kind or two or more kinds.

(Component B)

The methacrylate-containing composition according to the third aspect preferably comprises a polymerization inhibitor (component B). In the present specification, the polymerization inhibitor means a compound having a function of suppressing the polymerization reaction of the methacrylic acid. Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress a polymerization reaction of the methacrylic acid by a radical polymerization mechanism during the storage of the

methacrylic acid. In addition, the component B can trap the above-described hydroxyl radical generated during the storage of the methacrylic acid. That is, when the methacrylate-containing composition comprises the component B in addition to the component A3, the amount of the hydroxyl radical can be reduced by two different mechanisms of suppressing the generation of the hydroxyl radical by the component A3 and removing the generated hydroxyl radical by the component B. Therefore, it is considered that the reduction of the component B and the generation of the decomposition product can be efficiently suppressed.

Examples of the polymerization inhibitor as the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, tris(nonylphenyl)phosphite, 4,4'-isopropylidenediphenol alkylphosphite, tris(2,4-di-tert-butylphenyl)phosphite, tris(biphenyl) phosphite, distearyl pentaerythritol diphosphite, di(2,4-di-tert-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among the above, from the viewpoint of quality stability of the methyl methacrylate-containing composition during storage, as the component B, at least one polymerization inhibitor selected from the group consisting of a phenol-based compound, an N-oxyl-based compound, an amine-based compound, a phosphorus-containing compound, and a sulfur-containing compound is preferable, and for example, at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is preferable. As the component B, a phenol-based compound is more preferable, and for example, at least one polymerization inhibitor selected from the group consisting of hydroquinone, 4-methoxyphenol, 2,4-dimethyl-6-t-butylphenol, and 2,6-di-t-butyl-4-methylphenol is more preferable. As the component B, for example, at least one polymerization inhibitor selected from the group consisting of hydroquinone and 4-methoxyphenol is particularly preferable.

The component B may be one kind or two or more kinds.

When the methacrylate-containing composition comprises a compound corresponding to both the component A3 and the component B, the compound is regarded as the component A3. That is, when the methacrylate-containing composition comprises the component A3 and the component B, it means that the methacrylate-containing composition comprises another component B different from the compound. When the methacrylate-containing composition comprises two or more kinds of compounds corresponding to both the component A3 and the component B, a compound having the highest molar concentration in the methacrylate-containing composition is regarded as the component A3, and the other compounds are regarded as the component B.

(Concentrations of component A3 and component B)

When the concentration of the component A3 is indicated by $M_{A3}$ (μmol/L) and the concentration of the component B is indicated by $M_B$ (μmol/L), from the viewpoint of efficiency of suppressing generation of a methacrylic acid dimer and pyruvic acid, $M_B/M_{A3}$ is preferably 0.005 to 100 and more preferably 0.05 to 10.

$M_{A3}$ is preferably 1 to 50,000 μmol/L. When $M_{A3}$ is 1 μmol/L or more, the effect of suppressing the decomposition of the polymerization inhibitor can be sufficiently obtained. In addition, when the $M_{A3}$ is 50,000 μmol/L or less, the amount of impurities when a methacrylic acid polymer is produced by polymerization of the methacrylate-containing composition according to the third aspect can be reduced, and thus it is possible to prevent physical properties of the polymer from being adversely affected. The lower limit of $M_{A3}$ is more preferably 10 μmol/L or more, still more preferably 30 μmol/L or more, still more preferably 50 μmol/L or more, even more preferably 55 μmol/L or more, and particularly preferably 60 μmol/L or more. The upper limit of $M_{A3}$ is more preferably 25,000 μmol/L or less, still more preferably 10,000 μmol/L or less, and particularly preferably 7,500 μmol/L or less.

$M_B$ is preferably 1 to 50,000 μmol/L. When $M_B$ is 1 μmol/L or more, the effect of suppressing generation of a methacrylic acid dimer and pyruvic acid can be sufficiently obtained. In addition, when the $M_B$ is 50,000 μmol/L or less, the amount of impurities when a methacrylic acid polymer is produced by polymerization of the methacrylate-containing composition according to the third aspect can be reduced, and thus it is possible to prevent physical properties of the polymer from being adversely affected. The lower limit of $M_B$ is more preferably 10 μmol/L or more, still more preferably 100 μmol/L or more, especially preferably 1,500 μmol/L or more, and most preferably 2,000 μmol/L or more. The upper limit of $M_B$ is more preferably 45,000 μmol/L or less, still more preferably 40,000 μmol/L or less, particularly preferably 35,000

μmol/L or less, especially preferably 30,000 μmol/L or less, and most preferably 25,000 μmol/L or less.

(Concentration of methacrylic acid)

The concentration of the methacrylic acid in the methacrylate-containing composition according to the third aspect is 98.00% to 99.99% by mass. When the concentration of the methacrylic acid is 98.00% by mass or more, the amount of impurities when a methacrylic acid polymer is produced by polymerization of the methacrylate-containing composition according to the third aspect can be reduced, and thus it is possible to prevent physical properties of the polymer from being adversely affected. In addition, when the concentration of the methacrylic acid is 99.99% by mass or less, a purification cost can be reduced. The lower limit of the concentration of the methacrylic acid is more preferably 98.50% by mass or more, still more preferably 99.00% by mass or more, and particularly preferably 99.50% by mass or more.

(Component C)

The methacrylate-containing composition according to the third aspect may further comprise other compounds as the component C as long as the concentration of the methacrylic acid satisfies 98.00% to 99.99% by mass. Examples of the component C include impurities generated in the process of producing the methacrylic acid. For example, the methacrylic acid may contain diacetyl as an impurity, but from the viewpoint of reducing coloration of the methacrylate-containing composition, the concentration of the diacetyl is preferably 55 μmol/L or less, more preferably 20 μmol/L or less, still more preferably 10 μmol/L or less, and particularly preferably 1 μmol/L or less.

(Analysis of methacrylate-containing composition)

The fact that the methacrylate-containing composition comprises the component A3, the component B, the component C, and the water can be confirmed by, for example, GC-MS measurement. In a GC-MS chart of the methacrylate-containing composition, when a peak is present at the same retention time as a sample of the component A3 and an m/z value detected in a mass spectrum of the peak matches exact mass of the component A3, it can be determined that the methacrylate-containing composition comprises the component A3. When the sample of the component A3 cannot be obtained, when a pattern of the mass spectrum of the peak appearing in the GC-MS chart of the methacrylate-containing composition and a pattern of a mass spectrum of the component A3 in mass spectrum database and match each other, it can be determined that the peak is the peak of the component A3. That is, it can be determined that the methacrylate-containing composition comprises the component A3. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

In addition, the concentration of the methacrylic acid can be calculated, for example, by performing GC-FID measurement of the methacrylate-containing composition, quantifying by an area percentage method, and correcting the quantified moisture concentration using a Karl Fischer moisture meter. The concentration of the component A3 can be quantified, for example, by performing GC measurement of the methacrylate-containing composition and using an internal standard method. When a sample of the component A3 cannot be obtained and the component A3 cannot be quantified by the internal standard method, the concentration of the component A3 can be calculated using the following expression by performing GC-FID measurement on any organic compound having a known concentration under the same conditions as those of the methacrylate-containing composition.

$$\text{Concentration of component A3 } (\mu\text{mol/L}) = \frac{N}{N_{A3}} \times \frac{S_{A3}}{S} \times M$$

Here, N is the number of carbon atoms in one molecule of the organic compound having a known concentration, $N_{A3}$ is the number of carbon atoms in one molecule of the component A3, $S_{A3}$ is a peak area of the component A3, S is a peak area of the organic compound having a known concentration, and M is the concentration (μmol/L) of the organic compound having a known concentration.

When the volatility is low and the quantification cannot be performed by the GC measurement, the quantification can be performed using a chromatography method such as LC.

Concentrations of the component B and the component C can also be calculated by the same method as that for the component A3 described above.

In addition, the fact that the methacrylate-containing composition comprises water, and the concentration thereof

can be confirmed by Karl Fischer method.

[Method for producing methacrylate-containing composition]

Examples of a method for producing the methacrylate-containing composition according to the third aspect include a method of adding the component A3 to methacrylic acid, and preferred examples thereof include a method of adding the component A3 and the component B to methacrylic acid. As the methacrylic acid, a commercially available product may be used, or methacrylic acid produced by a known method such as an acetone cyanohydrin (ACH) method and a C4 direct oxidation method may be used. As the component A3 and the component B, a commercially available product may be used, or a component synthesized by a known method may be used. When methacrylic acid produced by a known method such as an acetone cyanohydrin (ACH) method and a C4 direct oxidation method is used, the methacrylate-containing composition may be produced by adding the component A3 or the component B in the middle of the process of a raw material step or a production step. In addition, when the component A3 or the component B is generated as a by-product in the methacrylic acid production process, the methacrylate-containing composition may be produced by leaving a part of the generated component A3 or component B.

[Evaluation method for storage stability and thermal stability]

The methacrylate-containing composition according to the third aspect has high quality stability during storage. Examples of an evaluation method for the quality stability of the methacrylate-containing composition during storage include a method of actually storing the methacrylate-containing composition for a long period of time and confirming the amount of the polymerization inhibitor reduced. In addition, from the viewpoint of ease of work, a method of heating the methacrylate-containing composition for a short period of time and confirming the amount of the polymerization inhibitor reduced may be used. When heating for a short period of time, the heating temperature is preferably 50°C to 100°C and a heating time is preferably 1 to 24 hours. In the present invention, the quality stability of the methacrylate-containing composition during storage is evaluated by the amount of decrease in polymerization inhibitor when the methacrylate-containing composition is stored at 25°C for 21 days.

[Method for producing methacrylic ester]

A method for producing a methacrylic ester according to the third aspect includes a step of esterifying the methacrylate-containing composition according to the third aspect.
The alcohol to be reated with the methacrylate-containing composition is not particularly limited, and examples thereof include methanol, ethanol, n-propanol, isopropanol, n-butanol, and isobutanol. Examples of the methacrylic ester to be obtained include methyl methacrylate, ethyl methacrylate, propyl methacrylate, isopropyl methacrylate, butyl methacrylate, and isobutyl methacrylate. The esterification reaction can be carried out in the presence of an acidic catalyst such as a sulfonic acid-type cation exchange resin. The temperature during the esterification reaction is preferably 50°C to 200°C.
The pressure during the esterification reaction, the location of the catalyst in the reaction vessel, the proportion of the catalyst in the reaction vessel, and the like are not particularly limited, and a commonly used form can be adopted.

[Method for producing methacrylic acid polymer according to third aspect]

A method for producing a methacrylic acid polymer according to the third aspect includes a step of polymerizing a polymerizable composition comprising the methacrylate-containing composition according to the third aspect.
The polymerizable composition may further comprise a monomer copolymerizable with the methacrylic acid and other additive substances, as necessary.
Examples of the monomer copolymerizable with the methacrylic acid include the following:

methacrylic esters such as methyl methacrylate, ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;
acrylic esters such as methyl acrylate, ethyl acrylate, n-butyl acrylate, iso-butyl acrylate, tert-butyl acrylate, and 2-ethylhexyl acrylate;
unsaturated carboxylic acids such as acrylic acid, maleic acid, and itaconic acid;
unsaturated carboxylic anhydrides such as maleic anhydride and itaconic anhydride;
maleimides such as N-phenylmaleimide and N-cyclohexylmaleimide;
hydroxy group-containing vinyl monomers such as 2-hydroxyethyl acrylate, 2-hydroxyethyl methacrylate, and 2-hydroxypropyl methacrylate;
vinyl esters such as vinyl acetate and vinyl benzoate;
vinyl chloride, vinylidene chloride, and derivatives thereof;
nitrogen-containing vinyl monomers such as methacrylamide and acrylonitrile;
epoxy group-containing monomers such as glycidyl acrylate and glycidyl methacrylate;
aromatic vinyl monomers such as styrene and $\alpha$-methylstyrene;
alkanediol di(meth)acrylates such as ethylene glycol di(meth)acrylate, 1,2-propylene glycol di(meth)acrylate, 1,3-butylene glycol di(meth)acrylate, and 1,6-hexanediol di(meth)acrylate;
polyoxyalkylene glycol di(meth)acrylates such as diethylene glycol di(meth)acrylate, dipropylene glycol di(meth) acrylate, triethylene glycol (meth)acrylate, tetraethylene glycol di(meth)acrylate, polyethylene glycol di(meth)acrylate, and neopentyl glycol di(meth)acrylate;
vinyl monomers having two or more ethylenically unsaturated bonds in a molecule, such as divinylbenzene;
unsaturated polyester prepolymer obtained from at least one polyvalent carboxylic acid including an ethylenically unsaturated polycarboxylic acid and at least one diol; and
vinyl ester prepolymer obtained by acrylic-modifying a terminal of epoxy group.

Among the above, the monomer copolymerizable with the methacrylic acid is preferably at least one selected from the group consisting of methacrylic ester and acrylic ester. The monomer copolymerizable with the methacrylic acid is more preferably methacrylic ester and particularly preferably methyl methacrylate.
The monomer copolymerizable with the methacrylic acid may be one kind or two or more kinds. In addition, when the component A3 is a monomer copolymerizable with the methacrylic acid, the component A3 may be used as the monomer copolymerizable with the methacrylic acid, or another monomer copolymerizable with the methacrylic acid may be used separately from the component A3.
In the polymerizable composition, a contained amount of the monomer copolymerizable with the methacrylic acid is preferably 50.00 to 99.99 parts by mass with respect to 100 parts by mass of the polymerizable composition. The lower limit of the contained amount of the monomer copolymerizable with the methacrylic acid is more preferably 60 parts by mass or more, still more preferably 70 parts by mass or more, particularly preferably 80 parts by mass or more, and most preferably 90 parts by mass or more with respect to 100 parts by mass of the polymerizable composition. The upper limit of the contained amount of the monomer copolymerizable with the methacrylic acid is more preferably 99.9 parts by mass or less and still more preferably 99 parts by mass or less with respect to 100 parts by mass of the polymerizable composition.
As the other additives, a polymerization initiator is preferable. In addition, the polymerizable composition may further comprise, as necessary, a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, a fluorescent agent, or the like. The other additives may be one kind or two or more kinds.
Examples of the polymerization initiator include the following:

azo compounds such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate;
organic peroxides such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxyisononanoate, di-t-butyl peroxide, di-t-hexyl peroxide, lauroyl peroxide, and dilauroyl peroxide;
persulfate compounds such as potassium persulfate; and
a redox polymerization initiator.

Among the above, from the viewpoint of storage stability and reactivity with the monomer copolymerizable with the methacrylic acid, the polymerization initiator is preferably at least one selected from the group consisting of an azo compound and an organic peroxide.
The amount of the polymerization initiator used is preferably 0.0001 to 1 part by mass with respect to 100 parts by mass of the total amount of the methacrylic acid and the monomer copolymerizable with the methacrylic acid.
Examples of a polymerization method for the polymerizable composition include a bulk polymerization method, a solution polymerization method, an emulsion polymerization method, and a suspension polymerization method.

The polymerization temperature is preferably 125°C to 210°C. In this manner, an appropriate polymerization rate can be obtained. The lower limit of the polymerization temperature is more preferably 130°C or higher, and the upper limit thereof is more preferably 180°C or lower. The polymerization time is not particularly limited, and can be, for example, 0.5 to 24 hours.

[Methacrylate-containing composition according to fourth aspect]

A methacrylate-containing composition according to a fourth aspect comprises methacrylic acid, a compound (component A4) represented by Formula (41), and a polymerization inhibitor (component B). The concentration of the methacrylic acid is 98.00% to 99.99% by mass.

$$R^d\text{-}C{\equiv}N \cdots \quad (41)$$

In Formula (41), $R^d$ represents an alkyl group having 1 to 6 carbon atoms, an alkenyl group having 2 to 6 carbon atoms, or an aryl group having 6 to 12 carbon atoms, where these groups may further have a substituent.

In addition, the methacrylate-containing composition may further comprise other compounds (component C) or water as long as the concentration of the methacrylic acid satisfies 98.00% to 99.99% by mass. Hereinafter, each item will be described in detail.

(Methacrylic acid)

The methacrylate-containing composition according to the fourth aspect comprises methacrylic acid. The methacrylic acid can be produced, for example, as a by-product of an acetone cyanohydrin (ACH) method or an intermediate of a C4 direct oxidation method. The methacrylic acid contained in the methacrylate-containing composition is preferably produced by a C4 direct oxidation method, and more preferably produced by a C4 direct oxidation method using isobutanol derived from a biomass as a starting raw material.

(Component A4)

The methacrylate-containing composition according to the fourth aspect comprises the compound (component A4) represented by Formula (41). Since the methacrylate-containing composition comprises the component A4, it is possible to suppress decomposition of the polymerization inhibitor. The reason for this is presumed to be as follows.

It is known that the methacrylic acid is polymerized by radicals generated during storage, and the polymerization inhibitor (component B) is added to prevent the polymerization. The component B has a function of preventing polymerization by trapping radicals generated during the storage of methacrylic acid, but the polymerization inhibitor is decomposed into another compound when trapping the radicals. Therefore, the concentration of the component B in the methacrylate-containing composition gradually decreases during the storage, and the function of preventing polymerization gradually decreases. Furthermore, unnecessary decomposition products generated by trapping the radicals by the component B gradually increase. Examples of the radicals generated during the storage of methacrylic acid include a hydroxyl radical generated by an oxygen molecule absorbing ultraviolet light derived from sunlight. Since the component A4 has weak basicity, generation of a hydroxyion ($OH^-$) is prioritized over the generation of the hydroxyl radical, and the generation of the hydroxyl radical is suppressed. Accordingly, since the number of radicals which need to be trapped by the component B is reduced, the decrease in concentration of the component B can be suppressed.

The molecular weight of the component A4 is preferably 1,000 or less. When the molecular weight thereof is 1,000 or less, the number of cyano groups per unit mass in the component A4 can be increased, and thus the effect of the present invention can be obtained with a small mass. The molecular weight of the component A4 is more preferably 800 or less, still more preferably 600 or less, and particularly preferably 400 or less.

$R^d$ in Formula (41) represents an alkyl group having 1 to 6 carbon atoms, an alkenyl group having 2 to 6 carbon atoms, or an aryl group having 6 to 12 carbon atoms, where these groups may further have a substituent.

When $R^d$ satisfies the above-described condition, the weak basicity of the component A4 and the reactivity with the acidic substance or the radical are maintained, and thus the effect of the present invention can be obtained. In addition, since it is a group having high stability, the component A4 can be prevented from being changed into other compounds during storage. $R^d$ is preferably an alkyl group having 1 to 3 carbon atoms, an alkenyl group having 2 or 3 carbon atoms, or an aryl group having 6 to 8 carbon atoms. $R^d$ is more preferably a methyl group, an ethyl group, a vinyl group, an isopropenyl group, a phenyl group, a methylthiophenyl group, an acetoxyphenyl group, or a cyanophenyl group; still more preferably a methyl group, an ethyl group, a vinyl group, an isopropenyl group, a methylthiophenyl group, an acetoxyphenyl group, or a cyanophenyl group; and particularly preferably a methyl group, an ethyl group, a vinyl group, an isopropenyl group, an acetoxyphenyl group, or a cyanophenyl group.

The alkyl group is a chain (linear or branched) alkyl group or a cyclic alkyl group. Examples of the chain alkyl group include a methyl group, an ethyl group, an n-propyl group, an isopropyl group, an n-butyl group, an isobutyl group, an s-butyl group, a t-butyl group, an n-pentyl group, and an isopentyl group. Among the above, a methyl group, an ethyl group, an n-propyl group, or an isopropyl group is preferable, and a methyl group or an ethyl group is more preferable. In addition, examples of the cyclic alkyl group include a cyclopropyl group, a cyclobutyl group, and a cyclopentyl group.

The alkenyl group is a chain (linear or branched) alkenyl group or a cyclic alkenyl group. Examples of the chain alkenyl group include a vinyl group, a 1-propenyl group, an isopropenyl group, a 2-butenyl group, a 1,3-butadienyl group, and a 2-pentenyl group. Among the above, a vinyl group, a 1-propenyl group, or an isopropenyl group is preferable, and a vinyl group or an isopropenyl group is more preferable. In addition, examples of the cyclic alkenyl group include a cyclopropenyl group, a cyclobutenyl group, and a cyclopentenyl group.

The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

When $R^d$ is an alkyl group, an alkenyl group, or an aryl group, each of which has a substituent, examples of the substituent include an alkyl group, an alkenyl group, an aryl group, a hydroxy group, an alkoxy group, an amino group, a monovalent group including a carbonyl group, an alkylthio group, and an arylthio group. Among the above, a hydroxy group, an alkoxy group, an amino group, a monovalent group including a carbonyl group, or an alkylthio group is preferable, and a hydroxy group, a methoxy group, an amino group, an acetyl group, or a methylthio group is more preferable. The molecular weight of the substituent is preferably 200 or less, more preferably 100 or less, and still more preferably 50 or less. The number of carbon atoms in $R^d$ is the number of carbon atoms in the alkyl group, the alkenyl group, or the aryl group, which includes carbon atoms of substituents of these groups. For example, in 4-(methylthio)benzonitrile, $R^d$ is a 4-(methylthio)phenyl group, and it is considered to have an aryl group having 7 carbon atoms.

The alkyl group as the substituent is the same as the above-described alkyl group as long as $R^d$ is an alkyl group having 1 to 5 carbon atoms, an alkenyl group having 2 to 5 carbon atoms, or an aryl group having 1 to 12 carbon atoms. The number of carbon atoms in the alkyl group as the substituent is 1 to 11, preferably 1 to 6 and more preferably 1 to 3.

The alkenyl group as the substituent is the same as the above-described alkenyl group as long as $R^d$ is an alkyl group having 1 to 5 carbon atoms, an alkenyl group having 2 to 5 carbon atoms, or an aryl group having 1 to 12 carbon atoms. The number of carbon atoms in the alkenyl group as the substituent is 2 to 11, preferably 2 to 6 and more preferably 2 or 3.

The aryl group as the substituent is the same as the above-described aryl group as long as $R^d$ is an alkyl group having 1 to 5 carbon atoms, an alkenyl group having 2 to 5 carbon atoms, or an aryl group having 1 to 12 carbon atoms. The number of carbon atoms in the aryl group as the substituent is 1 to 11, preferably 3 to 9 and more preferably 5 to 7.

The alkoxy group as the substituent is an alkoxy group having 1 to 11 carbon atoms, and is preferably an alkoxy group having 1 to 6 carbon atoms and more preferably an alkoxy group having 1 to 3 carbon atoms. However, $R^d$ is an alkyl group having 1 to 5 carbon atoms, an alkenyl group having 2 to 5 carbon atoms, or an aryl group having 1 to 12 carbon atoms. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group as the substituent includes an amino group ($-NH_2$) having no substituent on a nitrogen atom (number of carbon atoms: 0) and an amino group in which a part or all of hydrogen atoms bonded to the nitrogen atom are substituted with carbon atoms. The number of carbon atoms in the amino group in which a part or all of hydrogen atoms bonded to the nitrogen atom are substituted with carbon atoms is 1 to 11, preferably 1 to 6 and more preferably 1 to 3. However, $R^d$ is an alkyl group having 1 to 5 carbon atoms, an alkenyl group having 2 to 5 carbon atoms, or an aryl group having 1 to 12 carbon atoms. Examples of the amino group include an amino group having no substituent ($-NH_2$), a methylamino group, an ethylamino group, a propylamino group, a butylamino group, a dimethylamino group, a diethylamino group, an anilino group, a toluidino group, an anisidino group, and an N-methyl-N-phenylamino group.

Examples of the monovalent group including a carbonyl group as the substituent include a formyl group, an acyl group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is 2 to 11, preferably 2 to 7 and more preferably 2 to 4. However, $R^d$ is an alkyl group having 1 to 5 carbon atoms, an alkenyl group having 2 to 5 carbon atoms, or an aryl group having 1 to 12 carbon

atoms. Examples of the acyl group include an acetyl group, a propionyl group, a butylcarbonyl group, a vinylcarbonyl group, and a benzoyl group.

The amide group includes an amide group (-$CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and carbon atoms substituted on the nitrogen atom is 1 to 11, preferably 1 to 7 and more preferably 1 to 4. However, $R^d$ is an alkyl group having 1 to 5 carbon atoms, an alkenyl group having 2 to 5 carbon atoms, or an aryl group having 1 to 12 carbon atoms. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group. The total number of carbon atoms derived from the carbonyl group and carbon atoms derived from the alkoxy group is 2 to 11, preferably 2 to 7 and more preferably 2 to 4. However, $R^d$ is an alkyl group having 1 to 5 carbon atoms, an alkenyl group having 2 to 5 carbon atoms, or an aryl group having 1 to 12 carbon atoms. Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group. The total number of carbon atoms derived from the carbonyl group and carbon atoms derived from the alkylthio group or the arylthio group is 2 to 11, preferably 2 to 7 and more preferably 2 to 4. However, $R^d$ is an alkyl group having 1 to 5 carbon atoms, an alkenyl group having 2 to 5 carbon atoms, or an aryl group having 1 to 12 carbon atoms. Examples of the thioester group include a methylthiocarbonyl group, an ethylthiocarbonyl group, a butylthiocarbonyl group, and a phenylthiocarbonyl group.

In addition, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

The alkylthio group as the substituent is an alkylthio group having 1 to 11 carbon atoms, and is preferably an alkylthio group having 1 to 6 carbon atoms and more preferably an alkylthio group having 1 to 3 carbon atoms. However, $R^d$ is an alkyl group having 1 to 5 carbon atoms, an alkenyl group having 2 to 5 carbon atoms, or an aryl group having 1 to 12 carbon atoms. Examples of the alkylthio group include a methylthio group, an ethylthio group, a propylthio group, and an isopropylthio group.

The arylthio group as the substituent is an arylthio group having 1 to 11 carbon atoms, and is preferably an arylthio group having 3 to 10 carbon atoms and more preferably an arylthio group having 6 to 10 carbon atoms. However, $R^d$ is an alkyl group having 1 to 5 carbon atoms, an alkenyl group having 2 to 5 carbon atoms, or an aryl group having 1 to 12 carbon atoms. Examples of the arylthio group include a phenylthio group and a tolylthio group.

Among the compounds satisfying the above-described conditions, from the viewpoint of quality stability of the methacrylate-containing composition during storage, as the component A4, methacrylonitrile, acetonitrile, propionitrile, acrylonitrile, benzonitrile, 3-hydroxypropionitrile, 3-methoxypropionitrile, 4-aminobenzonitrile, 4'-cyanoacetophenone, 4-(methylthio)benzonitrile, 2-hydroxypropionitrile, 2-aminopropionitrile, or 4-cyanophenol is preferable; methacrylonitrile, acetonitrile, propionitrile, acrylonitrile, benzonitrile, 3-hydroxypropionitrile, 3-methoxypropionitrile, 4-aminobenzonitrile, 4'-cyanoacetophenone, or 4-(methylthio)benzonitrile is more preferable; methacrylonitrile, acetonitrile, propionitrile, acrylonitrile, 4-cyanoacetophenone, or 4-(methylthio)benzonitrile is still more preferable; and methacrylonitrile, acetonitrile, propionitrile, acrylonitrile, or 4-cyanoacetophenone is particularly preferable.

The component A4 may be one kind or two or more kinds.

(Component B)

The methacrylate-containing composition according to the fourth aspect comprises a polymerization inhibitor (component B). In the present specification, the polymerization inhibitor means a compound having a function of suppressing the polymerization reaction of the methacrylic acid. Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress a polymerization reaction of the methacrylic acid by a radical polymerization mechanism during the storage of the methacrylic acid. In addition, the component B can trap the above-described hydroxyl radical generated during the storage of the methacrylic acid. That is, when the methacrylate-containing composition comprises the component B in addition to the component A4, the amount of the hydroxyl radical can be reduced by two different mechanisms of suppressing the generation of the hydroxyl radical by the component A4 and removing the generated hydroxyl radical by the component B. Therefore, it is considered that the reduction of the component B can be efficiently suppressed.

Examples of the polymerization inhibitor as the phenol-based compound include alkylphenol, hydroxyphenol,

aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, tris(nonylphenyl)phosphite, 4,4'-isopropylidenediphenol alkylphosphite, tris(2,4-di-tert-butylphenyl)phosphite, tris(biphenyl) phosphite, distearyl pentaerythritol diphosphite, di(2,4-di-tert-butylphenyl)pentaerythritol diphosphite, di(nonylphenyl) pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride,

and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among the above, from the viewpoint of quality stability of the methacrylate-containing composition during storage, as the component B, at least one polymerization inhibitor selected from the group consisting of a phenol-based compound, an N-oxyl-based compound, an amine-based compound, a phosphorus-containing compound, and a sulfur-containing compound is preferable, and for example, at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is preferable. As the component B, a phenol-based compound is more preferable, and for example, at least one polymerization inhibitor selected from the group consisting of hydroquinone, 4-methoxyphenol, 2,4-dimethyl-6-t-butylphenol, and 2,6-di-t-butyl-4-methylphenol is more preferable. As the component B, for example, at least one polymerization inhibitor selected from the group consisting of hydroquinone and 4-methoxyphenol is particularly preferable.

The component B may be one kind or two or more kinds.

When the methacrylate-containing composition comprises a compound corresponding to both the component A4 and the component B, the compound is regarded as the component B. That is, the methacrylate-containing composition needs to contain another component A4 different from the compound. When the methacrylate-containing composition comprises two or more kinds of compounds corresponding to both the component A4 and the component B, a compound having the highest molar concentration in the methacrylate-containing composition is regarded as the component B, and the other compounds are regarded as the component A4.

(Concentrations of component A4 and component B)

When the concentration of the component A4 is indicated by $M_{A4}$ (μmol/L) and the concentration of the component B is indicated by $M_B$ (μmol/L), from the viewpoint of efficiency of suppressing generation of a methacrylic acid dimer and pyruvic acid, $M_B/M_{A4}$ is preferably 0.005 to 100 and more preferably 0.05 to 10.

$M_{A4}$ is preferably 1 to 40,000 μmol/L. When $M_{A4}$ is 1 μmol/L or more, the effect of suppressing the decomposition of the polymerization inhibitor can be sufficiently obtained. In addition, when the $M_{A4}$ is 40,000 μmol/L or less, the amount of impurities when a methacrylic acid polymer is produced by polymerization of the methacrylate-containing composition according to the fourth aspect can be reduced, and thus it is possible to prevent physical properties of the polymer from being adversely affected. The lower limit of $M_{A4}$ is more preferably 10 μmol/L or more, still more preferably 30 μmol/L or more, still more preferably 50 μmol/L or more, even more preferably 60 μmol/L or more, particularly preferably 70 μmol/L or more, and especially preferably 80 μmol/L or more. The upper limit of $M_{A4}$ is more preferably 30,000 μmol/L or less, still more preferably 25,000 μmol/L or less, and particularly preferably 20,000 μmol/L or less.

$M_B$ is preferably 1 to 50,000 μmol/L. When $M_B$ is 1 μmol/L or more, the effect of suppressing generation of a methacrylic acid dimer and pyruvic acid can be sufficiently obtained. In addition, when the $M_B$ is 50,000 μmol/L or less, the amount of impurities when a methacrylic acid polymer is produced by polymerization of the methacrylate-containing composition according to the fourth aspect can be reduced, and thus it is possible to prevent physical properties of the polymer from being adversely affected. The lower limit of $M_B$ is more preferably 10 μmol/L or more, still more preferably 100 μmol/L or more, particularly preferably 1,500 μmol/L or more, and especially preferably 2,000 μmol/L or more. The upper limit of $M_B$ is more preferably 45,000 μmol/L or less, still more preferably 40,000 μmol/L or less, particularly preferably 35,000 μmol/L or less, especially preferably 30,000 μmol/L or less, and most preferably 25,000 μmol/L or less.

(Concentration of methacrylic acid)

The concentration of the methacrylic acid in the methacrylate-containing composition according to the fourth aspect is 98.00% to 99.99% by mass. When the concentration of the methacrylic acid is 98.00% by mass or more, the amount of impurities when a methacrylic acid polymer is produced by polymerization of the methacrylate-containing composition according to the fourth aspect can be reduced, and thus it is possible to prevent physical properties of the

polymer from being adversely affected. In addition, when the concentration of the methacrylic acid is 99.99% by mass or less, a purification cost can be reduced. The lower limit of the concentration of the methacrylic acid is more preferably 98.50% by mass or more, still more preferably 99.00% by mass or more, particularly preferably 99.50% by mass or more, and most preferably 99.80% by mass or more.

(Component C)

The methacrylate-containing composition according to the fourth aspect may further comprise other compounds as the component C as long as the concentration of the methacrylic acid satisfies 98.00% to 99.99% by mass. Examples of the component C include impurities generated in the process of producing the methacrylic acid. For example, the methacrylic acid may contain diacetyl as an impurity, but from the viewpoint of reducing coloration of the methacrylate-containing composition, the concentration of the diacetyl is preferably 55 μmol/L or less, more preferably 20 μmol/L or less, still more preferably 10 μmol/L or less, and particularly preferably 1 μmol/L or less.

(Analysis of methacrylate-containing composition)

The fact that the methacrylate-containing composition comprises the component A4, the component B, the component C, and the water can be confirmed by, for example, GC-MS measurement. In a GC-MS chart of the methacrylate-containing composition, when a peak is present at the same retention time as a sample of the component A4 and an m/z value detected in a mass spectrum of the peak matches exact mass of the component A4, it can be determined that the methacrylate-containing composition comprises the component A4. When the sample of the component A4 cannot be obtained, when a pattern of the mass spectrum of the peak appearing in the GC-MS chart of the methacrylate-containing composition and a pattern of a mass spectrum of the component A4 in mass spectrum database and match each other, it can be determined that the peak is the peak of the component A4. That is, it can be determined that the methacrylate-containing composition comprises the component A4. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

In addition, the concentration of the methacrylic acid can be calculated, for example, by performing GC-FID measurement of the methacrylate-containing composition, quantifying by an area percentage method, and correcting the quantified moisture concentration using a Karl Fischer moisture meter. The concentration of the component A4 can be quantified, for example, by performing GC measurement of the methacrylate-containing composition and using an internal standard method. When a sample of the component A4 cannot be obtained and the component A4 cannot be quantified by the internal standard method, the concentration of the component A4 can be calculated using the following expression by performing GC-FID measurement on any organic compound having a known concentration under the same conditions as those of the methacrylate-containing composition.

$$\text{Concentration of component A4 } (\mu\text{mol/L}) = \frac{N}{N_{A4}} \times \frac{S_{A4}}{S} \times M$$

Here, N is the number of carbon atoms in one molecule of the organic compound having a known concentration, $N_{A4}$ is the number of carbon atoms in one molecule of the component A4, $S_{A4}$ is a peak area of the component A4, S is a peak area of the organic compound having a known concentration, and M is the concentration (μmol/L) of the organic compound having a known concentration.

When the volatility is low and the quantification cannot be performed by the GC measurement, the quantification can be performed using a chromatography method such as LC.

Concentrations of the component B and the component C can also be calculated by the same method as that for the component A4 described above.

In addition, the fact that the methacrylate-containing composition comprises water, and the concentration thereof can be confirmed by Karl Fischer method.

[Method for producing methacrylate-containing composition]

Examples of a method for producing the methacrylate-containing composition according to the fourth aspect include a method of adding the component A4 and the component B to methacrylic acid. As the methacrylic acid, a commercially available product may be used, or methacrylic acid produced by a known method such as an acetone cyanohydrin (ACH) method and a C4 direct oxidation method may be used. As the component A4 and the component B, a

commercially available product may be used, or a component synthesized by a known method may be used. When methacrylic acid produced by a known method such as an acetone cyanohydrin (ACH) method and a C4 direct oxidation method is used, the methacrylate-containing composition may be produced by adding the component A4 or the component B in the middle of the process of a raw material step or a production step. In addition, when the component A4 or the component B is generated as a by-product in the methacrylic acid production process, the methacrylate-containing composition may be produced by leaving a part of the generated component A4 or component B.

[Evaluation method for storage stability and thermal stability]

The methacrylate-containing composition according to the fourth aspect has high quality stability during storage. Examples of an evaluation method for the quality stability of the methacrylate-containing composition during storage include a method of actually storing the methacrylate-containing composition for a long period of time and confirming the amount of the polymerization inhibitor reduced. In addition, from the viewpoint of ease of work, a method of heating the methacrylate-containing composition for a short period of time and confirming the amount of the polymerization inhibitor reduced may be used. When heating for a short period of time, the heating temperature is preferably 50°C to 100°C and a heating time is preferably 1 to 24 hours. In the present invention, the quality stability of the methacrylate-containing composition during storage is evaluated by the amount of decrease in polymerization inhibitor when the methacrylate-containing composition is stored at 25°C for 21 days.

[Method for producing methacrylic ester]

A method for producing a methacrylic ester according to the fourth aspect includes a step of esterifying the methacrylate-containing composition according to the fourth aspect.

The alcohol to be reated with the methacrylate-containing composition is not particularly limited, and examples thereof include methanol, ethanol, n-propanol, isopropanol, n-butanol, and isobutanol. Examples of the methacrylic ester to be obtained include methyl methacrylate, ethyl methacrylate, propyl methacrylate, isopropyl methacrylate, butyl methacrylate, and isobutyl methacrylate. The esterification reaction can be carried out in the presence of an acidic catalyst such as a sulfonic acid-type cation exchange resin. The temperature during the esterification reaction is preferably 50°C to 200°C.

The pressure during the esterification reaction, the location of the catalyst in the reaction vessel, the proportion of the catalyst in the reaction vessel, and the like are not particularly limited, and a commonly used form can be adopted.

[Method for producing methacrylic acid polymer according to fourth aspect]

A method for producing a methacrylic acid polymer according to the fourth aspect includes a step of polymerizing a polymerizable composition comprising the methacrylate-containing composition according to the fourth aspect.

The polymerizable composition may further comprise a monomer copolymerizable with the methacrylic acid and other additive substances, as necessary.

Examples of the monomer copolymerizable with the methacrylic acid include the following:

methacrylic esters such as methyl methacrylate, ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;
acrylic esters such as methyl acrylate, ethyl acrylate, n-butyl acrylate, iso-butyl acrylate, tert-butyl acrylate, and 2-ethylhexyl acrylate;
unsaturated carboxylic acids such as acrylic acid, maleic acid, and itaconic acid;
unsaturated carboxylic anhydrides such as maleic anhydride and itaconic anhydride;
maleimides such as N-phenylmaleimide and N-cyclohexylmaleimide;
hydroxy group-containing vinyl monomers such as 2-hydroxyethyl acrylate, 2-hydroxyethyl methacrylate, and 2-hydroxypropyl methacrylate;
vinyl esters such as vinyl acetate and vinyl benzoate;
vinyl chloride, vinylidene chloride, and derivatives thereof;
nitrogen-containing vinyl monomers such as methacrylamide and acrylonitrile;
epoxy group-containing monomers such as glycidyl acrylate and glycidyl methacrylate;
aromatic vinyl monomers such as styrene and $\alpha$-methylstyrene;
alkanediol di(meth)acrylates such as ethylene glycol di(meth)acrylate, 1,2-propylene glycol di(meth)acrylate, 1,3-butylene glycol di(meth)acrylate, and 1,6-hexanediol di(meth)acrylate;
polyoxyalkylene glycol di(meth)acrylates such as diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate;
vinyl monomers having two or more ethylenically unsaturated bonds in a molecule, such as divinylbenzene;
unsaturated polyester prepolymer obtained from at least one polyvalent carboxylic acid including an ethylenically unsaturated polycarboxylic acid and at least one diol; and
vinyl ester prepolymer obtained by acrylic-modifying a terminal of epoxy group.

Among the above, the monomer copolymerizable with the methacrylic acid is preferably at least one selected from the group consisting of methacrylic ester and acrylic ester. The monomer copolymerizable with the methacrylic acid is more preferably methacrylic ester and particularly preferably methyl methacrylate.

The monomer copolymerizable with the methacrylic acid may be one kind or two or more kinds. In addition, when the component A4 is a monomer copolymerizable with the methacrylic acid, the component A4 may be used as the monomer copolymerizable with the methacrylic acid, or another monomer copolymerizable with the methacrylic acid may be used separately from the component A4.

In the polymerizable composition, a contained amount of the monomer copolymerizable with the methacrylic acid is preferably 50.00 to 99.99 parts by mass with respect to 100 parts by mass of the polymerizable composition. The lower limit of the contained amount of the monomer copolymerizable with the methacrylic acid is more preferably 60 parts by mass or more, still more preferably 70 parts by mass or more, particularly preferably 80 parts by mass or more, and most preferably 90 parts by mass or more with respect to 100 parts by mass of the polymerizable composition. The upper limit of the contained amount of the monomer copolymerizable with the methacrylic acid is more preferably 99.9 parts by mass or less and still more preferably 99 parts by mass or less with respect to 100 parts by mass of the polymerizable composition.

As the other additives, a polymerization initiator is preferable. In addition, the polymerizable composition may further comprise, as necessary, a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, a fluorescent agent, or the like. The other additives may be one kind or two or more kinds.

Examples of the polymerization initiator include the following:

azo compounds such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate;
organic peroxides such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxyisononanoate, di-t-butyl peroxide, di-t-hexyl peroxide, lauroyl peroxide, and dilauroyl peroxide;
persulfate compounds such as potassium persulfate; and
a redox polymerization initiator.

Among the above, from the viewpoint of storage stability and reactivity with the monomer copolymerizable with the methacrylic acid, the polymerization initiator is preferably at least one selected from the group consisting of an azo compound and an organic peroxide.

The amount of the polymerization initiator used is preferably 0.0001 to 1 part by mass with respect to 100 parts by mass of the total amount of the methacrylic acid and the monomer copolymerizable with the methacrylic acid.

Examples of a polymerization method for the polymerizable composition include a bulk polymerization method, a solution polymerization method, an emulsion polymerization method, and a suspension polymerization method.

The polymerization temperature is preferably 125°C to 210°C. In this manner, an appropriate polymerization rate can be obtained. The lower limit of the polymerization temperature is more preferably 130°C or higher, and the upper limit thereof is more preferably 180°C or lower. The polymerization time is not particularly limited, and can be, for example, 0.5 to 24 hours.

[Methacrylate-containing composition according to fifth aspect]

A methacrylate-containing composition according to a fifth aspect comprises methacrylic acid, a compound (component A5) represented by Formula (51), and a polymerization inhibitor (component B). The concentration of the

methacrylic acid is 98.00% to 99.99% by mass.

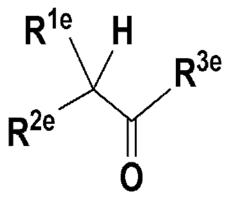

・・・(51)

In Formula (51), $R^{1e}$ and $R^{2e}$ each independently represent a hydrogen atom, an alkyl group, an alkenyl group, an aryl group, a hydroxy group, an alkoxy group, an alkylthio group, or an arylthio group. $R^{3e}$ represents a hydrogen atom, an alkyl group, an alkenyl group, an aryl group, a hydroxy group, an alkoxy group, an amino group, an alkylthio group, or an arylthio group. In addition, $R^{1e}$ and $R^{2e}$, $R^{2e}$ and $R^{3e}$, or $R^{3e}$ and $R^{1e}$ may be linked to each other to form a ring. Here, the total number of carbon atoms in $R^{1e}$ and $R^{2e}$ is 2 or more.

In addition, the methacrylate-containing composition may further comprise other compounds (component C) or water as long as the concentration of the methacrylic acid satisfies 98.00% to 99.99% by mass. Hereinafter, each item will be described in detail.

(Methacrylic acid)

The methacrylate-containing composition according to the fifth aspect comprises methacrylic acid. The methacrylic acid can be produced, for example, as a by-product of an acetone cyanohydrin (ACH) method or an intermediate of a C4 direct oxidation method. The methacrylic acid contained in the methacrylate-containing composition is preferably produced by a C4 direct oxidation method, and more preferably produced by a C4 direct oxidation method using isobutanol derived from a biomass as a starting raw material.

(Component A5)

The methacrylate-containing composition according to the fifth aspect comprises the compound (component A5) represented by Formula (51). "$\alpha$-hydrogen" represents a hydrogen atom bonded to a carbon atom adjacent to a carbon atom of a carbonyl group. By coexisting the component A5 and the component B described later, it is possible to suppress decomposition of the polymerization inhibitor. The reason for this is presumed to be as follows.

It is known that the methacrylic acid is polymerized by radicals generated during storage, and the polymerization inhibitor (component B) is added to prevent the polymerization. The component B has a function of preventing polymerization by trapping radicals generated during the storage of methacrylic acid, but the polymerization inhibitor is decomposed into another compound when trapping the radicals. Therefore, the concentration of the component B in the methacrylate-containing composition gradually decreases during the storage, and the function of preventing polymerization gradually decreases. Furthermore, unnecessary decomposition products generated by trapping the radicals by the component B gradually increase. Examples of the radicals generated during the storage of methacrylic acid include a hydroxyl radical generated by an oxygen molecule absorbing ultraviolet light derived from sunlight. The component A5 can trap a radical intermediate generated by a reaction between the hydroxyl radical and the methacrylic acid, and return the intermediate to the methacrylic acid. Accordingly, since the number of radicals which need to be trapped by the component B is reduced, the decrease in concentration of the component B can be suppressed.

In addition, when the methacrylate-containing composition comprises a carboxylic acid such as propionic acid and acrylic acid as an impurity, the concentration of the carboxylic acid is reduced during storage. This is presumed to be due to decomposition through decarboxylation. Since the component A5 serves as a proton donor, it is presumed that the component A5 can donate a proton to an anion intermediate generated by the decarboxylation, thereby promoting the decarboxylation.

The molecular weight of the component A5 is preferably 1,000 or less. When the molecular weight thereof is 1,000 or less, the number of $\alpha$-hydrogens per unit mass in the component A5 can be increased, and thus the effect of the present invention can be obtained with a small mass. The molecular weight of the component A5 is more preferably 800 or less, still more preferably 600 or less, and particularly preferably 400 or less.

$R^{1e}$ and $R^{2e}$ in Formula (51) each independently represent a hydrogen atom, an alkyl group, an alkenyl group, an aryl group, a hydroxy group, an alkoxy group, an alkylthio group, an arylthio group, or an alkylamino group. $R^{1e}$ and $R^{2e}$ may be the same or different from each other. In addition, $R^{3e}$ in Formula (51) represents a hydrogen atom, an alkyl group, an alkenyl group, an aryl group, a hydroxy group, an alkoxy group, an amino group, an alkylthio group, or an arylthio group. $R^{3e}$ and $R^{1e}$, and $R^{3e}$ and $R^{2e}$ may be the same or different from each other. In addition, $R^{1e}$ and $R^{2e}$, $R^{2e}$ and $R^{3e}$, or $R^{3e}$ and $R^{1e}$ may be linked to each other to form a ring.

In general, the α-hydrogen of the component A5 has a property of reacting with an anion or a radical, but reactivity may be reduced depending on the type of a substituent. When $R^{1e}$, $R^{2e}$, and $R^{3e}$ satisfy the above-described conditions, the reactivity of the α-hydrogen of the component A5 with an anion or a radical is maintained, and thus the effect of the present invention can be obtained. $R^{1e}$ and $R^{2e}$ are preferably a hydrogen atom, an alkyl group having 1 to 5 carbon atoms, an alkenyl group having 2 to 5 carbon atoms, an aryl group having 6 to 12 carbon atoms, a hydroxy group, an alkoxy group having 1 to 6 carbon atoms, an alkylthio group having 1 to 5 carbon atoms, an arylthio group having 6 to 10 carbon atoms, or an alkylamino group having 0 to 6 carbon atoms. Since these groups are substituents having high stability, the component A5 can be prevented from being changed into other compounds during storage. In addition, since the above-described substituent has low electron-donating properties, the acidity of the α-hydrogen of the component A5 is improved. $R^{1e}$ and $R^{2e}$ are more preferably a hydrogen atom, an alkyl group having 1 to 5 carbon atoms, an aryl group having 6 to 12 carbon atoms, an alkoxy group having 1 to 6 carbon atoms, an alkylthio group having 1 to 5 carbon atoms, an arylthio group having 6 to 10 carbon atoms, or an alkylamino group having 0 to 6 carbon atoms; still more preferably a hydrogen atom, an alkyl group having 1 to 5 carbon atoms, an aryl group having 6 to 12 carbon atoms, an alkoxy group having 1 to 6 carbon atoms, or an alkylamino group having 0 to 6 carbon atoms; particularly preferably a hydrogen atom, an alkyl group having 1 to 5 carbon atoms, an alkoxy group having 1 to 6 carbon atoms, or an alkylamino group having 0 to 6 carbon atoms; especially preferably a hydrogen atom, a methyl group, an ethyl group, an n-propyl group, an isopropyl group, an n-butyl group, an s-butyl group, an isobutyl group, a hydroxy group, a methoxy group, a cyclohexyl group, or an N,N-dimethylamino group; and most preferably a hydrogen atom, a methyl group, an ethyl group, an n-propyl group, an isopropyl group, a hydroxy group, a methoxy group, a cyclohexyl group, or an N,N-dimethylamino group.

In addition, $R^{3e}$ is more preferably a hydrogen atom, an alkyl group having 1 to 5 carbon atoms, an alkenyl group having 2 to 5 carbon atoms, an aryl group having 6 to 12 carbon atoms, a hydroxy group, an alkoxy group having 1 to 6 carbon atoms, an amino group having 0 to 6 carbon atoms, an alkylthio group having 1 to 5 carbon atoms, or an arylthio group having 6 to 10 carbon atoms. Since these groups are substituents having high stability, the component A5 can be prevented from being changed into other compounds during storage. $R^{3e}$ is more preferably a hydrogen atom, an alkyl group having 1 to 5 carbon atoms, an alkenyl group having 2 to 5 carbon atoms, a hydroxy group, an alkoxy group having 1 to 6 carbon atoms, or an amino group having 0 to 6 carbon atoms; still more preferably a hydrogen atom, an alkyl group having 1 to 5 carbon atoms, an alkenyl group having 2 to 5 carbon atoms, an amino group having 0 to 6 carbon atoms, or a hydroxy group; especially preferably a hydrogen atom, a methyl group, an ethyl group, an n-propyl group, an isopropyl group, an isopropenyl group, or a hydroxy group; and most preferably a hydrogen atom, a methyl group, an isopropyl group, or a hydroxy group.

The alkyl group is a chain (linear or branched) alkyl group or a cyclic alkyl group. As the chain alkyl group, a chain alkyl group having 1 to 20 carbon atoms is preferable, a chain alkyl group having 1 to 10 carbon atoms is more preferable, and a chain alkyl group having 1 to 5 carbon atoms is still more preferable. Examples of the linear alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, or an isopropyl group is preferable. In addition, as the cyclic alkyl group, a cyclic alkyl group having 3 to 20 carbon atoms is preferable, a cyclic alkyl group having 4 to 10 carbon atoms is more preferable, and a cyclic alkyl group having 5 to 7 carbon atoms is still more preferable. Examples of the cyclic alkyl group include a cyclopentyl group, a cyclohexyl group, and a cyclooctyl group.

The alkenyl group is a chain (linear or branched) alkenyl group or a cyclic alkenyl group. As the chain alkenyl group, a chain alkenyl group having 2 to 20 carbon atoms is preferable, a chain alkenyl group having 2 to 10 carbon atoms is more preferable, and a chain alkenyl group having 2 to 5 carbon atoms is still more preferable. Examples of the chain alkenyl group include a vinyl group, a 1-propenyl group, an isopropenyl group, a 2-butenyl group, a 1,3-butadienyl group, a 2-pentenyl group, and a 2-hexenyl group. As the cyclic alkenyl group, a cyclic alkenyl group having 3 to 20 carbon atoms is preferable, a cyclic alkenyl group having 4 to 10 carbon atoms is more preferable, and a cyclic alkenyl group having 5 to 7 carbon atoms is still more preferable. Examples of the cyclic alkenyl group include a cyclopentenyl group and a cyclohexenyl group.

The alkoxy group is preferably an alkoxy group having 1 to 20 carbon atoms, more preferably an alkoxy group having 1 to 10 carbon atoms, and still more preferably an alkoxy group having 1 to 6 carbon atoms. Examples of the alkoxy group include a methoxy group, an ethoxy group, a butoxy group, and a phenoxy group.

The amino group includes an amino group ($-NH_2$) having no substituent on a nitrogen atom (number of carbon atoms: 0) and an amino group in which a part or all of hydrogen atoms bonded to the nitrogen atom are substituted with carbon atoms. The number of carbon atoms in the amino group in which a part or all of hydrogen atoms bonded to the nitrogen atom are substituted with carbon atoms is preferably 1 to 20, more preferably 1 to 10, and still more preferably 1 to 6. Examples of the amino group include an amino group having no substituent ($-NH_2$), a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

The alkylthio group is preferably an alkylthio group having 1 to 20 carbon atoms, more preferably an alkylthio group having 1 to 10 carbon atoms, and still more preferably an alkylthio group having 1 to 5 carbon atoms. Examples of the alkylthio group include a methylthio group, an ethylthio group, a propylthio group, and an isopropylthio group.

The arylthio group is preferably an arylthio group having 1 to 20 carbon atoms, more preferably an arylthio group having 3 to 10 carbon atoms, and still more preferably an arylthio group having 6 to 10 carbon atoms. Examples of the arylthio group include a phenylthio group and a tolylthio group.

The aryl group is preferably an aryl group having 1 to 20 carbon atoms, and more preferably an aryl group having 1 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

$R^{1e}$ and $R^{2e}$, $R^{2e}$ and $R^{3e}$, or $R^{3e}$ and $R^{1e}$ may be linked to each other to form a ring. Examples of a compound in which $R^{1e}$ and $R^{2e}$ are linked to each other to form a ring include cyclohexanecarboxylic acid, methyl cyclohexanecarboxylate, cyclopentanecarboxylic acid, and methyl cyclopentanecarboxylate. Examples of a compound in which $R^{2e}$ and $R^{3e}$ are linked to each other to form a ring and a compound in which $R^{3e}$ and $R^{1e}$ are linked to each other to form a ring include 2,5-dimethyl-2-cyclopenten-1-one, 2-methyl-5-isopropyl-1-cyclohexanone, $\alpha$-methyl-$\delta$-valerolactone, and $\alpha$-methyl-$\gamma$-butyrolactone.

Among the compounds satisfying the above-described conditions, from the viewpoint of quality stability of the methacrylate-containing composition during storage, as the component A5, isobutyric acid, isovaleric acid, 2-methylbutyric acid, acetonylacetone, 3-methyl-3-buten-2-one, isobutylaldehyde, cyclohexanecarboxylic acid, 2-methoxypropionic acid, N,N-dimethylglycine, malonic acid, methylthioacetic acid, 3-butenic acid, butyric acid, palmitic acid, stearic acid, methyl isobutyrate, ethyl isobutyrate, isobutyrate isobutyl, isobutyrate isoamyl, isobutyrate phenyl, butyric acid methyl, methyl isovalerate, methyl isovalerate, methyl isopropyl ketone, 3-hexen-2-one, 2,5-dimethyl-2-cyclopentene-1-one, 2-methyl-5-isopropyl-1-cyclohexanone, 2-acetylfuran, butylaldehyde, 2-methylbutylaldehyde, 3-methylbutylaldehyde, (R)-(-)-3-hydroxyisobutyric acid, cyclopentanecarboxylic acid, $\alpha$-methyl-$\delta$-valerolactone, or $\alpha$-methyl-$\gamma$-butyrolactone is preferable; isobutyric acid, isovaleric acid, 2-methylbutyric acid, acetonylacetone, 3-methyl-3-buten-2-one, isobutylaldehyde, cyclohexanecarboxylic acid, 2-methoxypropionic acid, N,N-dimethylglycine, malonic acid, methylthioacetic acid, 3-butenic acid, or methyl isopropenyl ketone is more preferable; isobutyric acid, isovaleric acid, 2-methylbutyric acid, 2-methoxypropionic acid, N,N-dimethylglycine, acetonylacetone, 3-methyl-3-buten-2-one, isobutylaldehyde, or methyl isopropenyl ketone is still more preferable; and isobutyric acid, isovaleric acid, 2-methylbutyric acid, acetonylacetone, isopropenyl methyl ketone, isobutylaldehyde, cyclohexanecarboxylic acid, 2-methoxypropionic acid, or N,N-dimethylglycine is particularly preferable.

The component A5 may be one kind or two or more kinds.

(Component B)

The methacrylate-containing composition according to the fifth aspect comprises a polymerization inhibitor (component B). In the present specification, the polymerization inhibitor means a compound having a function of suppressing the polymerization reaction of the methacrylic acid. Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress a polymerization reaction of the methacrylic acid by a radical polymerization mechanism during the storage of the methacrylic acid. In addition, as described above, by coexisting the component A5 and the component B, it is possible to efficiently suppress the decrease of the component B.

Examples of the polymerization inhibitor as the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, tris(nonylphenyl)phosphite, 4,4'-isopropylidenediphenol alkylphosphite, tris(2,4-di-tert-butylphenyl)phosphite, tris(biphenyl) phosphite, distearyl pentaerythritol diphosphite, di(2,4-di-tert-butylphenyl)pentaerythritol diphosphite, di(nonylphenyl) pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among the above, from the viewpoint of quality stability of the methacrylate-containing composition during storage, the component B is preferably at least one polymerization inhibitor selected from the group consisting of a phenol-based compound, an N-oxyl-based compound, an amine-based compound, a phosphorus-containing compound, and a sulfur-containing compound, and for example, at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is preferable. As the component B, a phenol-based compound is more preferable, and for example, at least one polymerization inhibitor selected from the group consisting of hydroquinone, 4-methoxyphenol, 2,4-dimethyl-6-t-butylphenol, and 2,6-di-t-butyl-4-methylphenol is more preferable. As the component B, for example, at least one polymerization inhibitor selected from the group consisting of hydroquinone and 4-methoxyphenol is particularly preferable.

The component B may be one kind or two or more kinds.

When the methacrylate-containing composition comprises a compound corresponding to both the component A5 and the component B, the compound is regarded as the component B. That is, the methacrylate-containing composition needs to contain another component A5 different from the compound. When the methacrylate-containing composition comprises two or more kinds of compounds corresponding to both the component A5 and the component B, a compound having the highest molar concentration in the methacrylate-containing composition is regarded as the component B, and the other compounds are regarded as the component A5.

(Concentrations of component A5 and component B)

When the concentration of the component A5 is indicated by $M_{A5}$ (μmol/L) and the concentration of the component B is indicated by $M_B$ (μmol/L), $M_B/M_{A5}$ is preferably 0.005 to 100. From the viewpoint of efficiency of suppressing the generation of pyruvic acid, the lower limit of $M_B/M_{A5}$ is more preferably 0.05 or more, and the upper limit thereof is more preferably 10 or less.

$M_{A5}$ is preferably 1 to 40,000 μmol/L. When $M_{A5}$ is 1 μmol/L or more, the effect of suppressing the decomposition of the polymerization inhibitor can be sufficiently obtained. In addition, when the $M_{A5}$ is 40,000 μmol/L or less, the amount of impurities when a methacrylic acid polymer is produced by polymerization of the methacrylate-containing composition according to the fifth aspect can be reduced, and thus it is possible to prevent physical properties of the polymer from being adversely affected. The lower limit of $M_{A5}$ is more preferably 10 μmol/L or more, still more preferably 30 μmol/L or more, even more preferably 50 μmol/L or more, and even still more preferably 55 μmol/L or more. The upper limit of $M_{A3}$ is more preferably 30,000 μmol/L or less, still more preferably 25,000 μmol/L or less, and particularly preferably 20,000 μmol/L or less.

$M_B$ is preferably 1 to 50,000 μmol/L. When $M_B$ is 1 μmol/L or more, the effect of suppressing generation of a methacrylic acid dimer and pyruvic acid can be sufficiently obtained. In addition, when the $M_B$ is 50,000 μmol/L or less, the amount of impurities when a methacrylic acid polymer is produced by polymerization of the methacrylate-containing composition according to the fifth aspect can be reduced, and thus it is possible to prevent physical properties of the polymer from being adversely affected. The lower limit of $M_B$ is more preferably 10 μmol/L or more, still more preferably 100 μmol/L or more, particularly preferably 1,500 μmol/L or more, and especially preferably 2,000 μmol/L or more. The upper limit of $M_B$ is more preferably 45,000 μmol/L or less, still more preferably 40,000 μmol/L or less, particularly preferably 35,000 μmol/L or less, especially preferably 30,000 μmol/L or less, and most preferably 25,000 μmol/L or less.

(Concentration of methacrylic acid)

The concentration of the methacrylic acid in the methacrylate-containing composition according to the fifth aspect is 98.00% to 99.99% by mass. When the concentration of the methacrylic acid is 98.00% by mass or more, the amount of impurities when a methacrylic acid polymer is produced by polymerization of the methacrylate-containing composition according to the fifth aspect can be reduced, and thus it is possible to prevent physical properties of the polymer from being adversely affected. In addition, when the concentration of the methacrylic acid is 99.99% by mass or less, a purification cost can be reduced. The lower limit of the concentration of the methacrylic acid is more preferably 98.50% by mass or more, still more preferably 99.00% by mass or more, particularly preferably 99.50% by mass or more, and most preferably 99.80% by mass or more.

(Component C)

The methacrylate-containing composition according to the fifth aspect may further comprise other compounds as the component C as long as the concentration of the methacrylic acid satisfies 98.00% to 99.99% by mass. Examples of the component C include impurities generated in the process of producing the methacrylic acid. For example, the methacrylic acid may contain diacetyl as an impurity, but from the viewpoint of reducing coloration of the methacrylate-containing composition, the concentration of the diacetyl is preferably 55 μmol/L or less, more preferably 20 μmol/L or less, still more preferably 10 μmol/L or less, and particularly preferably 1 μmol/L or less.

(Analysis of methacrylate-containing composition)

The fact that the methacrylate-containing composition comprises the component A5, the component B, the component C, and the water can be confirmed by, for example, GC-MS measurement. In a GC-MS chart of the methacrylate-containing composition, when a peak is present at the same retention time as a sample of the component A5 and an m/z value detected in a mass spectrum of the peak matches exact mass of the component A5, it can be determined that the methacrylate-containing composition comprises the component A5. When the sample of the component A5 cannot be obtained, when a pattern of the mass spectrum of the peak appearing in the GC-MS chart of the methacrylate-containing composition and a pattern of a mass spectrum of the component A5 in mass spectrum database and match each other, it can be determined that the peak is the peak of the component A5. That is, it can be determined that the methacrylate-containing composition comprises the component A5. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

In addition, the concentration of the methacrylic acid can be calculated, for example, by performing GC-FID measurement of the methacrylate-containing composition, quantifying by an area percentage method, and correcting the quantified moisture concentration using a Karl Fischer moisture meter. The concentration of the component A5 can be quantified, for example, by performing GC measurement of the methacrylate-containing composition and using an internal standard method. When a sample of the component A5 cannot be obtained and the component A5 cannot be quantified by the internal standard method, the concentration of the component A5 can be calculated using the following expression by performing GC-FID measurement on any organic compound having a known concentration under the same conditions as those of the methacrylate-containing composition.

$$\text{Concentration of component A5 } (\mu\text{mol/L}) = \frac{N}{N_{A5}} \times \frac{S_{A5}}{S} \times M$$

Here, N is the number of carbon atoms in one molecule of the organic compound having a known concentration, $N_{A5}$ is the number of carbon atoms in one molecule of the component A5, $S_{A5}$ is a peak area of the component A5, S is a peak area of the organic compound having a known concentration, and M is the concentration (μmol/L) of the organic compound having a known concentration.

When the volatility is low and the quantification cannot be performed by the GC measurement, the quantification can be performed using a chromatography method such as LC.

Concentrations of the component B and the component C can also be calculated by the same method as that for the component A5 described above.

In addition, the fact that the methacrylate-containing composition comprises water, and the concentration thereof can be confirmed by Karl Fischer method.

[Method for producing methacrylate-containing composition]

Examples of a method for producing the methacrylate-containing composition according to the fifth aspect include a method of adding the component A5 and the component B to methacrylic acid. As the methacrylic acid, a commercially available product may be used, or methacrylic acid produced by a known method such as an acetone cyanohydrin (ACH) method and a C4 direct oxidation method may be used. As the component A5 and the component B, a commercially available product may be used, or a component synthesized by a known method may be used. When methacrylic acid produced by a known method such as an acetone cyanohydrin (ACH) method and a C4 direct oxidation method is used, the methacrylate-containing composition may be produced by adding the component A5 or the component B in the middle of the process of a raw material step or a production step. In addition, when the component A5 or the component B is generated as a by-product in the methacrylic acid production process, the methacrylate-containing composition may be produced by leaving a part of the generated component A5 or component B.

[Evaluation method for storage stability and thermal stability]

The methacrylate-containing composition according to the fifth aspect has high quality stability during storage. Examples of an evaluation method for the quality stability of the methacrylate-containing composition during storage include a method of actually storing the methacrylate-containing composition for a long period of time and confirming the amount of the polymerization inhibitor reduced. In addition, from the viewpoint of ease of work, a method of heating the methacrylate-containing composition for a short period of time and confirming the amount of the polymerization inhibitor reduced may be used. When heating for a short period of time, the heating temperature is preferably 50°C to 100°C and a heating time is preferably 1 to 24 hours. In the fifth aspect, the quality stability of the methacrylate-containing composition during storage is evaluated by the amount of decrease in polymerization inhibitor when the methacrylate-containing composition is stored at 25°C for 21 days.

[Method for producing methacrylic ester]

A method for producing a methacrylic ester according to the fifth aspect includes a step of esterifying the methacrylate-containing composition according to the fifth aspect.

The alcohol to be reated with the methacrylate-containing composition is not particularly limited, and examples thereof include methanol, ethanol, n-propanol, isopropanol, n-butanol, and isobutanol. Examples of the methacrylic ester to be obtained include methyl methacrylate, ethyl methacrylate, propyl methacrylate, isopropyl methacrylate, butyl methacrylate, and isobutyl methacrylate. The esterification reaction can be carried out in the presence of an acidic catalyst such as a sulfonic acid-type cation exchange resin. The temperature during the esterification reaction is preferably 50°C to 200°C.

The pressure during the esterification reaction, the location of the catalyst in the reaction vessel, the proportion of the catalyst in the reaction vessel, and the like are not particularly limited, and a commonly used form can be adopted.

[Method for producing methacrylic acid polymer according to fifth aspect]

A method for producing a methacrylic acid polymer according to the embodiment of the present invention includes a step of polymerizing a polymerizable composition comprising the methacrylate-containing composition according to the embodiment of the present invention.

The polymerizable composition may further comprise a monomer copolymerizable with the methacrylic acid and other additive substances, as necessary.

Examples of the monomer copolymerizable with the methacrylic acid include the following:

methacrylic esters such as ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;
acrylic esters such as methyl acrylate, ethyl acrylate, n-butyl acrylate, iso-butyl acrylate, tert-butyl acrylate, and 2-ethylhexyl acrylate;
unsaturated carboxylic acids such as acrylic acid, methacrylic acid, maleic acid, and itaconic acid;
unsaturated carboxylic anhydrides such as maleic anhydride and itaconic anhydride;
maleimides such as N-phenylmaleimide and N-cyclohexylmaleimide;
hydroxy group-containing vinyl monomers such as 2-hydroxyethyl acrylate, 2-hydroxyethyl methacrylate, and 2-hydroxypropyl methacrylate;
vinyl esters such as vinyl acetate and vinyl benzoate;
vinyl chloride, vinylidene chloride, and derivatives thereof;
nitrogen-containing vinyl monomers such as methacrylamide and acrylonitrile;
epoxy group-containing monomers such as glycidyl acrylate and glycidyl methacrylate;
aromatic vinyl monomers such as styrene and $\alpha$-methylstyrene;
alkanediol di(meth)acrylates such as ethylene glycol di(meth)acrylate, 1,2-propylene glycol di(meth)acrylate, 1,3-butylene glycol di(meth)acrylate, and 1,6-hexanediol di(meth)acrylate;
polyoxyalkylene glycol di(meth)acrylates such as diethylene glycol di(meth)acrylate, dipropylene glycol di(meth) acrylate, triethylene glycol (meth)acrylate, tetraethylene glycol di(meth)acrylate, polyethylene glycol di(meth)acrylate, and neopentyl glycol di(meth)acrylate;
vinyl monomers having two or more ethylenically unsaturated bonds in a molecule, such as divinylbenzene;
unsaturated polyester prepolymer obtained from at least one polyvalent carboxylic acid including an ethylenically unsaturated polycarboxylic acid and at least one diol; and

vinyl ester prepolymer obtained by acrylic-modifying a terminal of epoxy group.

Among the above, the monomer copolymerizable with the methacrylic acid is preferably at least one selected from the group consisting of methacrylic ester and acrylic ester. The monomer copolymerizable with the methacrylic acid is more preferably methacrylic ester and particularly preferably methyl methacrylate.

The monomer copolymerizable with the methacrylic acid may be one kind or two or more kinds.

In the polymerizable composition, a contained amount of the monomer copolymerizable with the methacrylic acid is preferably 50.00 to 99.99 parts by mass with respect to 100 parts by mass of the polymerizable composition. The lower limit of the contained amount of the monomer copolymerizable with the methacrylic acid is more preferably 60 parts by mass or more, still more preferably 70 parts by mass or more, particularly preferably 80 parts by mass or more, and most preferably 90 parts by mass or more with respect to 100 parts by mass of the polymerizable composition. The upper limit of the contained amount of the monomer copolymerizable with the methacrylic acid is more preferably 99.9 parts by mass or less and still more preferably 99 parts by mass or less with respect to 100 parts by mass of the polymerizable composition.

As the other additives, a polymerization initiator is preferable. In addition, the polymerizable composition may further comprise, as necessary, a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, a fluorescent agent, or the like. The other additives may be one kind or two or more kinds.

Examples of the polymerization initiator include the following:

azo compounds such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate;
organic peroxides such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxyisononanoate, di-t-butyl peroxide, di-t-hexyl peroxide, lauroyl peroxide, and dilauroyl peroxide;
persulfate compounds such as potassium persulfate; and
a redox polymerization initiator.

Among the above, from the viewpoint of storage stability and reactivity with the monomer copolymerizable with the methacrylic acid, the polymerization initiator is preferably at least one selected from the group consisting of an azo compound and an organic peroxide.

The amount of the polymerization initiator used is preferably 0.0001 to 1 part by mass with respect to 100 parts by mass of the total amount of the methacrylic acid and the monomer copolymerizable with the methacrylic acid.

Examples of a polymerization method for the polymerizable composition include a bulk polymerization method, a solution polymerization method, an emulsion polymerization method, and a suspension polymerization method.

The polymerization temperature is preferably 125°C to 210°C. In this manner, an appropriate polymerization rate can be obtained. The lower limit of the polymerization temperature is more preferably 130°C or higher, and the upper limit thereof is more preferably 180°C or lower. The polymerization time is not particularly limited, and can be, for example, 0.5 to 24 hours.

[Methacrylate-containing composition according to sixth aspect]

A methacrylate-containing composition according to a sixth aspect comprises methacrylic acid, a compound (component A6) represented by Formula (61), and a polymerization inhibitor (component B). The concentration of the methacrylic acid is 98.00% to 99.99% by mass.

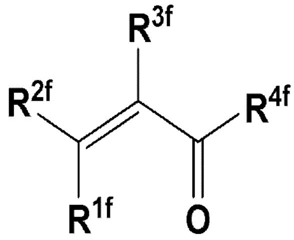

· · · (61)

In Formula (61), $R^{1f}$, $R^{2f}$, and $R^{3f}$ each independently represent a hydrogen atom, an alkyl group, an alkenyl group, an aryl group, a hydroxy group, an alkoxy group, an amino group, an alkylthio group, or an arylthio group. $R^{4f}$ represents an alkyl group, an alkenyl group, an aryl group, a hydroxy group, an alkoxy group, an amino group, a monovalent group including a carbonyl group, an alkylthio group, or an arylthio group. Here, a case where $R^{1f}$ = H, $R^{2f}$ = H, $R^{3f}$ = $CH_3$, and $R^{4f}$ = OH, that is, a case where the compound represented by Formula (61) is methacrylic acid is excluded. H represents a hydrogen atom, C represents a carbon atom, and O represents an oxygen atom.

In addition, the methacrylate-containing composition may further comprise other compounds (component C) or water as long as the concentration of the methacrylic acid satisfies 98.00% to 99.99% by mass. Hereinafter, each item will be described in detail.

(Methacrylic acid)

The methacrylate-containing composition according to the sixth aspect comprises methacrylic acid. The methacrylic acid can be produced, for example, as a by-product of an acetone cyanohydrin (ACH) method or an intermediate of a C4 direct oxidation method. The methacrylic acid contained in the methacrylate-containing composition is preferably produced by a C4 direct oxidation method, and more preferably produced by a C4 direct oxidation method using isobutanol derived from a biomass as a starting raw material.

(Component A6)

The methacrylate-containing composition according to the sixth aspect comprises the compound (component A6) represented by Formula (61). By coexisting the component A6 and the component B described later, it is possible to suppress decomposition of the polymerization inhibitor during storage of the methacrylate-containing composition. In addition, when the methacrylate-containing composition comprises impurities such as acrylic acid and propionic acid, decomposition of the impurities can be promoted to reduce the concentration of the impurities. The reason for this is presumed to be as follows.

It is known that the methacrylic acid is polymerized by radicals generated during storage, and the polymerization inhibitor (component B) is added to prevent the polymerization. The component B has a function of preventing polymerization by trapping radicals generated during the storage of methacrylic acid, but the polymerization inhibitor is decomposed into another compound when trapping the radicals. Therefore, the concentration of the component B in the methacrylate-containing composition gradually decreases during the storage, and the function of preventing polymerization gradually decreases. Furthermore, unnecessary decomposition products generated by trapping the radicals by the component B gradually increase. Examples of the radicals generated during the storage of methacrylic acid include a hydroxyl radical generated by an oxygen molecule absorbing ultraviolet light derived from sunlight. In addition, the hydroxyl radical also causes generation of pyruvic acid by oxidation of the methacrylic acid. Since the component A6 has a conjugated double bond, the component A6 absorbs the ultraviolet light, and an absorption wavelength thereof changes depending on the type of the substituent. The component A6 can absorb ultraviolet light in a wide wavelength range. Therefore, when the methacrylate-containing composition comprises the component A6, the ultraviolet light having a wide wavelength range is absorbed, and the generation of the hydroxyl radical is suppressed. Accordingly, since the number of radicals which need to be trapped by the component B is reduced, the decrease in concentration of the component B can be suppressed.

Among various organic compounds which absorb ultraviolet light, the component A6 has a molecular structure similar to that of the methacrylic acid, so that, when a polymer is produced using the methacrylate-containing composition as a raw material, it is possible to reduce the adverse effect of the component A6 which is contained as an impurity.

The molecular weight of the component A6 is preferably 1,000 or less. When the molecular weight is 1,000 or less, the number of conjugated double bonds per unit mass in the component A6 can be increased, and thus the effect of the present invention can be obtained with a small mass. The molecular weight of the component A6 is more preferably 800 or less, still more preferably 600 or less, and particularly preferably 400 or less.

$R^{1f}$, $R^{2f}$, and $R^{3f}$ in Formula (61) each independently represent a hydrogen atom, an alkyl group, an alkenyl group,

an aryl group, a hydroxy group, an alkoxy group, an amino group, an alkylthio group, or an arylthio group. In addition, $R^{4f}$ in Formula (61) represents an alkyl group, an alkenyl group, an aryl group, a hydroxy group, an alkoxy group, an amino group, a monovalent group including a carbonyl group, an alkylthio group, or an arylthio group. $R^{1f}$, $R^{2f}$, $R^{3f}$, and $R^{4f}$ may be the same or different from each other.

When $R^{1f}$, $R^{2f}$, $R^{3f}$, and $R^{4f}$ satisfy the above-described conditions, a $\pi$-conjugated system of the component A6 is maintained, and thus a property of absorbing ultraviolet light in a wide wavelength range is provided, and the effect of the present invention can be obtained. $R^{1f}$, $R^{2f}$, and $R^{3f}$ are preferably a hydrogen atom, an alkyl group having 1 to 10 carbon atoms, an alkenyl group having 2 to 10 carbon atoms, an aryl group having 6 to 12 carbon atoms, a hydroxy group, an alkoxy group having 1 to 6 carbon atoms, an amino group having 0 to 6 carbon atoms, an alkylthio group having 1 to 5 carbon atoms, or an arylthio group having 6 to 10 carbon atoms. Since these groups are substituents having high stability, the component A6 can be prevented from being changed into other compounds during storage. In addition, when these substituents are included, a sufficient amount of ultraviolet light which can be absorbed by one molecule of the component A6 is obtained. $R^{1f}$, $R^{2f}$, and $R^{3f}$ are more preferably a hydrogen atom, an alkyl group having 1 to 5 carbon atoms, or an aryl group having 6 to 12 carbon atoms; still more preferably a hydrogen atom, an alkyl group having 1 to 5 carbon atoms, or an aryl group having 6 to 12 carbon atoms, in which at least one or more thereof are the alkyl group having 1 to 5 carbon atoms or the aryl group having 6 to 12 carbon atoms; particularly preferably a hydrogen atom, a methyl group, an ethyl group, an n-propyl group, an isopropyl group, or a phenyl group, in which at least one or more thereof are the methyl group, the ethyl group, the n-propyl group, the isopropyl group, or the phenyl group; and particularly preferably a hydrogen atom, a methyl group, an ethyl group, an isopropyl group, or a phenyl group, in which at least one or more thereof are the methyl group, the ethyl group, the isopropyl group, or the phenyl group.

In addition, $R^{4f}$ is preferably an alkyl group having 1 to 10 carbon atoms, an alkenyl group having 2 to 10 carbon atoms, an aryl group having 6 to 12 carbon atoms, a hydroxy group, an alkoxy group having 1 to 6 carbon atoms, an amino group having 0 to 6 carbon atoms, a monovalent group including a carbonyl group having 1 to 6 carbon atoms, an alkylthio group having 1 to 5 carbon atoms, or an arylthio group having 6 to 10 carbon atoms. Since these groups are substituents having high stability, the component A6 can be prevented from being changed into other compounds during storage. $R^{4f}$ is more preferably an alkyl group having 1 to 5 carbon atoms, a hydroxy group, an alkoxy group having 1 to 6 carbon atoms, or an amino group having 0 to 6 carbon atoms; particularly preferably a methyl group, an ethyl group, an n-propyl group, an isopropyl group, a hydroxy group, a methoxy group, or an amino group; and especially preferably a methyl group, a hydroxy group, a methoxy group, or an amino group. When $R^{4f}$ has the structure, the quality stability of the methacrylate-containing composition during storage can be improved.

The alkyl group is a chain (linear or branched) alkyl group or a cyclic alkyl group. An alkyl group having 1 to 20 carbon atoms is preferable, an alkyl group having 1 to 10 carbon atoms is more preferable, and an alkyl group having 1 to 5 carbon atoms is still more preferable. Examples of the linear alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, or an isopropyl group is preferable. **In** addition, examples of the cyclic alkyl group include a cyclopentyl group, a cyclohexyl group, and a cyclooctyl group.

The alkenyl group is a chain (linear or branched) alkenyl group or a cyclic alkenyl group. An alkenyl group having 2 to 20 carbon atoms is preferable, an alkenyl group having 2 to 10 carbon atoms is more preferable, and an alkenyl group having 2 to 5 carbon atoms is still more preferable. Examples of the chain alkenyl group include a vinyl group, a 1-propenyl group, an isopropenyl group, a 2-butenyl group, a 1,3-butadienyl group, a 2-pentenyl group, and a 2-hexenyl group. In addition, examples of the cyclic alkenyl group include a cyclopentenyl group and a cyclohexenyl group.

The aryl group is preferably an aryl group having 1 to 20 carbon atoms, and more preferably an aryl group having 1 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The alkoxy group is preferably an alkoxy group having 1 to 20 carbon atoms, more preferably an alkoxy group having 1 to 10 carbon atoms, and still more preferably an alkoxy group having 1 to 6 carbon atoms. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group ($-NH_2$) having no substituent on a nitrogen atom (number of carbon atoms: 0) and an amino group in which a part or all of hydrogen atoms bonded to the nitrogen atom are substituted with

carbon atoms. The number of carbon atoms in the amino group in which a part or all of hydrogen atoms bonded to the nitrogen atom are substituted with carbon atoms is preferably 1 to 20, more preferably 1 to 10, and still more preferably 1 to 6. Examples of the amino group include an amino group having no substituent (-$NH_2$), a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group include a formyl group, an acyl group, a carboxy group, an amide group, an alkoxycarbonyl group, a thiocarboxy group, and a thioester group; and a monovalent group including a carbonyl group having 1 to 6 carbon atoms is preferable.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6. Examples of the acyl group include an acetyl group, a propionyl group, a butylcarbonyl group, a vinylcarbonyl group, and a benzoyl group.

The amide group includes an amide group (-$CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group. The total number of carbon atoms derived from the carbonyl group and carbon atoms derived from the alkoxy group is preferably 2 to 20, more preferably 2 to 10, and still more preferably 2 to 6. Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group. The total number of carbon atoms derived from the carbonyl group and carbon atoms derived from the alkylthio group or the arylthio group is preferably 2 to 20, more preferably 2 to 10, and still more preferably 2 to 6. Examples of the thioester group include a methylthiocarbonyl group, an ethylthiocarbonyl group, a butylthiocarbonyl group, and a phenylthiocarbonyl group.

In addition, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

The alkylthio group is preferably an alkylthio group having 1 to 20 carbon atoms, more preferably an alkylthio group having 1 to 10 carbon atoms, and still more preferably an alkylthio group having 1 to 5 carbon atoms. Examples of the alkylthio group include a methylthio group, an ethylthio group, a propylthio group, and an isopropylthio group.

The arylthio group is preferably an arylthio group having 1 to 20 carbon atoms, more preferably an arylthio group having 3 to 10 carbon atoms, and still more preferably an arylthio group having 6 to 10 carbon atoms. Examples of the arylthio group include a phenylthio group and a tolylthio group.

Among the compounds satisfying the above-described conditions, from the viewpoint of quality stability of the methacrylate-containing composition during storage, as the component A6, crotonic acid, 2-methylcrotonic acid, 3,3-dimethylacrylic acid, 2-methylene-3-butenoic acid, 2-pentenoic acid, 2-methyl-2-pentenoic acid, methyl methacrylate, methyl acrylate, 3-methyl-3-buten-2-one (isopropenyl methyl ketone), N-propyl methacrylamide, cinnamic acid, 3-methoxyacrylic acid, cis-crotonic acid, 2-ethylacrylic acid, 2-methylene pentanoic acid, 4-methyl-2-pentenoic acid, 2,4-pentadienoic acid, 2-methyl-2,4-pentadienoic acid, 2-heptenoic acid, 2-methyl-2-heptenoic acid, 2-methylene hexanoic acid, 2-heptenoic acid, 2,4-heptadienoic acid, 2-octenoic acid, 2,4-octadienoic acid, 2,6-dimethyl-2-heptenoic acid, 6-methyl-2-methylene heptanoic acid, 2,6-dimethyl-2,4-heptadienoic acid, 6-methyl-2-methylene-3-heptenoic acid, 2,5-dimethyl-2,4-hexadienoic acid, ethyl methacrylate, isobutyl methacrylate, butyl methacrylate, propyl methacrylate, isoamyl methacrylate, cyclohexyl methacrylate, isobornyl methacrylate, hexyl methacrylate, propyl acrylate, butyl acrylate, methyl crotonate, cis-methyl crotonate, methyl 3,3-dimethylacrylate, methyl 2-ethylacrylate, methyl 2-methylene-3-butenoic acid, methyl 2-pentenoate, methyl 2,4-pentadienoate, dimethyl fumarate, trans-3-hexen-2-one, methyl vinyl ketone, acrolein, crotonaldehyde, cis-crotonaldehyde, 2-methylene-3-butanal, 3,3-dimethylacrolein, 2-ethylacrolein, 2,4-pentadienal, 2-pentanal, methyl 2-methyl-2,4-pentadienal, 4-methyl-2-pentanal, 2-methyl-2-pentanal, 2-methylene pentanal, 2-hexanal, methyl 2-hexenal, 2-methylene hexanal, 2-heptanal, 2,4-heptadienal, 2-octenal, 2,4-octadienal, 2,6-dimethyl-2-heptenal, 6-methyl-2-methylene heptanal, 2,6-dimethyl-2,4-heptadienal, 6-methyl-2-methylene-3-heptenal, 2,5-dimethyl-2,4-hexadiene diol, methacrylamide, hexadecanamide, 9-octadecenamide, or a methacrylic acid anhydride is preferable; crotonic acid, 2-methylcrotonic acid, 3,3-dimethylacrylic acid, 2-methylene-3-butenoic acid, 2-pentenoic acid, 2-methyl-2-pentenoic acid, methyl methacrylate, methyl acrylate, 3-methyl-3-buten-2-one (isopropenyl methyl ketone), N-propyl methacrylamide, cinnamic acid, or 3-methoxyacrylic acid is more preferable; crotonic acid, 2-methylcrotonic acid, 3,3-dimethylacrylic acid, 2-methylene-3-butenoic acid, 2-pentenoic acid, 2-methyl-2-pentenoic acid, methyl acrylate, 3-methyl-3-buten-2-one (isopropenyl methyl ketone), N-propyl methacrylamide, methacrylamide, or cinnamic acid is still more preferable; and crotonic acid, 2-methylcrotonic acid, 3,3-dimethylacrylic acid, 2-pentenoic acid, 2-methyl-2-pentenoic acid, methyl acrylate, isopropenyl methyl ketone, methacrylamide, or cinnamic acid is particularly preferable.

The component A6 may be one kind or two or more kinds.

(Component B)

The methacrylate-containing composition according to the sixth aspect comprises a polymerization inhibitor (component B). In the present specification, the polymerization inhibitor means a compound having a function of suppressing the polymerization reaction of the methacrylic acid. Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress a polymerization reaction of the methacrylic acid by a radical polymerization mechanism during the storage of the methacrylic acid. In addition, the component B can trap the above-described hydroxyl radical generated during the storage of the methacrylic acid. That is, when the methacrylate-containing composition comprises the component B in addition to the component A6, the amount of the hydroxyl radical can be reduced by two different mechanisms of suppressing the generation of the hydroxyl radical by the component A6 and removing the generated hydroxyl radical by the component B. Therefore, it is considered that the reduction of the component B can be efficiently suppressed.

Examples of the polymerization inhibitor as the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include $\alpha$-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, tris(nonylphenyl)phosphite, 4,4'-isopropylidenediphenol alkylphosphite, tris(2,4-di-tert-butylphenyl)phosphite, tris(biphenyl) phosphite, distearyl pentaerythritol diphosphite, di(2,4-di-tert-butylphenyl)pentaerythritol diphosphite, di(nonylphenyl) pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among the above, from the viewpoint of quality stability of the methacrylate-containing composition during storage, as the component B, at least one polymerization inhibitor selected from the group consisting of a phenol-based compound, an N-oxyl-based compound, an amine-based compound, a phosphorus-containing compound, and a sulfur-containing compound is preferable, and for example, at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is preferable. As the component B, a phenol-based compound is more preferable, and for example, at least one polymerization inhibitor selected from the group consisting of hydroquinone, 4-methoxyphenol, 2,4-dimethyl-6-t-butylphenol, and 2,6-di-t-butyl-4-methylphenol is more preferable. As the component B, for example, at least one polymerization inhibitor selected from the group consisting of hydroquinone, 4-methoxyphenol, and 2,6-di-t-butyl-4-methylphenol is still more preferable. As the component B, for example, at least one polymerization inhibitor selected from the group consisting of hydroquinone and 4-methoxyphenol is particularly preferable.

The component B may be one kind or two or more kinds.

When the methacrylate-containing composition comprises a compound corresponding to both the component A6 and the component B, the compound is regarded as the component B. That is, the methacrylate-containing composition needs to contain another component A6 different from the compound. When the methacrylate-containing composition comprises two or more kinds of compounds corresponding to both the component A6 and the component B, a compound having the highest molar concentration in the methacrylate-containing composition is regarded as the component B, and the other compounds are regarded as the component A6.

(Concentrations of component A6 and component B)

When the concentration of the component A6 is indicated by $M_{A6}$ (μmol/L) and the concentration of the component B is indicated by $M_B$ (μmol/L), from the viewpoint of efficiency of suppressing consumption of the polymerization inhibitor, $M_B/M_{A6}$ is preferably 0.0005 to 500, more preferably 0.001 to 450, still more preferably 0.005 to 400, and even

more preferably 0.005 to 350.

$M_{A6}$ is preferably 1 to 85,000 μmol/L. When $M_{A6}$ is 1 μmol/L or more, the effect of suppressing the decomposition of the polymerization inhibitor can be sufficiently obtained. In addition, when the $M_{A6}$ is 85,000 μmol/L or less, the amount of impurities when a methacrylic acid polymer is produced by polymerization of the methacrylate-containing composition according to the sixth aspect can be reduced, and thus it is possible to prevent physical properties of the polymer from being adversely affected. The lower limit of $M_{A6}$ is more preferably 10 μmol/L or more, still more preferably 30 μmol/L or more, still more preferably 50 μmol/L or more, particularly preferably 70 μmol/L or more, especially preferably 80 μmol/L or more, and most preferably 85 μmol/L or more. In addition, the upper limit of $M_{A6}$ is more preferably 65,000 μmol/L or less, still more preferably 45,000 μmol/L or less, particularly preferably 25,000 μmol/L or less, especially preferably 15,000 μmol/L or less, and most preferably 5,000 μmol/L or less.

$M_B$ is preferably 1 to 50,000 μmol/L. When $M_B$ is 1 μmol/L or more, the effect of suppressing the decomposition of the polymerization inhibitor can be sufficiently obtained. In addition, when the $M_B$ is 50,000 μmol/L or less, the amount of impurities when a methacrylic acid polymer is produced by polymerization of the methacrylate-containing composition according to the sixth aspect can be reduced, and thus it is possible to prevent physical properties of the polymer from being adversely affected. The lower limit of $M_B$ is more preferably 10 μmol/L or more, still more preferably 100 μmol/L or more, particularly preferably 1,000 μmol/L or more, and especially preferably 2,000 μmol/L or more. The upper limit of $M_B$ is more preferably 40,000 μmol/L or less, still more preferably 30,000 μmol/L or less, and particularly preferably 25,000 μmol/L or less.

(Concentration of methacrylic acid)

The concentration of the methacrylic acid in the methacrylate-containing composition according to the sixth aspect is 98.00% to 99.99% by mass. When the concentration of the methacrylic acid is 98.00% by mass or more, the amount of impurities when a methacrylic acid polymer is produced by polymerization of the methacrylate-containing composition can be reduced, and thus it is possible to prevent physical properties of the polymer from being adversely affected. In addition, when the concentration of the methacrylic acid is 99.99% by mass or less, a purification cost can be reduced. The lower limit of the concentration of the methacrylic acid is more preferably 98.50% by mass or more, still more preferably 99.00% by mass or more, particularly preferably 99.50% by mass or more, and most preferably 99.80% by mass or more.

(Component C)

The methacrylate-containing composition according to the sixth aspect may further comprise other compounds as the component C as long as the concentration of the methacrylic acid satisfies 98.00% to 99.99% by mass. Examples of the component C include impurities generated in the process of producing the methacrylic acid. For example, the methacrylic acid may contain diacetyl as an impurity, but from the viewpoint of reducing coloration of the methacrylate-containing composition, the concentration of the diacetyl is preferably 55 μmol/L or less, more preferably 20 μmol/L or less, still more preferably 10 μmol/L or less, and particularly preferably 1 μmol/L or less.

(Analysis of methacrylate-containing composition)

The fact that the methacrylate-containing composition comprises the component A6, the component B, the component C, and the water can be confirmed by, for example, GC-MS measurement. In a GC-MS chart of the methacrylate-containing composition, when a peak is present at the same retention time as a sample of the component A6 and an m/z value detected in a mass spectrum of the peak matches exact mass of the component A6, it can be determined that the methacrylate-containing composition comprises the component A6. When the sample of the component A6 cannot be obtained, when a pattern of the mass spectrum of the peak appearing in the GC-MS chart of the methacrylate-containing composition and a pattern of a mass spectrum of the component A6 in mass spectrum database and match each other, it can be determined that the peak is the peak of the component A6. That is, it can be determined that the methacrylate-containing composition comprises the component A6. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

In addition, the concentration of the methacrylic acid can be calculated, for example, by performing GC-FID measurement of the methacrylate-containing composition, quantifying by an area percentage method, and correcting the quantified moisture concentration using a Karl Fischer moisture meter. The concentration of the component A6 can be quantified, for example, by performing GC measurement of the methacrylate-containing composition and using an internal standard method. When a sample of the component A6 cannot be obtained and the component A6 cannot be quantified by

the internal standard method, the concentration of the component A6 can be calculated using the following expression by performing GC-FID measurement on any organic compound having a known concentration under the same conditions as those of the methacrylate-containing composition.

$$\text{Concentration of component A6 } (\mu\text{mol/L}) = \frac{N}{N_{A6}} \times \frac{S_{A6}}{S} \times M$$

Here, N is the number of carbon atoms in one molecule of the organic compound having a known concentration, $N_{A6}$ is the number of carbon atoms in one molecule of the component A6, $S_{A6}$ is a peak area of the component A6, S is a peak area of the organic compound having a known concentration, and M is the concentration (μmol/L) of the organic compound having a known concentration.

When the volatility is low and the quantification cannot be performed by the GC measurement, the quantification can be performed using a chromatography method such as LC.

Concentrations of the component B and the component C can also be calculated by the same method as that for the component A6 described above.

In addition, the fact that the methacrylate-containing composition comprises water, and the concentration thereof can be confirmed by Karl Fischer method.

[Method for producing methacrylate-containing composition]

Examples of a method for producing the methacrylate-containing composition according to the sixth aspect include a method of adding the component A6 and the component B to methacrylic acid. As the methacrylic acid, a commercially available product may be used, or methacrylic acid produced by a known method such as an acetone cyanohydrin (ACH) method and a C4 direct oxidation method may be used. As the component A6 and the component B, a commercially available product may be used, or a component synthesized by a known method may be used. When methacrylic acid produced by a known method such as an acetone cyanohydrin (ACH) method and a C4 direct oxidation method is used, the methacrylate-containing composition may be produced by adding the component A6 or the component B in the middle of the process of a raw material step or a production step. In addition, when the component A6 or the component B is generated as a by-product in the methacrylic acid production process, a methacrylate-containing composition may be produced by leaving a part of the component A6 or the component B to be generated.

[Evaluation method for storage stability and thermal stability]

The methacrylate-containing composition according to the sixth aspect has high quality stability during storage. Examples of an evaluation method for the quality stability of the methacrylate-containing composition during storage include a method of actually storing the methacrylate-containing composition for a long period of time and confirming the amount of the polymerization inhibitor reduced. In addition, from the viewpoint of ease of work, a method of heating the methacrylate-containing composition for a short period of time and confirming the amount of the polymerization inhibitor reduced may be used. When heating for a short period of time, the heating temperature is preferably 50°C to 100°C and a heating time is preferably 1 to 24 hours. In the present invention, the quality stability of the methacrylate-containing composition during storage is evaluated by the amount of decrease in polymerization inhibitor when the methacrylate-containing composition is stored at 25°C for 21 days.

[Method for producing methacrylic ester]

A method for producing a methacrylic ester according to the sixth aspect includes a step of esterifying the methacrylate-containing composition according to the sixth aspect.

The alcohol to be reated with the methacrylate-containing composition is not particularly limited, and examples thereof include methanol, ethanol, n-propanol, isopropanol, n-butanol, and isobutanol. Examples of the methacrylic ester to be obtained include methyl methacrylate, ethyl methacrylate, propyl methacrylate, isopropyl methacrylate, butyl methacrylate, and isobutyl methacrylate. The esterification reaction can be carried out in the presence of an acidic catalyst such as a sulfonic acid-type cation exchange resin. The temperature during the esterification reaction is preferably 50°C to 200°C.

The pressure during the esterification reaction, the location of the catalyst in the reaction vessel, the proportion of the catalyst in the reaction vessel, and the like are not particularly limited, and a commonly used form can be adopted.

[Method for producing methacrylic acid polymer according to sixth aspect]

A method for producing a methacrylic acid polymer according to the sixth aspect includes a step of polymerizing a polymerizable composition comprising the methacrylate-containing composition according to the sixth aspect.

The polymerizable composition may further comprise a monomer copolymerizable with the methacrylic acid and other additive substances, as necessary.

Examples of the monomer copolymerizable with the methacrylic acid include the following:

methacrylic esters such as methyl methacrylate, ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;
acrylic esters such as methyl acrylate, ethyl acrylate, n-butyl acrylate, iso-butyl acrylate, tert-butyl acrylate, and 2-ethylhexyl acrylate;
unsaturated carboxylic acids such as acrylic acid, maleic acid, and itaconic acid;
unsaturated carboxylic anhydrides such as maleic anhydride and itaconic anhydride;
maleimides such as N-phenylmaleimide and N-cyclohexylmaleimide;
hydroxy group-containing vinyl monomers such as 2-hydroxyethyl acrylate, 2-hydroxyethyl methacrylate, and 2-hydroxypropyl methacrylate;
vinyl esters such as vinyl acetate and vinyl benzoate;
vinyl chloride, vinylidene chloride, and derivatives thereof;
nitrogen-containing vinyl monomers such as methacrylamide and acrylonitrile;
epoxy group-containing monomers such as glycidyl acrylate and glycidyl methacrylate;
aromatic vinyl monomers such as styrene and $\alpha$-methylstyrene;
alkanediol di(meth)acrylates such as ethylene glycol di(meth)acrylate, 1,2-propylene glycol di(meth)acrylate, 1,3-butylene glycol di(meth)acrylate, and 1,6-hexanediol di(meth)acrylate;
polyoxyalkylene glycol di(meth)acrylates such as diethylene glycol di(meth)acrylate, dipropylene glycol di(meth) acrylate, triethylene glycol (meth)acrylate, tetraethylene glycol di(meth)acrylate, polyethylene glycol di(meth)acrylate, and neopentyl glycol di(meth)acrylate;
vinyl monomers having two or more ethylenically unsaturated bonds in a molecule, such as divinylbenzene;
unsaturated polyester prepolymer obtained from at least one polyvalent carboxylic acid including an ethylenically unsaturated polycarboxylic acid and at least one diol; and
vinyl ester prepolymer obtained by acrylic-modifying a terminal of epoxy group.

Among the above, the monomer copolymerizable with the methacrylic acid is preferably at least one selected from the group consisting of methacrylic ester and acrylic ester. The monomer copolymerizable with the methacrylic acid is more preferably methacrylic ester and particularly preferably methyl methacrylate.

The monomer copolymerizable with the methacrylic acid may be one kind or two or more kinds. In addition, when the component A6 is a monomer copolymerizable with the methacrylic acid, the component A6 may be used as the monomer copolymerizable with the methacrylic acid, or another monomer copolymerizable with the methacrylic acid may be used separately from the component A6.

In the polymerizable composition, the upper limit of the contained amount of the monomer copolymerizable with the methacrylic acid is preferably 99.99 parts by mass or less with respect to 100 parts by mass of the polymerizable composition. The lower limit of the contained amount of the monomer copolymerizable with the methacrylic acid is preferably 50 parts by mass or more, more preferably 60 parts by mass or more, still more preferably 70 parts by mass or more, particularly preferably 80 parts by mass or more, and most preferably 90 parts by mass or more with respect to 100 parts by mass of the polymerizable composition. The upper limit of the contained amount of the monomer copolymerizable with the methacrylic acid is more preferably 99.9 parts by mass or less and still more preferably 99 parts by mass or less with respect to 100 parts by mass of the polymerizable composition.

As the other additives, a polymerization initiator is preferable. In addition, the polymerizable composition may further comprise, as necessary, a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, a fluorescent agent, or the like. The other additives may be one kind or two or more kinds.

Examples of the polymerization initiator include the following:

azo compounds such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate;

organic peroxides such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxyisononanoate, di-t-butyl peroxide, di-t-hexyl peroxide, lauroyl peroxide, and dilauroyl peroxide;
persulfate compounds such as potassium persulfate; and
a redox polymerization initiator.

Among the above, from the viewpoint of storage stability and reactivity with the monomer copolymerizable with the methacrylic acid, the polymerization initiator is preferably at least one selected from the group consisting of an azo compound and an organic peroxide.

The amount of the polymerization initiator used is preferably 0.0001 to 1 part by mass with respect to 100 parts by mass of the total amount of the methacrylic acid and the monomer copolymerizable with the methacrylic acid.

Examples of a polymerization method for the polymerizable composition include a bulk polymerization method, a solution polymerization method, an emulsion polymerization method, and a suspension polymerization method.

The polymerization temperature is preferably 125°C to 210°C. In this manner, an appropriate polymerization rate can be obtained. The lower limit of the polymerization temperature is more preferably 130°C or higher, and the upper limit thereof is more preferably 180°C or lower. The polymerization time is not particularly limited, and can be, for example, 0.5 to 24 hours.

Examples

Hereinafter, the embodiment will be described in more detail with reference to Examples and Comparative Examples, but the present invention is not limited to these examples. Unless otherwise specified, "%" and "ppm" in Examples and Comparative Examples mean "% by weight" and "ppm by weight".

The moisture concentration of the methacrylic acid of the reagent was calculated by Karl Fischer method. The formulation of constituent components of the methacrylate-containing composition before storage was calculated from the addition amount of each raw material. The amount of a polymerization inhibitor after storage was quantified by an absolute calibration curve method using GC-FID. Measurement conditions of GC-FID were as follows.
Device: GC-FID (product name: GC7890B, manufactured by Agilent)

[GC conditions]

Column (product name: Rtx-1701, manufactured by Restek Corporation)
length: 30 m, inner diameter: 0.25 mm, film thickness: 1.00 μm
Injection volume: 1.0 μL
Vaporization chamber temperature: 260°C
Column oven temperature: held at 40°C for 10 minutes, raised from 40°C to 240°C at 10 °C/min, and held at 240°C for 10 minutes
Carrier gas: helium
Injection mode: split (split ratio: 50)
Control mode: constant linear velocity (39.723 cm/sec)
Detector temperature: 260°C
Flow rate of detector H2: 30 mL/min
Air flow rate of detector: 400 mL/min
Make-up He flow rate: 25 mL/min

The moisture concentration was quantified by Karl Fischer method using an automatic moisture measurement device (product name: AQV-2200, manufactured by HIRUNUMA Co., Ltd.).

(Example a1)

A reagent of 2-isopropylhydroquinone was used as a component A1, and 0.0192 g of the component A1 was

added to 10.0081 g of a reagent of methacrylic acid (moisture concentration: 366 ppm; containing 4-methoxyphenol as a component B1 with the concentration of 306 ppm) to prepare a methacrylic acid solution (A-a1 solution). The concentration of the component A1 in the A-a1 solution is shown in Table 1.

Next, 0.0526 g of the A-a1 solution was added to 9.9585 g of a reagent of methacrylic acid of the reagent (moisture concentration: 366 ppm, concentration of 4-methoxyphenol as a component B1: 306 ppm) to prepare a methacrylate-containing composition. The concentration of each component in the methacrylate-containing composition is shown in Table 2.

The obtained methacrylate-containing composition was stored at 25°C for 21 days. Table 2 shows the reduction rate of the polymerization inhibitor (component B1) in the methacrylate-containing composition after the storage.

(Examples a2, a3, and a5)

An A-a solution was prepared in the same manner as in Example a1, except that a compound shown in Table 1 was used as the component A and the amounts of the reagent MAA and the component A were changed as shown in Table 1.

Next, an MAA-containing composition was prepared in the same manner as in Example a1, except that the amounts of the reagent MAA and the A-a solution were changed as shown in Table 2. Table 2 shows the reduction rate of the polymerization inhibitor (component B1) in the methacrylate-containing composition after the storage.

(Example a4)

An A-a4 solution was prepared by the same method as in Example a1, except that a reagent of 2-phenylisobutyric acid was used as the component A.

Next, hydroquinone was used as a component B2, and 0.4323g of the component B2 was added to 40.0050 g of a reagent of methacrylic acid (moisture concentration: 366 ppm, concentration of 4-methoxyphenol as a component B1: 306 ppm) to prepare a methacrylic acid solution (B-a4). The concentration of the component B2 in the B-a4 solution is shown in Table 1.

Next, 0.0600 g of the A-a4 solution and 0.2507 g of the B-a4 solution was added to 9.7103 g of a reagent of methacrylic acid of the reagent (moisture concentration: 366 ppm, concentration of 4-methoxyphenol as a component B1: 306 ppm) to prepare a methacrylate-containing composition. The concentration of each component in the methacrylate-containing composition is shown in Table 2. Table 2 shows the reduction rate of the polymerization inhibitor (component B1) in the methacrylate-containing composition after the storage.

(Example a6)

A reagent of 2-phenylisobutyric acid was used as a component A1, and 0.0089 g of the component A1 was added to 9.9955 g of a reagent of methacrylic acid (moisture concentration: 366 ppm, concentration of 4-methoxyphenol as a component B1: 306 ppm) to prepare a methacrylate-containing composition. The concentration of each component in the methacrylate-containing composition is shown in Table 2.

(Examples a7 and a8)

An A-a solution was prepared in the same manner as in Example a1, except that a compound shown in Table 1 was used as the component A and the amounts of the reagent MAA and the component A were changed as shown in Table 1.

A B-b solution was prepared in the same manner as in Example a4, except that a compound shown in Table 1 was used as the component B and the amounts of the reagent MAA and the component B were changed as shown in Table 1.

Next, an MAA-containing composition was prepared in the same manner as in Example a4, except that the amounts of the reagent MAA, the A-a solution, and the B-b solution were changed as shown in Table 2. Table 2 shows the reduction rate of the polymerization inhibitor (component B1) in the methacrylate-containing composition after the storage.

(Comparative Example a1)

A reagent of 4-methoxyphenol was used as a component B1, and 0.4016 g of 4-methoxyphenol was added to 40.0219 g of a reagent of methacrylic acid (moisture concentration: 366 ppm, concentration of 4-methoxyphenol as the component B1: 306 ppm) to prepare a B-a1 solution.

Next, 0.5000 g of the B-a1 solution was added to 19.5000 g of a reagent of methacrylic acid (moisture concentration: 366 ppm) to prepare a methacrylate-containing composition. The concentration of each component in

the methacrylate-containing composition is shown in Table 2.

The obtained methacrylate-containing composition was stored in the same manner as in Example a1. Table 2 shows the reduction rate of the polymerization inhibitor (component B1) in the methacrylate-containing composition after the storage.

(Comparative Example a2)

A reagent of methacrylic acid (moisture concentration: 366 ppm, concentration of 4-methoxyphenol as a component B1: 306 ppm) was stored at 25°C for 21 days. Table 2 shows the reduction rate of the polymerization inhibitor (component B1) in the methacrylate-containing composition after the storage.

(Comparative Example a3)

An A-a solution was prepared in the same manner as in Example a1, except that a compound shown in Table 1 was used as the component A and the amounts of the reagent MAA and the component A were changed as shown in Table 1.

0.0971 g of the A-a solution and 0.0565 g of pure water were added to 19.4088 g of a reagent of methacrylic acid of the reagent (moisture concentration: 366 ppm, concentration of 4-methoxyphenol: 306 ppm) to prepare a methacrylate-containing composition. The concentration of each component in the methacrylate-containing composition is shown in Table 2. Table 2 shows the reduction rate of the polymerization inhibitor (component B1) in the methacrylate-containing composition after the storage.

[Table 1]

| | a solution | | | | b solution | | | |
|---|---|---|---|---|---|---|---|---|
| | Addition amount of reagent of MAA [g] | Component A | | | Addition amount of reagent of MAA [g] | Component B2 | | |
| | | Compound name | Addition amount [g] | Concentration [ppm] | | Compound name | Addition amount [g] | Concentration [ppm] |
| Example a1 | 10.0081 | 2-Isopropylhydroquinone | 0.0192 | 1915 | - | - | - | - |
| Example a2 | 10.0063 | 2-Isopropyl-4-methoxyphenol | 0.0228 | 2273 | - | - | - | - |
| Example a3 | 9.9984 | Methyl 2-phenylisobutyrate | 0.0246 | 2454 | - | - | - | - |
| Example a4 | 10.0070 | 2-Phenylisobutyric acid | 0.0235 | 2343 | 40.0050 | Hydroquinone | 0.4323 | 10691 |
| Example a5 | 10.0687 | 2-Phenylisobutyric acid | 0.0212 | 2101 | - | - | - | - |
| Example a6 | - | 2-Phenylisobutyric acid | - | - | - | - | - | - |
| Example a7 | 10.0070 | 2-Phenylisobutyric acid | 0.0235 | 2343 | 40.0122 | 4-Methoxyphenol | 0.4063 | 10052 |
| Example a8 | 10.0070 | 2-Phenylisobutyric acid | 0.0235 | 2343 | 40.0122 | 4-Methoxyphenol | 0.4063 | 10052 |
| Comparative Example a1 | - | - | - | - | 40.0219 | 4-Methoxyphenol | 0.4016 | 9935 |
| Comparative Example a2 | - | - | - | - | - | - | - | - |
| Comparative Example a3 | 10.0070 | 2-Phenylisobutyric acid | 0.0235 | 2343 | - | - | - | - |

[Table 2]

| | Addition amount [g] | | | | | | MMA-containing composition | | | | | | | Result of storage stability |
|---|---|---|---|---|---|---|---|---|---|---|---|---|---|---|
| | | | | | | | MAA | Component A | | Component B1 | | Component B2 | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Example a1 | 9.9585 | 0.0526 | - | - | - | - | 99.93 | 2-Isopropylhydroquinone | 67 | 4-Methoxyphenol | 2392 | - | - | -1.8 |
| Example a2 | 9.9607 | 0.0526 | - | - | - | - | 99.93 | 2-Isopropyl-4-methoxyphenol | 73 | 4-Methoxyphenol | 2392 | - | - | -3.38 |
| Example a3 | 9.9635 | 0.0509 | - | - | - | - | 99.93 | Methyl 2-phenylisobutyrate | 71 | 4-Methoxyphenol | 2392 | - | - | -3.64 |
| Example a4 | 9.7103 | 0.0600 | 0.2507 | - | - | - | 99.91 | 2-Phenylisobutyric acid | 87 | 4-Methoxyphenol | 2391 | Hydroquinone | 2478 | -0.85 |
| Example a5 | 9.5127 | 0.4583 | - | - | - | - | 99.92 | 2-Phenylisobutyric acid | 600 | 4-Methoxyphenol | 2391 | - | - | -0.13 |
| Example a6 | 9.9955 | - | - | 0.0089 | - | - | 99.85 | 2-Phenylisobutyric acid | 5526 | 4-Methoxyphenol | 2389 | - | - | -0.05 |
| Example a7 | 9.9713 | 0.0549 | 0.2602 | - | - | - | 99.91 | 2-Phenylisobutyric acid | 78 | 4-Methoxyphenol | 4480 | 4-Methoxyphenol | - | 2.58 |
| Example a8 | 74434 | 0.0510 | 2.5114 | - | - | - | 99.68 | 2-Phenylisobutyric acid | 74 | 4-Methoxyphenol | 23116 | 4-Methoxyphenol | - | -3.04 |
| Comparative Example a1 | 19.5000 | - | 0.5000 | - | - | - | 99.91 | - | - | 4-Methoxyphenol | 4556 | 4-Methoxyphenol | - | -15.17 |
| Comparative Example a2 | 20.0000 | - | - | - | - | - | 99.93 | - | - | 4-Methoxyphenol | 2516 | - | - | -13.74 |
| Comparative Example a3 | 19.4088 | 0.0971 | - | - | - | 0.0565 | 99.64 | 2-Phenylisobutyric acid | 72 | 4-Methoxyphenol | 2509 | - | - | -12.32 |

As shown in Tables 1 and 2, in Examples a1 to a8, the reduction rate of the polymerization inhibitor (component B1) in the methacrylate-containing composition after the storage was suppressed. It can be said that the quality stability during the storage was high.

(Examples b1 to b9)

A C-a solution was prepared in the same manner as in Example a1, except that a compound shown in Table 3 was used as a component A21 and the amounts of the reagent MAA and the component A21 were changed as shown in Table 3.

Next, an MAA-containing composition was prepared in the same manner as in Example a1, except that the amounts of the reagent MAA and the C-a solution were changed as shown in Table 4. The concentration of each component in the methacrylate-containing composition is shown in Table 4. Table 4 shows the reduction rate of the polymerization inhibitor (component B1) in the methacrylate-containing composition after being stored at 25°C for 21 days.

(Comparative Example b1)

A reagent of 2,3,5,6-tetramethylpyrazine was used as a component A21, and 0.4611 g of the component A21 was added to 19.5520 g of a reagent of methacrylic acid (moisture concentration: 366 ppm, concentration of 4-methoxyphenol as a component B1: 306 ppm) to prepare a methacrylate-containing composition. The concentration of each component in the methacrylate-containing composition is shown in Table 4. Table 4 shows the reduction rate of the polymerization inhibitor (component B1) in the methacrylate-containing composition after being stored at 25°C for 21 days.

[Table 3]

| | a solution | | | | b solution | | | |
|---|---|---|---|---|---|---|---|---|
| | Addition amount of reagent of MAA [g] | Component A | | | Addition amount of reagent of MAA [g] | Component B2 | | |
| | | Compound name | Addition amount [g] | Concentration [ppm] | | Compound name | Addition amount [g] | Concentration [ppm] |
| Example b1 | 10.0152 | 2,3,5,6-Tetramethylpyrazine | 0.0204 | 2033 | - | - | - | - |
| Example b2 | 10.0033 | Pyrazine | 0.0268 | 2672 | - | - | - | - |
| Example b3 | 10.0222 | 2,3,5-Trimethylpyrazine | 0.0630 | 6247 | - | - | - | - |
| Example b4 | 10.0115 | 2-Methoxypyrazine | 0.0248 | 2471 | - | - | - | - |
| Example b5 | 10.0273 | 2-Isopropyl-3-methoxypyrazine | 0.0195 | 1941 | - | - | - | - |
| Example b6 | 9.9972 | 2,5-Dimethylpyrazine | 0.0204 | 2036 | - | - | - | - |
| Example b7 | 10.0247 | Methyl 2-pyrazinecarboxylate | 0.0200 | 1991 | - | - | - | - |
| Example b8 | 10.0192 | 2-(Methylthio)pyrazine | 0.0210 | 2092 | - | - | - | - |
| Example b9 | 10.0138 | 2-Pyrazinemethanol | 0.0286 | 2848 | - | - | - | - |
| Comparative Example b1 | - | 2,3,5,6-Tetramethylpyrazine | - | - | - | - | - | - |

[Table 4]

| | Addition amount [g] | | | | | | MMA-containing composition | | | | | | | Result of storage stability |
|---|---|---|---|---|---|---|---|---|---|---|---|---|---|---|
| | | | | | | | MAA | Component A | | Component B1 | | Component B2 | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Example b1 | 9.9711 | 0.0500 | - | - | - | - | 99.93 | 2,3,5,6-Tetramethylpyrazine | 76 | 4-Methoxyphenol | 2392 | - | - | -2.12 |
| Example b2 | 9.9580 | 0.0535 | - | - | - | - | 99.93 | Pyrazine | 182 | 4-Methoxyphenol | 2392 | - | - | -5.01 |
| Example b3 | 9.9597 | 0.0530 | - | - | - | - | 99.93 | 2,3,5-Trimethylpyrazine | 276 | 4-Methoxyphenol | 2391 | - | - | -5.50 |
| Example b4 | 9.9606 | 0.0538 | - | - | - | - | 99.93 | 2-Methoxypyrazine | 123 | 4-Methoxyphenol | 2392 | - | - | -4.00 |
| Example b5 | 9.9609 | 0.0507 | - | - | - | - | 99.93 | 2-Isopropyl-3-methoxypyrazine | 66 | 4-Methoxyphenol | 2392 | - | - | -4.14 |
| Example b6 | 9.9486 | 0.0616 | - | - | - | - | 99.93 | 2,5-Dimethylpyrazine | 118 | 4-Methoxyphenol | 2392 | - | - | -5.11 |
| Example b7 | 9.9572 | 0.0625 | - | - | - | - | 99.93 | Methyl 2-pyrazinecarboxylate | 92 | 4-Methoxyphenol | 2392 | - | - | -5.23 |
| Example b8 | 9.9539 | 0.0655 | - | - | - | - | 99.93 | 2-(Methylthio)pyrazine | 111 | 4-Methoxyphenol | 2392 | - | - | -4.70 |
| Example b9 | 9.9534 | 0.0614 | - | - | - | - | 99.93 | 2-Pyrazinemethanol | 162 | Methoxyphenol | 2392 | - | - | -4.95 |
| Comparative Example b1 | 19.5520 | - | - | 0.4611 | - | - | 97.63 | 2,3,5,6-Tetramethylpyrazine | 172546 | 4-Methoxyphenol | 2458 | - | - | -13.88 |

As shown in Tables 3 and 4, in Examples b1 to b9, the decomposition of the polymerization inhibitor (component B1) in the methacrylate-containing composition after the storage was suppressed. It can be said that the quality stability during the storage was high.

(Examples c1, c2, c3, and c5)

A C-a solution was prepared in the same manner as in Example a1, except that a compound shown in Table 5 was used as a component A3 and the amounts of the reagent MAA and the component A3 were changed as shown in Table 5.

Next, an MAA-containing composition was prepared in the same manner as in Example a1, except that the amounts of the reagent MAA and the C-a solution were changed as shown in Table 6. The concentration of each component in the methacrylate-containing composition is shown in Table 6. Table 6 shows the reduction rate of the polymerization inhibitor (component B1) in the methacrylate-containing composition after being stored at 25°C for 21 days.

(Example c4)

An MAA-containing composition was prepared by the same method as in Example a4, except that a reagent of t-butylphenyl ether was used as the component A3. The concentration of each component in the methacrylate-containing composition is shown in Table 6. Table 6 shows the reduction rate of the polymerization inhibitor (component B1) in the methacrylate-containing composition after being stored at 25°C for 21 days.

(Example c6)

An MAA-containing composition was prepared by the same method as in Example a6, except that a reagent of t-butylphenyl ether was used as the component A3. The concentration of each component in the methacrylate-containing composition is shown in Table 6. Table 6 shows the reduction rate of the polymerization inhibitor (component B1) in the methacrylate-containing composition after being stored at 25°C for 21 days.

(Examples c7 and c8)

A C-a solution was prepared in the same manner as in Example a1, except that t-butylphenyl ether was used as the component A3. Next, a C-b solution was prepared by the same method as in Example a7.

A methacrylate-containing composition was prepared with the amounts of the reagent MAA, the C-a solution, and the C-b solution added as shown in Table 6. The concentration of each component in the methacrylate-containing composition is shown in Table 6. Table 6 shows the reduction rate of the polymerization inhibitor (component B1) in the methacrylate-containing composition after being stored at 25°C for 21 days.

(Comparative Example c1)

A methacrylate-containing composition was prepared in the same manner as in Comparative Example b1, except that t-butylphenyl ether was used as the component A. The concentration of each component in the methacrylate-containing composition is shown in Table 6. Table 6 shows the reduction rate of the polymerization inhibitor (component B1) in the methacrylate-containing composition after being stored at 25°C for 21 days.

(Comparative Example c2)

A methacrylate-containing composition was prepared in the same manner as in Comparative Example a3, except that t-butylphenyl ether was used as the component A3. The concentration of each component in the methacrylate-containing composition is shown in Table 6. Table 6 shows the reduction rate of the polymerization inhibitor (component B1) in the methacrylate-containing composition after being stored at 25°C for 21 days.

[Table 5]

| | a solution | | | | b solution | | | |
|---|---|---|---|---|---|---|---|---|
| | Addition amount of reagent of MAA [g] | Component A | | | Addition amount of reagent of MAA [g] | Component B2 | | |
| | | Compound name | Addition amount [g] | Concentration [ppm] | | Compound name | Addition amount [g] | Concentration [ppm] |
| Example c1 | 10.0042 | t-Butylphenyl ether | 0.0167 | 1667 | - | - | - | - |
| Example c2 | 10.0026 | Isopropylphenyl ether | 0.0218 | 2175 | - | - | - | - |
| Example c3 | 10.0172 | 4-Isopropoxyphenol | 0.0252 | 2509 | - | - | - | - |
| Example c4 | 10.0042 | t-Butylphenyl ether | 0.0167 | 1667 | 40.0029 | Hydroquinone | 0.3957 | 9795 |
| Example c5 | 10.0042 | t-Butylphenyl ether | 0.0167 | 1667 | - | - | - | - |
| Example c6 | - | t-Butylphenyl ether | - | - | - | - | - | - |
| Example c7 | 10.0042 | t-Butylphenyl ether | 0.0167 | 1667 | 39.9976 | 4-Methoxyphenol | 0.3996 | 9892 |
| Example c8 | 10.0042 | t-Butylphenyl ether | 0.0169 | 1686 | 39.9976 | 4-Methoxyphenol | 0.3996 | 9892 |
| Comparative Example c1 | - | t-Butylphenyl ether | - | - | - | - | - | - |
| Comparative Example c2 | 10.0042 | t-Butylphenyl ether | 0.0167 | 1667 | - | - | - | - |

[Table 6]

| | Addition amount [g] | | | | | | MMA-containing composition | | | | | | | Result of storage stability |
|---|---|---|---|---|---|---|---|---|---|---|---|---|---|---|
| | | | | | | | MAA | Component A | | Component B1 | | Component B2 | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Example c1 | 9.9615 | 0.0654 | - | - | - | - | 99.93 | t-Butylphenyl ether | 74 | Methoxyphenol | 2516 | - | - | -10.46 |
| Example c2 | 9.9616 | 0.0499 | - | - | - | - | 9993 | Isopropylphenyl ether | 81 | 4-Methoxyphenol | 2516 | - | - | -9.91 |
| Example c3 | 9.9622 | 0.0546 | - | - | - | - | 99.93 | 4-[sopropoxyphenol | 92 | 4-Methoxyphenol | 2392 | - | - | -3.92 |
| Example c4 | 9.7000 | 0.0500 | 0.2500 | - | - | - | 99.91 | t-Butylphenyl ether | 57 | Methoxyphenol | 2391 | Hydroquinone | 2268 | -2.57 |
| Example c5 | 9.5150 | 0.0580 | - | - | - | - | 99.93 | t-Butylphenyl ether | 69 | Methoxyphenol | 2392 | - | - | -6.01 |
| Example c6 | 10.066-C | - | - | 0.0090 | - | - | 99.84 | t-Butylphenyl ether | 6066 | Methoxyphenol | 2514 | - | - | -8.94 |
| Example c7 | 9.6970 | 0.0557 | 0.2534 | - | - | - | 99.91 | t-Butylphenyl ether | 63 | 4-Methoxyphenol | 4449 | 4-Methoxyphenol | - | -0.02 |
| Example c8 | 7.4555 | 0.0612 | 2.4995 | - | - | - | 99.69 | t-Butylphenyl ether | 70 | Methoxyphenol | 22792 | 4-Methoxyphenol | - | 7.87 |
| Comparative Example c1 | 19.5535 | - | - | 0.4589 | - | - | 97.64 | t-Butylphenyl ether | 155701 | 4-Methoxyphenol | 2458 | - | - | -13.26 |
| Comparative Example c2 | 19.3984 | 0.0992 | - | - | - | 0.5077 | 97.40 | t-Butylphenyl ether | 56 | 4-Methoxyphenol | 2452 | - | - | -13.04 |

As shown in Tables 5 and 6, in Examples c1 to c8, the decomposition of the polymerization inhibitor (component B1) in the methacrylate-containing composition after the storage was suppressed. It can be said that the quality stability during the storage was high.

(Examples d1 to d6)

A D-a solution was prepared in the same manner as in Example a1, except that a compound shown in Table 7 was used as a component A4 and the amounts of the reagent MAA and the component A4 were changed as shown in Table 7.

Next, an MAA-containing composition was prepared in the same manner as in Example a1, except that the amounts of the reagent MAA and the D-a solution were changed as shown in Table 8. The concentration of each component in the methacrylate-containing composition is shown in Table 8. Table 8 shows the reduction rate of the polymerization inhibitor (component B1) in the methacrylate-containing composition after being stored at 25°C for 21 days.

(Example d7)

A methacrylate-containing composition was prepared in the same manner as in Example a4, except that methacrylonitrile was used as the component A4. The concentration of each component in the methacrylate-containing composition is shown in Table 8. Table 8 shows the reduction rate of the polymerization inhibitor (component B1) in the methacrylate-containing composition after being stored at 25°C for 21 days.

(Example d8)

A D-a solution was prepared in the same manner as in Example a1, except that a compound shown in Table 7 was used as the component A4 and the amounts of the reagent MAA and the component A4 were changed as shown in Table 7.

Next, an MAA-containing composition was prepared in the same manner as in Example a1, except that the amounts of the reagent MAA and the D-a solution were changed as shown in the table. The concentration of each component in the methacrylate-containing composition is shown in Table 8. Table 8 shows the reduction rate of the polymerization inhibitor (component B1) in the methacrylate-containing composition after being stored at 25°C for 21 days.

(Example d9)

A methacrylate-containing composition was prepared in the same manner as in Example a6, except that methacrylonitrile was used as the component A4. The concentration of each component in the methacrylate-containing composition is shown in Table 8. Table 8 shows the reduction rate of the polymerization inhibitor (component B1) in the methacrylate-containing composition after being stored at 25°C for 21 days.

(Examples d10 and d11)

A D-a solution was prepared in the same manner as in Example a1, except that methacrylonitrile was used as the component A4. Next, a D-b solution was prepared by the same method as in Example a7.

A methacrylate-containing composition was prepared with the amounts of the reagent MAA, the D-a solution, and the D-b solution added as shown in Table 8. The concentration of each component in the methacrylate-containing composition is shown in Table 8. Table 8 shows the reduction rate of the polymerization inhibitor (component B1) in the methacrylate-containing composition after being stored at 25°C for 21 days.

(Comparative Example d1)

A methacrylate-containing composition was prepared in the same manner as in Comparative Example b1, except that methacrylonitrile was used as the component B. The concentration of each component in the methacrylate-containing composition is shown in Table 8.

The obtained methacrylate-containing composition was stored in the same manner as in Example d1. Table 8 shows the reduction rate of the polymerization inhibitor (component B1) in the methacrylate-containing composition after the storage.

(Comparative Example d2)

A methacrylate-containing composition was prepared in the same manner as in Comparative Example a1, except that methacrylonitrile was used as the component B. The concentration of each component in the methacrylate-containing composition is shown in Table 8. Table 8 shows the reduction rate of the polymerization inhibitor (component B1) in the methacrylate-containing composition after being stored at 25°C for 21 days.

[Table 7]

| | a solution | | | | b solution | | | |
|---|---|---|---|---|---|---|---|---|
| | Addition amount of reagent of MAA [g] | Component A | | | Addition amount of reagent of MAA [g] | Component B2 | | |
| | | Compound name | Addition amount [g] | Concentration [ppm] | | Compound name | Addition amount [g] | Concentration [ppm] |
| Example d1 | 10.0197 | Methacrylonitrile | 0.0156 | 1555 | - | - | - | - |
| Example d2 | 10.0001 | Acetonitrile | 0.0191 | 1906 | - | - | - | - |
| Example d3 | 10.0107 | Propionitrile | 0.0146 | 1456 | - | - | - | - |
| Example d4 | 9.9909 | Acrylonitrile | 0.0186 | 1858 | - | - | - | - |
| Example d5 | 9.9931 | 4'-Cyanoacetophenone | 0.0243 | 2426 | - | - | - | - |
| Example d6 | 10.0185 | 4-(Methylthio)benzonitrile | 0.0269 | 2678 | - | - | - | - |
| Example d7 | 10.0197 | Methacrylonitrile | 0.0156 | 1555 | 40.0119 | Hydroquinone | 0.4015 | 9935 |
| Example d8 | 10.0197 | Methacrylonitrile | 0.0156 | 1555 | - | - | - | - |
| Example d9 | - | Methacrylonitrile | - | - | - | - | - | - |
| Example d10 | 10.0197 | Methacrylonitrile | 0.0156 | 1555 | 40.0040 | 4-Methoxyphenol | 0.3993 | 9883 |
| Example d11 | 10.0197 | Methacrylonitrile | 0.0156 | 1555 | 40.0040 | 4-Methoxyphenol | 0.3993 | 9883 |
| Comparative Example d1 | - | Methacrylonitrile | - | - | - | - | - | - |
| Comparative Example d2 | 10.0197 | Methacrylonitrile | 0.0156 | 1555 | - | - | - | - |

[Table 8]

| | Addition amount [g] | | | | | | MMA-containing composition | | | | | | | | Result of storage stability |
|---|---|---|---|---|---|---|---|---|---|---|---|---|---|---|---|
| | | | | | | | MAA | Component A | | Component B1 | | Component B2 | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Example d1 | 9.9649 | 0.0556 | - | - | - | - | 99.93 | Methacrylonitrile | 131 | 4-Methoxyphenol | 2392 | - | - | -4.51 |
| Example d2 | 9.9587 | 0.0539 | - | - | - | - | 9993 | Acetonitrile | 255 | 4-Methoxyphenol | 2392 | - | - | -5.28 |
| Example d3 | 9.9655 | 0.0549 | - | - | - | - | 9993 | Propionitrile | 148 | 4-Methoxyphenol | 2392 | - | - | -6.85 |
| Example d4 | 9.9607 | 0.0504 | - | - | - | - | 99.93 | Acrylonitrile | 180 | 4-Methoxyphenol | 2392 | - | - | -6.26 |
| Example d5 | 9.9527 | 0.0491 | - | - | - | - | 99.93 | 4'-Cyanoacetophenone | 84 | 4-Methoxyphenol | 2392 | - | - | -6.84 |
| Example d6 | 9.9652 | 0.0468 | - | - | - | - | 99.93 | 4-(Methylthio)benzonitrile | 86 | 4-Methoxyphenol | 2392 | - | - | -7.18 |
| Example d7 | 9.7186 | 0.0514 | 0.2526 | - | - | - | 99.91 | Methacrylonitrile | 121 | 4-Methoxyphenol | 2391 | Hydroquinone | 2319 | -4.49 |
| Example d8 | 9.5155 | 0.5000 | - | - | - | - | 99.93 | Methacrylonitrile | 1180 | 4-Methoxyphenol | 2391 | - | - | -5.40 |
| Example d9 | 10.0112 | - | - | 0.0077 | - | - | 99.86 | Methacrylonitrile | 11685 | 4-Methoxyphenol | 2390 | - | - | -5.08 |
| Example d10 | 9.7013 | 0.0563 | 0.2621 | - | - | - | 99.91 | Methacrylonitrile | 133 | 4-Methoxyphenol | 4515 | 4-Methoxyphenol | - | -0.51 |
| Example d11 | 7.4636 | 0.0518 | 2.5015 | - | - | - | 99.69 | Methacrylonitrile | 122 | 4-Methoxyphenol | 22664 | 4-Methoxyphenol | - | -0.19 |
| Comparative Example d1 | 19.5500 | - | - | 0.4500 | - | - | 97.68 | Methacrylonitrile | 342078 | 4-Methoxyphenol | 2459 | - | - | -14.35 |

(continued)

| | Addition amount [g] | | | | | | MMA-containing composition | | | | | | | | Result of storage stability |
|---|---|---|---|---|---|---|---|---|---|---|---|---|---|---|---|
| | | | | | | | MAA | Component A | | Component B1 | | Component B2 | | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | | |
| Comparative Example d2 | 19.3951 | 0.1102 | - | - | - | 0.4997 | 97.44 | Methacrylonitrile | 130 | 4-Methoxyphenol | 2453 | - | - | | -13.08 |

As shown in Tables 7 and 8, in Examples d1 to d11, the decomposition of the polymerization inhibitor (component B1) in the methacrylate-containing composition after the storage was suppressed. It can be said that the quality stability during the storage was high.

(Examples e1 to e9)

An E-a solution was prepared in the same manner as in Example a1, except that a compound shown in Table 9 was used as a component A5 and the amounts of the reagent MAA and the component A5 were changed as shown in Table 9.

Next, an MAA-containing composition was prepared in the same manner as in Example a1, except that the amounts of the reagent MAA and the E-a solution were changed as shown in the table. The concentration of each component in the methacrylate-containing composition is shown in Table 10. Table 10 shows the reduction rate of the polymerization inhibitor (component B1) in the methacrylate-containing composition after being stored at 25°C for 21 days.

(Example e10)

A methacrylate-containing composition was prepared in the same manner as in Example a4, except that isobutyric acid was used as the component A5. The concentration of each component in the methacrylate-containing composition is shown in Table 10. Table 10 shows the reduction rate of the polymerization inhibitor (component B1) in the methacrylate-containing composition after being stored at 25°C for 21 days.

(Example e11)

A methacrylate-containing composition was prepared in the same manner as in Example a6, except that isobutyric acid was used as the component A5. The concentration of each component in the methacrylate-containing composition is shown in Table 10. Table 10 shows the reduction rate of the polymerization inhibitor (component B1) in the methacrylate-containing composition after being stored at 25°C for 21 days.

(Example e12)

A methacrylate-containing composition was prepared in the same manner as in Example a7, except that isobutyric acid was used as the component A5. The concentration of each component in the methacrylate-containing composition is shown in Table 10. Table 10 shows the reduction rate of the polymerization inhibitor (component B1) in the methacrylate-containing composition after being stored at 25°C for 21 days.

(Comparative Example e1)

A methacrylate-containing composition was prepared by the same method as in Comparative Example b1, except that isobutyric acid was used as the component A5. The concentration of each component in the methacrylate-containing composition is shown in Table 10. Table 10 shows the reduction rate of the polymerization inhibitor (component B1) in the methacrylate-containing composition after being stored at 25°C for 21 days.

[Table 9]

| | a solution | | | | b solution | | | |
|---|---|---|---|---|---|---|---|---|
| | Addition amount of reagent of MAA [g] | Component A | | | Addition amount of reagent of MAA [g] | Component B2 | | |
| | | Compound name | Addition amount [g] | Concentration [ppm] | | Compound name | Addition amount [g] | Concentration [ppm] |
| Example e1 | 10.0037 | Isobutyric acid | 0.0147 | 1467 | - | - | - | - |
| Example e2 | 10.0011 | Isovaleric acid | 0.0166 | 1657 | - | - | - | - |
| Example e3 | 10.0010 | 2-Methylbutyric acid | 0.0182 | 1817 | - | - | - | - |
| Example e4 | 10.0006 | Acetonylacetone | 0.0208 | 2076 | - | - | - | - |
| Example e5 | 9.9988 | Isopropenyl methyl ketone | 0.0187 | 1867 | - | - | - | - |
| Example e6 | 9.9985 | Isobutylaldehyde | 0.0148 | 1478 | - | - | - | - |
| Example e7 | 10.0037 | Cyclohexanecarboxylic acid | 0.0150 | 1497 | - | - | - | - |
| Example e8 | 10.0026 | 2-Methoxypropionic acid | 0.0200 | 1995 | - | - | - | - |
| Example e9 | 9.9980 | N,N-Dimethylglycine | 0.0200 | 1996 | - | - | - | - |
| Example e10 | 9.9931 | Isobutyric acid | 0.0265 | 2645 | 40.0473 | Hydroquinone | 0.3965 | 9804 |
| Example e11 | - | Isobutyric acid | - | - | - | - | - | - |
| Example e12 | 9.9931 | Isobutyric acid | 0.0265 | 2645 | 40.0219 | 4-Methoxyphenol | 0.4016 | 9935 |
| Comparative Example e1 | - | Isobutyric acid | - | - | - | - | - | - |

[Table 10]

| | Addition amount [g] | | | | | | MMA-containing composition | | | | | | | Result of storage stability |
|---|---|---|---|---|---|---|---|---|---|---|---|---|---|---|
| | | | | | | | MAA | Component A | | Component B1 | | Component B2 | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Example e1 | 9.9627 | 0.0518 | - | - | - | - | 99.93 | Isobutyric acid | 88 | 4-Methoxyphenol | 2516 | - | - | -6.13 |
| Example e2 | 9.9597 | 0.0607 | - | - | - | - | 99.93 | Isovaleric acid | 100 | 4-Methoxyphenol | 2516 | - | - | -5.40 |
| Example e3 | 9.9597 | 0.0624 | - | - | - | - | 99.93 | 2-Methylbutyric acid | 113 | 4-Methoxyphenol | 2516 | - | - | -6.58 |
| Example e4 | 9.9468 | 0.0501 | - | - | - | - | 9993 | Acetonylacetone | 93 | 4-Methoxyphenol | 2516 | - | - | -6.96 |
| Example e5 | 9.9410 | 0.0478 | - | - | - | - | 99.93 | Isopropenyl methyl ketone | 108 | 4-Methoxyphenol | 2516 | - | - | -4.58 |
| Example e6 | 9.9557 | 0.0502 | - | - | - | - | 99.93 | Isobutylaldehyde | 105 | 4-Methoxyphenol | 2516 | - | - | -6.11 |
| Example e7 | 9.9445 | 0.0496 | - | - | - | - | 99.93 | Cyclohexanecarboxylic acid | 59 | 4-Methoxyphenol | 2516 | - | - | -5.25 |
| Example e8 | 9.9626 | 0.0507 | - | - | - | - | 99.93 | 2-Methoxypropionic acid | 99 | 4-Methoxyphenol | 2516 | - | - | -5.99 |
| Example e9 | 9.9521 | 0.0573 | - | - | - | - | 99.93 | N,N-Dimethylglycine | 113 | 4-Methoxyphenol | 2516 | - | - | -5.76 |
| Example e10 | 9.9918 | 0.0487 | 0.2500 | - | - | - | 99.91 | Isobutyric acid | 145 | 4-Methoxyphenol | 2515 | Hydroquinone | 2206 | -3.96 |
| Example e11 | 10.0111 | - | - | 0.0152 | - | - | 99.78 | Isobutyric acid | 17550 | 4-Methoxyphenol | 2512 | - | - | -6.95 |
| Example e12 | 9.7046 | 0.0501 | 0.2491 | - | - | - | 99.91 | Isobutyric acid | 153 | 4-Methoxyphenol | 4548 | 4-Methoxyphenol | - | 0.41 |

(continued)

| | Addition amount [g] | | | | | | MMA-containing composition | | | | | | | | Result of storage stability |
|---|---|---|---|---|---|---|---|---|---|---|---|---|---|---|---|
| | | | | | | | MAA | Component A | | Component B1 | | Component B2 | | Concentration change rate of component B1 [%] |
| | Reagent of MAA | a solution | b solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Comparative Example e1 | 19.5500 | - | - | 0.4500 | - | - | 97.68 | Isobutyric acid | 260470 | 4-Methoxyphenol | 2459 | - | - | -13.51 |

As shown in Tables 9 and 10, in Examples e1 to e12, the decomposition of the polymerization inhibitor (component B1) in the methacrylate-containing composition after the storage was suppressed. It can be said that the quality stability during the storage was high.

(Examples f1 to f10)

An F-a solution was prepared in the same manner as in Example a1, except that a compound shown in Table 11 was used as a component A6 and the amounts of the reagent MAA and the component A6 were changed as shown in Table 11.

Next, an MAA-containing composition was prepared in the same manner as in Example a1, except that the amounts of the reagent MAA and the F-a solution were changed as shown in the table. The concentration of each component in the methacrylate-containing composition is shown in Table 12. Table 12 shows the reduction rate of the polymerization inhibitor (component B1) in the methacrylate-containing composition after being stored at 25°C for 21 days.

[Table 11]

| | a solution | | | | b solution | | | |
|---|---|---|---|---|---|---|---|---|
| | Addition amount of reagent of MAA [g] | Component A | | | Addition amount of reagent of MAA [g] | Component B2 | | |
| | | Compound name | Addition amount [g] | Concentration [ppm] | | Compound name | Addition amount [g] | Concentration [ppm] |
| Example f1 | 10.0048 | Crotonic acid | 0.0249 | 2483 | - | - | - | - |
| Example f2 | 10.0053 | 2-Methylcrotonic acid | 0.0251 | 2502 | - | - | - | - |
| Example f3 | 10.0061 | 3,3-Dimethylacrylic acid | 0.0225 | 2244 | - | - | - | - |
| Example f4 | 10.0025 | 2-Pentenoic acid | 0.0225 | 2244 | - | - | - | - |
| Example f5 | 9.9957 | 2-Methyl-2-pentenoic acid | 0.0185 | 1847 | - | - | - | - |
| Example f6 | 10.0000 | Methyl acrylate | 0.0200 | 1996 | - | - | - | - |
| Example f7 | 9.9988 | Isopropenyl methyl ketone | 0.0187 | 1867 | - | - | - | - |
| Example f8 | 9.9496 | Methacrolein | 0.0172 | 1726 | - | - | - | - |
| Example f9 | 10.0115 | Methacrylamide | 0.0208 | 2073 | - | - | - | - |
| Example f10 | 10.0109 | Cinnamic acid | 0.0215 | 2143 | - | - | - | - |

[Table 12]

| | Addition amount [g] | | | | | | | MAA-containing composition | | | | | | | Result of storage stability |
|---|---|---|---|---|---|---|---|---|---|---|---|---|---|---|---|
| | | | | | | | | MAA | Component A | | Component B1 | | Component B2 | | Concentration change rate of component B1 [%] |
| | Reagent of MAA | a solution | b solution | c solution | Component A | Component B2 | Pure water | Concentration [%] | Compound name | Concentration MA [μmol/L] | Compound name | Concentration MB1 [μmol/L] | Compound name | Concentration MB2 [μmol/L] | |
| Example f1 | 9.9572 | 0.0465 | - | - | - | - | - | 99.93 | Crotonic acid | 137 | 4-Methoxyphenol | 2516 | - | - | -3.98 |
| Example f2 | 9.9493 | 0.0662 | - | - | - | - | - | 99.93 | 2-Methylcrotonic acid | 169 | 4-Methoxyphenol | 2516 | - | - | -3.44 |
| Example f3 | 9.9512 | 0.0496 | - | - | - | - | - | 99.93 | 3,3-Dimethylacrylic acid | 113 | 4-Methoxyphenol | 2516 | - | - | -3.44 |
| Example f4 | 9.9618 | 0.0564 | - | - | - | - | - | 99.93 | 2-Pentenoic acid | 129 | 4-Methoxyphenol | 2516 | - | - | -3.62 |
| Example f5 | 99435 | 0.0525 | - | - | - | - | - | 99.93 | 2-Methyl-2-pentenoic acid | 87 | 4-Methoxyphenol | 2516 | - | - | -4.63 |
| Example f6 | 9.9451 | 0.0437 | - | - | - | - | - | 99.93 | Methyl acrylate | 103 | 4-Methoxyphenol | 2516 | - | - | -3.96 |
| Example f7 | 9.9410 | 0.0478 | - | - | - | - | - | 99.93 | Isopropenyl methyl ketone | 108 | 4-Methoxyphenol | 2516 | - | - | -4.58 |
| Example f8 | 9.9449 | 0.0532 | - | - | - | - | - | 99.93 | Methacrolein | 134 | 4-Methoxyphenol | 2516 | - | - | -8.03 |
| Example f9 | 9.9554 | 0.0575 | - | - | - | - | - | 99.93 | Methacrylamide | 143 | 4-Methoxyphenol | 2516 | - | - | -4.23 |
| Example f10 | 9.9565 | 0.0622 | - | - | - | - | - | 99.93 | Cinnamic acid | 92 | 4-Methoxyphenol | 2516 | - | - | -4.32 |

As shown in Tables 11 and 12, in Examples f1 to f10, the decomposition of the polymerization inhibitor (component B1) in the methacrylate-containing composition after the storage was suppressed. It can be said that the quality stability during the storage was high.

A methacrylic ester could be obtained by esterifying the methacrylate-containing composition obtained in the present example. In addition, a methacrylic acid polymer could be obtained by polymerizing a polymerizable composition comprising the methacrylate-containing composition obtained in the present example.

INDUSTRIAL APPLICABILITY

According to the present invention, it is possible to provide a methacrylate-containing composition having high quality stability, in which decomposition of a polymerization inhibitor during storage is suppressed.

According to the present invention, a methacrylate-containing composition which can be used as a raw material or the like of an acrylic resin can be stably stored for a long period of time, which is industrially useful.

## Claims

**1.** A methacrylate-containing composition comprising:

methacrylic acid; and
at least one selected from the group consisting of a component A1 which is a compound represented by Formula (11), a component A21 which is a compound represented by Formula (21), a component A3 which is a compound represented by Formula (31), a component A4 which is a compound represented by Formula (41), a component A5 which is a compound represented by Formula (51), and a component A6 which is a compound represented by Formula (61),
wherein the methacrylate-containing composition may further optionally comprise a component B which is a polymerization inhibitor as necessary, and
a concentration of the methacrylic acid is 98.00% to 99.99% by mass,

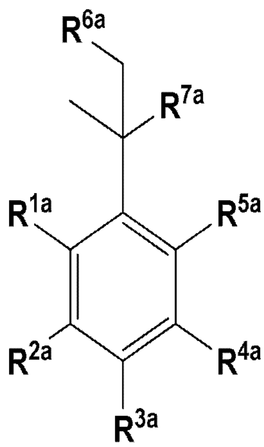 ・・・(11)

in Formula (11),
$R^{1a}$, $R^{2a}$, $R^{3a}$, $R^{4a}$, $R^{5a}$, and $R^{6a}$ each independently represent a hydrogen atom, an alkyl group, an alkenyl group, a hydroxy group, an alkoxy group, an amino group, a monovalent group including a carbonyl group, an alkylthio group, or an arylthio group,
two or more of $R^{1a}$, $R^{2a}$, $R^{3a}$, $R^{4a}$, and $R^{5a}$ are groups other than a hydrogen atom, and
$R^{7a}$ represents a hydrogen atom, an alkenyl group, an aryl group, a hydroxy group, an alkoxy group, an amino group, a monovalent group including a carbonyl group, an alkylthio group, or an arylthio group,

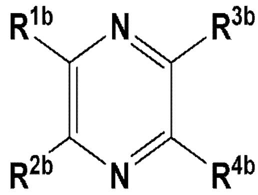

・・・(21)

in Formula (21),
$R^{1b}$, $R^{2b}$, $R^{3b}$, and $R^{4b}$ each independently represent a hydrogen atom, an alkyl group, an alkenyl group, an aryl group, a hydroxy group, an alkoxy group, an amino group, a monovalent group including a carbonyl group, an alkylthio group, or an arylthio group,

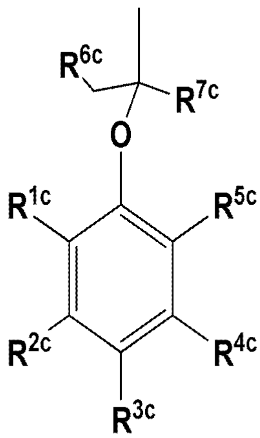

・・・(31)

in Formula (31),
$R^{1c}$, $R^{2c}$, $R^{3c}$, $R^{4c}$, $R^{5c}$, $R^{6c}$, and $R^{7c}$ independently represent a hydrogen atom, an alkyl group, an alkenyl group, an aryl group, a hydroxy group, an alkoxy group, an amino group, a monovalent group including a carbonyl group, an alkylthio group, or an arylthio group,

$R^d$-C≡N ··· (41)

in Formula (41),
$R^d$ represents an alkyl group having 1 to 6 carbon atoms, an alkenyl group having 2 to 6 carbon atoms, or an aryl group having 1 to 12 carbon atoms, where these groups may further have a substituent,

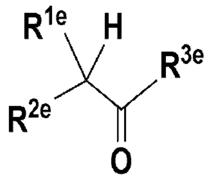

・・・(51)

in Formula (51),
$R^{1e}$ and $R^{2e}$ each independently represent a hydrogen atom, an alkyl group, an alkenyl group, an aryl group, a hydroxy group, an alkoxy group, an alkylthio group, or an arylthio group,
$R^{3e}$ represents a hydrogen atom, an alkyl group, an alkenyl group, an aryl group, a hydroxy group, an alkoxy group, an amino group, an alkylthio group, or an arylthio group, and
$R^{1e}$ and $R^{2e}$, $R^{2e}$ and $R^{3e}$, or $R^{3e}$ and $R^{1e}$ may be linked to each other to form a ring,
provided that a total number of carbon atoms in $R^{1e}$ and $R^{2e}$ is 2 or more,

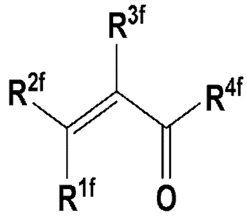

・・・(61)

in Formula (61),
$R^{1f}$, $R^{2f}$, and $R^{3f}$ each independently represent a hydrogen atom, an alkyl group, an alkenyl group, an aryl group, a hydroxy group, an alkoxy group, an amino group, an alkylthio group, or an arylthio group, and
$R^{4f}$ represents an alkyl group, an alkenyl group, an aryl group, a hydroxy group, an alkoxy group, an amino group, a monovalent group including a carbonyl group, an alkylthio group, or an arylthio group,
provided that a case where $R^{1f}$ = H, $R^{2f}$ = H, $R^{3f}$ = $CH_3$, and $R^{4f}$ = OH, that is, a case where the component A6 is methacrylic acid is excluded, in which H represents a hydrogen atom, C represents a carbon atom, and O represents an oxygen atom, and a total number of carbon atoms in $R^{1f}$, $R^{2f}$, and $R^{3f}$ is 1 or more.

**2.** The methacrylate-containing composition according to Claim 1,
wherein, when a concentration of the component A1 is indicated by $M_{A1}$ (μmol/L) and a concentration of the component B is indicated by $M_B$ (μmol/L), $M_B/M_{A1}$ is 0.005 to 100.

**3.** The methacrylate-containing composition according to Claim 1,
wherein, when a concentration of the component A1 is indicated by $M_{A1}$ (μmol/L), $M_{A1}$ is 1 to 50000 μmol/L.

**4.** The methacrylate-containing composition according to Claim 3,
wherein $M_{A1}$ is 10 to 30000 μmol/L.

**5.** The methacrylate-containing composition according to Claim 1,
wherein, when a concentration of the component A21 is indicated by $M_{A21}$ (μmol/L) and a concentration of the component B is indicated by $M_B$ (μmol/L), $M_B/M_{A21}$ is 0.005 to 100.

**6.** The methacrylate-containing composition according to Claim 1,
wherein, when a concentration of the component A21 is indicated by $M_{A21}$ (μmol/L), $M_{A21}$ is 1 to 50000 μmol/L.

**7.** The methacrylate-containing composition according to Claim 6,
wherein $M_{A21}$ is 10 to 30000 μmol/L.

**8.** The methacrylate-containing composition according to Claim 1,
wherein, when a concentration of the component A3 is indicated by $M_{A3}$ (μmol/L), $M_{A3}$ is 1 to 50000 μmol/L.

**9.** The methacrylate-containing composition according to Claim 8,
wherein $M_{A3}$ is 10 to 30000 μmol/L.

**10.** The methacrylate-containing composition according to Claim 1,
wherein, when a concentration of the component A3 is indicated by $M_{A3}$ (μmol/L) and a concentration of the component B is indicated by $M_B$ (μmol/L), $M_B/M_{A3}$ is 0.005 to 100.

**11.** The methacrylate-containing composition according to Claim 1,
wherein, when a concentration of the component A4 is indicated by $M_{A4}$ (μmol/L) and a concentration of the component B is indicated by $M_B$ (μmol/L), $M_B/M_{A4}$ is 0.005 to 100.

**12.** The methacrylate-containing composition according to Claim 1,
wherein, when a concentration of the component A4 is indicated by $M_{A4}$ (μmol/L), $M_{A4}$ is 1 to 20000 μmol/L.

**13.** The methacrylate-containing composition according to Claim 12,
wherein $M_{A4}$ is 10 to 15000 μmol/L.

**14.** The methacrylate-containing composition according to Claim 1,

wherein, when a concentration of the component A5 is indicated by $M_{A5}$ (μmol/L) and a concentration of the component B is indicated by $M_B$ (μmol/L), $M_B/M_{A5}$ is 0.005 to 100.

**15.** The methacrylate-containing composition according to Claim 1,
wherein, when a concentration of the component A5 is indicated by $M_{A5}$ (μmol/L), $M_{A5}$ is 1 to 50000 μmol/L.

**16.** The methacrylate-containing composition according to Claim 15,
wherein $M_{A5}$ is 10 to 30000 μmol/L.

**17.** The methacrylate-containing composition according to Claim 1,
wherein, when a concentration of the component A6 is indicated by $M_{A6}$ (μmol/L) and a concentration of the component B is indicated by $M_B$ (μmol/L), $M_B/M_{A6}$ is 0.005 to 100.

**18.** The methacrylate-containing composition according to Claim 1,
wherein, when a concentration of the component A6 is indicated by $M_{A6}$ (μmol/L), $M_{A6}$ is 1 to 85000 μmol/L.

**19.** The methacrylate-containing composition according to Claim 18,
wherein $M_{A6}$ is 10 to 40000 μmol/L.

**20.** The methacrylate-containing composition according to Claim 1,
wherein, when a concentration of the component B is indicated by $M_B$ (μmol/L), $M_B$ is 1 to 50000 μmol/L.

**21.** The methacrylate-containing composition according to Claim 20,
wherein $M_B$ is 10 to 10000 μmol/L.

**22.** The methacrylate-containing composition according to Claim 1,

wherein, in Formula (11), $R^{1a}$, $R^{2a}$, $R^{3a}$, $R^{4a}$, $R^{5a}$, and $R^{6a}$ are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group having 0 to 6 carbon atoms, or a monovalent group including a carbonyl group having 1 to 6 carbon atoms, and
$R^{7a}$ is a hydrogen atom, an alkenyl group having 2 to 5 carbon atoms, an aryl group having 1 to 12 carbon atoms, a hydroxy group, an alkoxy group having 1 to 6 carbon atoms, an amino group having 0 to 6 carbon atoms, or a monovalent group including a carbonyl group having 1 to 6 carbon atoms.

**23.** The methacrylate-containing composition according to Claim 1,

wherein, in Formula (11), $R^{1a}$, $R^{2a}$, $R^{3a}$, $R^{4a}$, $R^{5a}$, and $R^{6a}$ are each independently a hydrogen atom, an alkyl group having 1 to 5 carbon atoms, a hydroxy group, or an alkoxy group having 1 to 6 carbon atoms, and
$R^{7a}$ is a hydrogen atom, a carboxy group, or an alkoxycarbonyl group having 2 to 6 carbon atoms.

**24.** The methacrylate-containing composition according to Claim 1,

wherein, in Formula (11), $R^{1a}$, $R^{2a}$, $R^{3a}$, $R^{4a}$, $R^{5a}$, and $R^{6a}$ are each independently a hydrogen atom, a methyl group, an ethyl group, an n-propyl group, an isopropyl group, an n-butyl group, an s-butyl group, an i-butyl group, a t-butyl group, a hydroxy group, or a methoxy group, and
$R^{7a}$ is a hydrogen atom, a carboxy group, or a methoxycarbonyl group.

**25.** The methacrylate-containing composition according to Claim 1,
wherein $R^{1b}$, $R^{2b}$, $R^{3b}$, and $R^{4b}$ in Formula (21) are each independently a hydrogen atom, an alkyl group having 1 to 5 carbon atoms, an alkenyl group having 2 to 5 carbon atoms, an aryl group having 1 to 12 carbon atoms, an alkoxy group having 1 to 6 carbon atoms, an amino group having 0 to 6 carbon atoms, a monovalent group including a carbonyl group having 1 to 6 carbon atoms, an alkylthio group having 1 to 5 carbon atoms, or an arylthio group having 6 to 10 carbon atoms.

**26.** The methacrylate-containing composition according to Claim 1,
wherein $R^{1b}$, $R^{2b}$, $R^{3b}$, and $R^{4b}$ in Formula (21) are each independently a hydrogen atom, an alkyl group having 1 to 5 carbon atoms, or an alkoxy group having 1 to 6 carbon atoms.

**27.** The methacrylate-containing composition according to Claim 1, wherein $R^{1b}$, $R^{2b}$, $R^{3b}$, and $R^{4b}$ in Formula (21) are each independently a hydrogen atom, a methyl group, an ethyl group, an n-propyl group, an isopropyl group, or a methoxy group.

**28.** The methacrylate-containing composition according to Claim 1, wherein $R^{1c}$, $R^{2c}$, $R^{3c}$, $R^{4c}$, $R^{5c}$, $R^{6c}$, and $R^{7c}$ in Formula (31) are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group having 0 to 6 carbon atoms, or a monovalent group including a carbonyl group having 1 to 6 carbon atoms.

**29.** The methacrylate-containing composition according to Claim 1, wherein $R^{1c}$, $R^{2c}$, $R^{3c}$, $R^{4c}$, $R^{5c}$, $R^{6c}$, and $R^{7c}$ in Formula (31) are each independently a hydrogen atom, an alkyl group having 1 to 5 carbon atoms, a hydroxy group, an alkoxy group having 1 to 5 carbon atoms, a carboxy group, or an alkoxycarbonyl group having 2 to 6 carbon atoms.

**30.** The methacrylate-containing composition according to Claim 1, wherein $R^{1c}$, $R^{2c}$, $R^{3c}$, $R^{4c}$, $R^{5c}$, $R^{6c}$, and $R^{7c}$ in Formula (31) are each independently a hydrogen atom, a methyl group, an ethyl group, an n-propyl group, an isopropyl group, an n-butyl group, an s-butyl group, an i-butyl group, a t-butyl group, a hydroxy group, a methoxy group, a carboxy group, or a methoxycarbonyl group.

**31.** The methacrylate-containing composition according to Claim 1, wherein $R^d$ in Formula (41) is an alkyl group having 1 to 3 carbon atoms, an alkenyl group having 2 or 3 carbon atoms, or an aryl group having 6 to 8 carbon atoms.

**32.** The methacrylate-containing composition according to Claim 1, wherein $R^d$ in Formula (41) is a methyl group, an ethyl group, a vinyl group, an isopropenyl group, or a phenyl group.

**33.** The methacrylate-containing composition according to Claim 1,

wherein, in Formula (51), $R^{1e}$ and $R^{2e}$ are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an alkylthio group having 1 to 5 carbon atoms, or an arylthio group having 6 to 10 carbon atoms, and
$R^{3e}$ is a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group having 0 to 6 carbon atoms, an alkylthio group having 1 to 5 carbon atoms, or an arylthio group having 6 to 10 carbon atoms.

**34.** The methacrylate-containing composition according to Claim 1,

wherein, in Formula (51), $R^{1e}$ and $R^{2e}$ are each independently a hydrogen atom or an alkyl group having 1 to 5 carbon atoms, and
$R^{3e}$ is a hydrogen atom, an alkyl group having 1 to 5 carbon atoms, a hydroxy group, or an alkoxy group having 1 to 6 carbon atoms.

**35.** The methacrylate-containing composition according to Claim 1,

wherein, in Formula (51), $R^{1e}$ and $R^{2e}$ are each independently a hydrogen atom, a methyl group, an ethyl group, an n-propyl group, or an isopropyl group, and
$R^{3e}$ is a hydroxy group.

**36.** The methacrylate-containing composition according to Claim 1,

wherein, in Formula (61), $R^{1f}$, $R^{2f}$, and $R^{3f}$ are each independently a hydrogen atom, an alkyl group having 1 to 10 carbon atoms, an alkenyl group having 2 to 10 carbon atoms, an aryl group having 1 to 12 carbon atoms, a hydroxy group, an alkoxy group having 1 to 6 carbon atoms, an amino group having 0 to 6 carbon atoms, a monovalent group including a carbonyl group having 1 to 6 carbon atoms, an alkylthio group having 1 to 5 carbon atoms, or an arylthio group having 6 to 10 carbon atoms, and

$R^{4f}$ is an alkyl group having 1 to 10 carbon atoms, an alkenyl group having 2 to 10 carbon atoms, an aryl group having 1 to 12 carbon atoms, a hydroxy group, an alkoxy group having 1 to 6 carbon atoms, an amino group having 0 to 6 carbon atoms, a monovalent group including a carbonyl group having 1 to 6 carbon atoms, an alkylthio group having 1 to 5 carbon atoms, or an arylthio group having 6 to 10 carbon atoms.

**37.** The methacrylate-containing composition according to Claim 1,

wherein, in Formula (61), $R^{1f}$, $R^{2f}$, and $R^{3f}$ are each independently a hydrogen atom or an alkyl group having 1 to 5 carbon atoms, and
$R^{4f}$ is an alkyl group having 1 to 5 carbon atoms, a hydroxy group, or an alkoxy group having 1 to 6 carbon atoms.

**38.** The methacrylate-containing composition according to Claim 1,

wherein, in Formula (61), $R^{1f}$, $R^{2f}$, and $R^{3f}$ are each independently a hydrogen atom, a methyl group, an ethyl group, an n-propyl group, or an isopropyl group, and
$R^{4f}$ is a hydroxy group.

**39.** The methacrylate-containing composition according to Claim 1,
wherein a molecular weight of the component A1 is 2,000 or less.

**40.** The methacrylate-containing composition according to Claim 1,
wherein a molecular weight of the component A21 is 1,000 or less.

**41.** The methacrylate-containing composition according to Claim 1,
wherein a molecular weight of the component A3 is 2,000 or less.

**42.** The methacrylate-containing composition according to Claim 1,
wherein a molecular weight of the component A4 is 1,000 or less.

**43.** The methacrylate-containing composition according to Claim 1,
wherein a molecular weight of the component A5 is 1,000 or less.

**44.** The methacrylate-containing composition according to Claim 1,
wherein a molecular weight of the component A6 is 1,000 or less.

**45.** The methacrylate-containing composition according to Claim 1,
wherein the component B is at least one selected from the group consisting of a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

**46.** The methacrylate-containing composition according to Claim 1,
wherein the component B is at least one selected from the group consisting of a phenol-based compound, an N-oxyl-based compound, an amine-based compound, a phosphorus-containing compound, and a sulfur-containing compound.

**47.** The methacrylate-containing composition according to Claim 1,
wherein the component B is a phenol-based compound.

**48.** The methacrylate-containing composition according to Claim 1,
wherein the component B is at least one selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine.

**49.** The methacrylate-containing composition according to Claim 1,
wherein the component B is at least one selected from the group consisting of hydroquinone, 4-methoxyphenol, 2,4-dimethyl-6-t-butylphenol, and 2,6-di-t-butyl-4-methylphenol.

**50.** The methacrylate-containing composition according to Claim 1,
wherein the concentration of the methacrylic acid is 98.50% to 99.99% by mass.

**51.** The methacrylate-containing composition according to Claim 1,
wherein the methacrylate-containing composition comprises no diacetyl, or a concentration of contained diacetyl is 55 μmol/L or less.

**52.** A method for producing a methacrylic ester, comprising:
esterifying the methacrylic acid in the methacrylate-containing composition according to any one of Claims 1 to 51.

**53.** A polymerizable composition comprising:
the methacrylate-containing composition according to any one of Claims 1 to 51.

**54.** The polymerizable composition according to Claim 53, further comprising:
a monomer copolymerizable with the methacrylic acid.

**55.** A method for producing a methacrylic acid polymer, comprising:
polymerizing a polymerizable composition comprising the methacrylate-containing composition according to any one of Claims 1 to 51.

**56.** The production method according to Claim 55,
wherein the polymerizable composition further comprises a monomer copolymerizable with the methacrylic acid.

**57.** The production method according to Claim 56,
wherein the polymerizable composition comprises 50 parts by mass or more of the monomer copolymerizable with the methacrylic acid with respect to 100 parts by mass of the polymerizable composition.

**INTERNATIONAL SEARCH REPORT**

International application No.
**PCT/JP2023/038845**

**A. CLASSIFICATION OF SUBJECT MATTER**

***C08F 20/06***(2006.01)i; ***C07C 43/205***(2006.01)i; ***C07C 53/124***(2006.01)i; ***C07C 57/075***(2006.01)i; ***C07C 57/30***(2006.01)i; ***C07C 67/00***(2006.01)i; ***C07C 69/54***(2006.01)i; ***C07C 255/08***(2006.01)i; ***C08F 2/40***(2006.01)i; ***C08F 2/44***(2006.01)i; ***C08F 220/06***(2006.01)i

FI: C08F20/06; C07C43/205 C; C07C53/124; C07C57/075; C07C57/30; C07C67/00; C07C69/54 Z; C07C255/08; C08F2/40; C08F2/44 B; C08F220/06

According to International Patent Classification (IPC) or to both national classification and IPC

**B. FIELDS SEARCHED**

Minimum documentation searched (classification system followed by classification symbols)

C08F20/06; C07C43/205; C07C53/124; C07C57/075; C07C57/30; C07C67/00; C07C69/54; C07C255/08; C08F2/40; C08F2/44; C08F220/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2001-72643 A (MITSUBISHI RAYON CO., LTD.) 21 March 2001 (2001-03-21) claims, examples | 1, 17-53<br>2-16, 54-57 |
| X<br>A | JP 2005-23060 A (ROHM & HAAS CO.) 27 January 2005 (2005-01-27) claims, examples | 1, 18-19, 22-54<br>2-17, 20-21, 55-57 |
| X<br>A | JP 63-145250 A (BASF AG) 17 June 1988 (1988-06-17) claims, examples | 1, 18-19, 22-57<br>2-17, 20-21 |
| A | WO 2010/016493 A1 (MITSUBISHI RAYON CO., LTD.) 11 February 2010 (2010-02-11) claims, examples | 1-57 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

INTERNATIONAL SEARCH REPORT

International application No.
PCT/JP2023/038845

**Box No. III Observations where unity of invention is lacking (Continuation of item 3 of first sheet)**

This International Searching Authority found multiple inventions in this international application, as follows:

The claims are classified into the following six inventions.

(Invention 1) Claims 1-57 (parts pertaining to component A1)

A special technical feature has been found in the invention first described in claim 1, a "methacrylic acid-containing composition comprising methacrylic acid and a component A1 that is a compound represented by formula (11), wherein the composition optionally further comprises a component B that is a polymerization inhibitor and contains methacrylic acid at 98.00-99.99% by mass".

Thus, the invention in claim 1 pertaining to component A1 for which the presence or absence of a special technical feature has been determined, and the inventions in claims 2-57 referring to said invention are classified as invention 1.

(Invention 2) Claims 1-57 (parts pertaining to component A2)

The invention pertaining to a methacrylic acid-containing composition comprising methacrylic acid and component A2 among the inventions in claim 1 and the invention first described in claim 1 share the technical feature of a "methacrylic acid-containing composition comprising methacrylic acid and an additional organic compound, wherein the composition contains methacrylic acid at 98.00-99.99% by mass". However, this feature does not make a contribution over the prior art in light of the contents disclosed in documents 1-3, and thus cannot be said to be a special technical feature. In addition, there are no other same or corresponding special technical features between the invention first described in claim 1 and the invention pertaining to the methacrylic acid-containing composition comprising methacrylic acid and component A2.

Accordingly, the invention pertaining to the methacrylic acid-containing composition comprising methacrylic acid and component A2 among the inventions in claim 1 cannot be classified as invention 1.

Thus, the invention in claim 1 pertaining to component A2 and the inventions in claims 2-57 referring to said invention are classified as invention 2.

(Invention 3) Claims 1-57 (parts pertaining to component A3)

The invention pertaining to a methacrylic acid-containing composition comprising methacrylic acid and component A3 among the inventions in claim 1 and inventions 1 and 2 share the technical feature of a "methacrylic acid-containing composition comprising methacrylic acid and an additional organic compound, wherein the composition contains methacrylic acid at 98.00-99.99% by mass". However, this feature does not make a contribution over the prior art in light of the contents disclosed in documents 1-3, and thus cannot be said to be a special technical feature. In addition, there are no other same or corresponding special technical features between invention 1 or 2 and the invention pertaining to the methacrylic acid-containing composition comprising methacrylic acid and component A3.

Accordingly, the invention pertaining to the methacrylic acid-containing composition comprising methacrylic acid and component A3 among the inventions in claim 1 cannot be classified as invention 1 or 2.

Thus, the invention in claim 1 pertaining to component A3 and the inventions in claims 2-57 referring to said invention are classified as invention 3.

(Invention 4) Claims 1-57 (parts pertaining to component A4)

The invention pertaining to a methacrylic acid-containing composition comprising methacrylic acid and component A4 among the inventions in claim 1 and inventions 1-3 share the technical feature of a "methacrylic acid-containing composition comprising methacrylic acid and an additional organic compound, wherein the composition contains methacrylic acid at 98.00-99.99% by mass". However, this feature does not make a contribution over the prior art in light of the contents disclosed in documents 1-3, and thus cannot be said to be a special technical feature. In addition, there are no other same or corresponding special technical features between inventions 1-3 and the invention pertaining to the methacrylic acid-containing composition comprising methacrylic acid and component A4.

Accordingly, the invention pertaining to the methacrylic acid-containing composition comprising methacrylic acid and component A4 among the inventions in claim 1 cannot be classified as inventions 1-3.

INTERNATIONAL SEARCH REPORT

International application No.
PCT/JP2023/038845

**Box No. III Observations where unity of invention is lacking (Continuation of item 3 of first sheet)**

Thus, the invention in claim 1 pertaining to component A4 and the inventions in claims 2-57 referring to said invention are classified as invention 4.

(Invention 5) Claims 1-57 (parts pertaining to component A5)

The invention pertaining to a methacrylic acid-containing composition comprising methacrylic acid and component A5 among the inventions in claim 1 and inventions 1-4 share the technical feature of a "methacrylic acid-containing composition comprising methacrylic acid and an additional organic compound, wherein the composition contains methacrylic acid at 98.00-99.99% by mass". However, this feature does not make a contribution over the prior art in light of the contents disclosed in documents 1-3, and thus cannot be said to be a special technical feature. In addition, there are no other same or corresponding special technical features between inventions 1-4 and the invention pertaining to the methacrylic acid-containing composition comprising methacrylic acid and component A5.

Accordingly, the invention pertaining to the methacrylic acid-containing composition comprising methacrylic acid and component A5 among the inventions in claim 1 cannot be classified as inventions 1-4.

Thus, the invention in claim 1 pertaining to component A5 and the inventions in claims 2-57 referring to said invention are classified as invention 5.

(Invention 6) Claims 1-57 (parts pertaining to component A6)

The invention pertaining to a methacrylic acid-containing composition comprising methacrylic acid and component A6 among the inventions in claim 1 and inventions 1-5 share the technical feature of a "methacrylic acid-containing composition comprising methacrylic acid and an additional organic compound, wherein the composition contains methacrylic acid at 98.00-99.99% by mass". However, this feature does not make a contribution over the prior art in light of the contents disclosed in documents 1-3, and thus cannot be said to be a special technical feature. In addition, there are no other same or corresponding special technical features between inventions 1-5 and the invention pertaining to the methacrylic acid-containing composition comprising methacrylic acid and component A6.

Accordingly, the invention pertaining to the methacrylic acid-containing composition comprising methacrylic acid and component A6 among the inventions in claim 1 cannot be classified as inventions 1-5.

Thus, the invention in claim 1 pertaining to component A6 and the inventions in claims 2-57 referring to said invention are classified as invention 6.

Form PCT/ISA/210 (continuation of first sheet) (January 2015)

**INTERNATIONAL SEARCH REPORT**

International application No.
**PCT/JP2023/038845**

**Box No. III Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/038845**

| Patent document cited in search report | Publication date (day/month/year) | Patent family member(s) | Publication date (day/month/year) |
|---|---|---|---|
| JP 2001-72643 A | 21 March 2001 | (Family: none) | |
| JP 2005-23060 A | 27 January 2005 | US 2004/0267050 A1<br>claims, examples<br>EP 1493728 A1<br>CN 1576266 A<br>KR 10-2011-0123712 A | |
| JP 63-145250 A | 17 June 1988 | US 4828652 A<br>claims, examples<br>EP 270999 A1 | |
| WO 2010/016493 A1 | 11 February 2010 | US 2011/0137072 A1<br>claims, examples<br>EP 2308820 A1<br>KR 10-2011-0041562 A<br>CN 102105429 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022172927 A
- JP 2022172928 A
- JP 2022173123 A
- JP 2022173124 A
- JP 2022173222 A
- JP 2022173223 A
- JP 2004155757 A
- JP 2001072639 A
- JP 2008101013 A
- JP 2002533309 W
- JP 2002513034 W


### Non-patent literature cited in the description


- **T. KURODA**. Development of Catalyst for Producing Methyl Methacrylate. *Catalysts, The Catalysis Society of Japan*, 2003, vol. 45 (5), 366-371
- On the Function of Polymerization Inhibitor. **TAKAYUKI OTSU**. Organic Synthesis Chemistry. The Chemical Society of Japan, 1975, vol. 33, 634-640